(19) Europäisches Patentamt
European Patent Office
Office européen des brevets

(11) EP 4 385 984 A1

# (12) EUROPEAN PATENT APPLICATION

(43) Date of publication:
**19.06.2024 Bulletin 2024/25**

(21) Application number: **22213353.0**

(22) Date of filing: **14.12.2022**

(51) International Patent Classification (IPC):
***C07D 401/12*** *(2006.01)* ***C07D 405/14*** *(2006.01)*
***A61P 29/00*** *(2006.01)* ***A61K 31/4155*** *(2006.01)*

(52) Cooperative Patent Classification (CPC):
**C07D 405/14; A61P 29/00; C07D 401/12**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Grünenthal GmbH**
**52078 Aachen (DE)**

(72) Inventors:
- **Krüger, Sebastian**
  **52078 Achen (DE)**
- **Mülbaier, Marcel**
  **52078 Achen (DE)**
- **Dialer, Clemens**
  **52078 Achen (DE)**

## (54) PYRIDINE-N-OXIDES AS INHIBITORS OF NAV1.8

(57) The invention relates to compounds according to general formula (I)

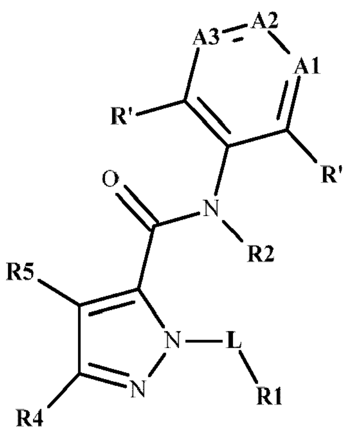

(I)

which act as inhibitors of $Na_V1.8$ and can be used in the treatment of pain.

EP 4 385 984 A1

## Description

The invention relates to compounds according to general formula (I)

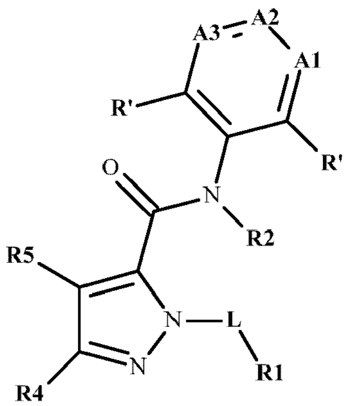

(I)

which act as inhibitors of $Na_V1.8$ and can be used in the treatment of pain.

Normal pain sensation (nociception) serves primarily as a survival mechanism, the body's way of self-protection, alerting against (further) tissue damage and disease from noxious stimuli. For instance, acute pain can arise when the external environment (temperature, pressure, chemicals) activates modality specific receptors (nociceptors) and ion channels within the skin. The peripheral terminals of pain-signaling neurons - whose cell bodies are found in the dorsal root ganglia [DRG] and trigeminal ganglia [TG] - convert the external stimuli into electrochemical generator potentials. Specific voltage-gated sodium channels ($Na_Vs$) integrate and amplify these generator signals until the threshold for an action potential [AP] is reached. Thus, what starts as a noxious stimuli in the periphery eventually leads to action potential firing that travels towards the central nervous system, synapsing first onto neurons in the spinal cord and then towards the brain. Navs also function to support propagation of action potentials to the central terminals within the spinal cord. At the end of its travels along the somatosensory pathway, the action potential signal is interpreted as pain by the brain (Lumpkin and Caterina, Nature (2007), Vol.445 pp 858-865; and Crawford and Caterina Toxicologic Pathology (2020) 48(1)-174; Goodwin G and McMahon S.B. Nature Reviews Neuroscience (2021) Vol 22 pp 263-274; Bennett D.L. et al. Physiol Rev 99 (2019) Vol 99 pp 1079-1151).

Abnormal persistent neuropathic pain arises as a consequence of a lesion or disease of this somatosensory pathway. In response to nerve injury or inflammation, abnormal changes in ion channel expression can cause hyper-excitability of pain-signaling neurons and their nerves/axons, thus resulting in pathological pain.

The voltage-gated $Na_V1.8$ sodium channel is a therapeutic target for analgesia because of its restricted expression profile (almost exclusive to peripheral sensory tissues), its placement along the pain pathway (free nerve endings, sciatic nerve, and DRG), prominent physiological role in pain signaling (supports upstroke of AP and facilitates repetitive AP firing), and supporting genetic/pharmaco-phenotypic evidence (human/animal studies showing changes in $Na_V1.8$ function cause parallel changes in pain sensitivity).

Regarding its expression profile along the pain pathway, because $Na_V1.8$ was first found predominantly in peripheral sensory neurons of the dorsal root ganglia (DRG) and trigeminal ganglia (TG), it was originally termed SNS (sensory neuron specific) (Akopian A.N. et al Nature (1996) Vol 379 pp 257-261) or PN3 (peripheral nerve 3) (Sangameswaran L. et al J. Biol. Chem. (1996) Vol 271 pp 5953-5956). As well, $Na_V1.8$ is localized at free nerve endings, where pain signaling is initiated in the skin (Persson A.K. et al Mol. Pain. (2010) 6:84) and is diffusely localized along the entire length of non-myelinated axons of sciatic nerve (Rush A.M. et al. Eur.J.Neurosci (2005) Vol 22 pp 39-49).

In contrast, $Na_V1.8$ has minimal expression in nonneuronal tissue, such as heart and skeletal muscle, and in the CNS, including brain and spinal cord (9, 10, 338, 406) (Akopian A.N. op. cit.; Akopian A.N. et al. Nat. Neurosci (1999) Vol 2 pp 541-548; Novakovic S.D. et al. J. Neurosci. (1998) Vol 18, pp 2184-2187; and Sagameswaran L. op. cit.).

Regarding its physiological role, $Na_V1.8$ contributes the majority of the inward current during the rising phase of an all-or-none action potential in nociceptive sensory neurons (Blair N.T. et al. J. Neurosci. (2003) Vol 23 pp 10338-10350 and Renganathan M et al. J. Neurophysiol (2001) Vol 86 pp 629-640) - and also contributes most of the current in subsequent spikes during repetitive firing in DRG neurons (Choi J.S. J. Neurophysiol (2011) Vol 106 pp 3173-3184; and Tan Z.Y. et al. J. Neurosci. (2014) Vol 34 pp 7190-7197).

Regarding genetic and pharmacology studies, gain-of-function mutations in $Na_V1.8$ were found in patients with

chronic neuropathic pain such as small fiber neuropathy (Faber C.G. et al. (2012) Ann. Neurol Vol 71 pp 26-39; Han C et al. J. Neurol Neurosurg Psychiatry (2014) Vol 85 pp 499-505; and, Kist A.M. et al. PLoS One (2016) Vol 11 e0161789); Eijkenboom I. et al J. Neurol Neurosurg Psychiatry (2019) 90 (3) pp 342-352); loss-of-function (gene knockout) studies in mice reduced pain sensitivity, notably in nociception (Laird J.M. et al. J. Neurosci (2002) J. Neurosci Vol 22 pp 8352-8356; Jarvis M.F. et al Proc Natl Acad Sci USA (2007) Vol 104 pp 8520-8525; Joshi S.K. et al Pain (2006) Vol 123 pp 75-82) and in neuropathic models (Roza C. et al J Physiol (2003) Vol 550 pp 921-926); $Na_V1.8$-selective small molecule inhibitors reduced pain in rodents, specifically in inflammatory and neuropathic models (Jarvis et al. op. cit.; Kort M.E. et al Bioorg Med Chem Lett (2010) Vol 20 pp 6812-6815; Scanio M.J. et al Bioorg Med Chem (2010) Vol 18 pp 7816-7825; Payne C.E. et al Br J Pharmacol (2015) Vol 172 pp 2654-2670).

Currently, non-selective $Na_V$ channel inhibitors are used to treat epilepsy, cardiac arrhythmia, and chronic pain (Hille, B. J. Gen. Physiol. (1977) Vol. 69 pp. 497-515; Hille. B, Ion Channels of Excitable Membranes (1992) pp. 391-421; Sunderland, Mass., Sinauer Associates, Inc. 3rd ed.; Hondeghem L.M. and Katzung B.G. Annu. Rev. Pharmacol. Toxicol. (1984) Vol. 24. Pp. 387-423; Catterall W.A. Trends Pharmacol. Sci. (1987) Vol. 8 pp.57-65) - however, all of these analgesics have limited efficacy owing to dose-limiting adverse side-effects related to inhibiting $Na_V1.1/Na_V1.2/1.6$ (seizure liability), inhibiting $Na_V1.4$ (muscle weakness/paralysis), inhibiting $Na_V1.5$ (arrhythmia risk).

There is a need to develop a $Na_V1.8$-selective small molecule inhibitor as an effective and safe analgesic.

$Na_v1.8$ inhibitors are also known from WO 2020/092187, WO 2020/092667, WO 2009/049180, WO 2009/049183, WO 2009/049181 and WO 2021/113627.

It was an object of the invention to provide novel compounds which are inhibitors, preferably selective inhibitors, of $Na_V1.8$, and which preferably have advantages over the compounds of the prior art. The novel compounds should in particular be suitable for use in the treatment of pain.

This object has been achieved by the subject-matter of the patent claims.

It was surprisingly found that the compounds according to the invention are highly potent and selective inhibitors of the $Na_V1.8$ channel.

The invention relates to a compound according to general formula (I)

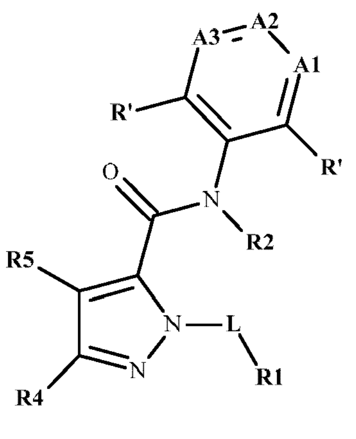

(I)

wherein

(a) one of **A1**, **A2** and **A3** represents C-**R3**; another one of **A1**, **A2** and **A3** represents C-**R'**; and the remaining one of **A1**, **A2** and **A3** represents $N^+$-$O^-$; or

(b) one of **A1**, **A2** and **A3** represents C-**R3'**; another one of **A1**, **A2** and **A3** represents C-**R'**; and the remaining one of **A1**, **A2** and **A3** represents N or C**R'**;

**R'** represents H, F, $CH_3$, $CH_2F$, $CHF_2$, $CF_3$, or $OCH_3$;

**L** represents $CH_2$, $CH(CH_3)$ or $CH(CH_2CH_3)$;

**R1** represents $C_{3-10}$-cycloalkyl, 5 to 11-membered spiroalkyl or dispiroalkyl, 4 to 11-membered bicycloalkyl, 4 to 10-membered heterocycloalkyl, or 5 to 11-membered heterobicycloalkyl;

**R2** represents H or $C_{1-6}$-alkyl;

**R3** represents C(O)-$NH_2$, C(O)-N(H)($C_{1-6}$-alkyl), C(O)-N(H)($C_{3-6}$-cycloalkyl), C(O)-N($C_{1-6}$-alkyl)$_2$, C(O)-N($C_{1-6}$-alkyl)($C_{3-6}$-cycloalkyl), $S(O)_2$-$C_{1-6}$-alkyl, $S(O)_2$-($C_{3-6}$-cycloalkyl), $S(O)_2$-(4 to 6-membered heterocycloalkyl), $S(O)_2$-phenyl, $S(O)_2$-(5 or 6-membered heteroaryl), S(O)-$NH_2$, S(O)-N(H)($C_{1-6}$-alkyl), S(O)-N($C_{1-6}$-alkyl)$_2$, $S(O)_2$-$NH_2$, $S(O)_2$-N(H)($C_{1-6}$-alkyl), $S(O)_2$-N(H)($C_{3-6}$-cycloalkyl), $S(O)_2$-N($C_{1-6}$-alkyl)$_2$, S(O)-$C_{1-6}$-alkyl, S(O)-($C_{3-6}$-cycloalkyl), S(O)-(4 to 6-membered heterocycloalkyl), S(O)-phenyl, S(O)-(5 or 6-membered heteroaryl), $OCF_3$, $OCF_2H$, CN, OH, O-$C_{3-6}$-cycloalkyl, O-(4 to 6-membered heterocycloalkyl), or S(O)(NR**3a**)**R3b**;

**R3'** represents C(O)OH, C(O)O$C_{1-6}$-alkyl, or P(O)($C_{1-6}$-alkyl)$_2$;

wherein

**R3a** represents H, $C_{1-6}$-alkyl, C(O)$C_{1-5}$-alkyl, C(O)$C_{1-5}$-alkyl-$NH_2$, C(O)$C_{1-5}$-alkyl-NH-$CH_3$, C(O)$C_{1-5}$-alkyl-N($CH_3$)$_2$, $C_{3-10}$-cycloalkyl, $C_{1-6}$-alkylene-$C_{3-10}$-cycloalkyl, 4 to 10-membered heterocycloalkyl, or $C_{1-6}$-alkylene-(4 to 10-membered heterocycloalkyl);

**R3b** represents $NH_2$, $C_{1-6}$-alkyl, $C_{3-10}$-cycloalkyl, $C_{1-6}$-alkylene-$C_{3-10}$-cycloalkyl, 4 to 10-membered heterocycloalkyl, or $C_{1-6}$-alkylene-(4 to 10-membered heterocycloalkyl); or **R3a** and **R3b** mean $(CH_2)_{3-5}$ and together with the atoms to which they are attached form a ring;

**R4** represents H, F, Cl, Br, CN, $CHF_2$, $CH_2F$, $CF_3$, $C_{1-6}$-alkyl, $C_{3-10}$-cycloalkyl, $C_{1-6}$-alkylene-$C_{3-10}$-cycloalkyl, 4 to 11-membered bicycloalkyl, 5 to 11-membered spiroalkyl or bisspiroalkyl, 4 to 10-membered heterocycloalkyl, $C_{1-6}$-alkylene-(4 to 10-membered heterocycloalkyl), $NH_2$, N(H)($C_{1-6}$-alkyl), N($C_{1-6}$-alkyl)$_2$, O-$C_{1-6}$-alkyl, O-$C_{3-10}$-cycloalkyl, O-$C_{1-6}$-alkylene-$C_{3-10}$-cycloalkyl, O-(4 to 6-membered heterocycloalkyl), or O-$C_{1-6}$-alkylene-(4 to 6-membered heterocycloalkyl);

**R5** represents H, F, Cl, Br, CN, $CHF_2$, $CH_2F$, $CF_3$, $C_{1-6}$-alkyl, $C_{3-10}$-cycloalkyl, $C_{1-6}$-alkylene-$C_{3-10}$-cycloalkyl, 4 to 10-membered heterocycloalkyl, $C_{1-6}$-alkylene-(4 to 10-membered heterocycloalkyl), $NH_2$, N(H)($C_{1-6}$-alkyl), N($C_{1-6}$-alkyl)$_2$, O-$C_{1-6}$-alkyl, O-$C_{3-10}$-cycloalkyl, O-$C_{1-6}$-alkylene-$C_{3-10}$-cycloalkyl, O-(4 to 6-membered heterocycloalkyl), or O-$C_{1-6}$-alkylene-(4 to 6-membered heterocycloalkyl);

wherein $C_{1-6}$-alkyl and $C_{1-6}$-alkylene in each case independently from one another is linear or branched;

wherein $C_{1-6}$-alkyl, $C_{1-6}$-alkylene, $C_{3-10}$-cycloalkyl, $C_{3-6}$-cycloalkyl, 4 to 11-membered bicycloalkyl, 5 to 11-membered spiroalkyl or dispiroalkyl, 4 to 10-membered heterocycloalkyl, 4 to 6-membered heterocycloalkyl, and 5 to 11-membered heterobicycloalkyl in each case independently from one another are unsubstituted or substituted with one, two, three, four or more substituents independently from one another selected from the group consisting of F, Cl, CN, $C_{1-6}$-alkyl, $C_{1-6}$-alkylene-OH, $C_{1-6}$-alkylene-$OCH_3$, $CF_3$, $CF_2H$, $CFH_2$, C(O)-$C_{1-6}$-alkyl, OH, =O, $OCF_3$, $OCF_2H$, $OCFH_2$, O-$C_{1-6}$-alkyl, $C_{1-4}$-alkylene-O-$C_{1-4}$-alkylene-O-$CH_3$, $C_{0-4}$-alkylene-O-($C_{1-4}$-alkylene-O)$_{1-4}$-$CH_3$, $NH_2$, NH-$C_{1-6}$-alkyl, or N($C_{1-6}$-alkyl)$_2$;

wherein phenyl, 5 to 10-membered heteroaryl and 5 or 6-membered heteroaryl in each case independently from one another are unsubstituted or mono- or polysubstituted with one or more substituents selected from the group consisting of F, Cl, Br, CN, $C_{1-6}$-alkyl, $C_{3-6}$-cycloalkyl, $CF_3$, $CF_2H$, $CFH_2$, $C_{1-6}$-alkylene-$CF_3$, $C_{1-6}$-alkylene-$CF_2H$, $C_{1-6}$-alkylene-$CFH_2$, $C_{1-6}$-alkylene-OH, $C_{1-6}$-alkylene-$OCH_3$, C(O)-$C_{1-6}$-alkyl, $OCF_3$, $OCF_2H$, $OCFH_2$, and O-$C_{-6}$-alkyl;

in the form of the free compound or a physiologically acceptable salt thereof.

In a preferred embodiment, the compound according to the invention is present in form of the free compound. For the purpose of specification, "free compound" preferably means that the compound according to the invention is not present in form of a salt. Methods to determine whether a chemical substance is present as the free compound or as a salt are known to the skilled artisan such as $^{14}N$ or $^{15}N$ solid state NMR, x-ray diffraction, x-ray powder diffraction, IR, Raman, XPS. $^{1}H$-NMR recorded in solution may also be used to consider the presence of protonation.

In another preferred embodiment, the compound according to the invention is present in form of a physiologically acceptable salt. For the purposes of this specification, the term "physiologically acceptable salt" preferably refers to a

salt obtained from a compound according to the invention and a physiologically acceptable acid or base.

According to the invention, the compound according to the invention may be present in any possible form including solvates, cocrystals and polymorphs. For the purposes of this specification, the term "solvate" preferably refers to an adduct of (i) a compound according to the invention and/or a physiologically acceptable salt thereof with (ii) distinct molecular equivalents of one or more solvents.

Further, the compound according to the invention may be present in form of the racemate, enantiomers, diastereomers, tautomers or any mixtures thereof.

The compounds according to the invention may have one or more stereocenter. The person skilled in art knows by looking at a chemical structure whether the depicted compound has one or more stereocenters or not.

For some compounds according to the invention that have one or more stereocenters and which chemical structures are disclosed in the examples of the present application, the chemical structure includes bold bonds and/or hashed bonds to indicate the relative structural orientation of those substituents connected by the bold bonds and/or hashed bonds to the superior structure. If the bold bonds and/or hashed bonds are depicted in form of a wedge, the absolute stereochemical configuration of the compound is known and thereby indicated. If the bold bonds and/or hashed bonds are depicted as a straight bond (i.e. no wedge), the absolute stereochemical configuration of the compound has not been determined. In that case, the bold bonds and/or hashed bonds merely serve to indicate that this particular compound is present as one enantiomer or one diastereomer (e.g. cis-diastereomer (i.e. mixture of two cis-enantiomers) or trans-diastereomer (i.e. mixture of two trans-enantiomers)). All compounds according to the invention that have one or more stereocenters but which chemical structures disclosed in the examples of the present application do not include bold bonds and/or hashed bonds, are present as a mixture of the respective stereoisomers.

The invention also includes isotopic isomers of a compound of the invention, wherein at least one atom of the compound is replaced by an isotope of the respective atom which is different from the naturally predominantly occurring isotope, as well as any mixtures of isotopic isomers of such a compound. Preferred isotopes are $^{2}H$ (deuterium), $^{3}H$ (tritium), $^{13}C$ and $^{14}C$. Isotopic isomers of a compound of the invention can generally be prepared by conventional procedures known to a person skilled in the art.

According to the invention, the terms "$C_{1-6}$-alkyl" and "$C_{1-4}$-alkyl" preferably mean acyclic and preferably saturated hydrocarbon residues, which can be linear (i.e. unbranched) or branched and which can be unsubstituted or mono- or polysubstituted (e.g. di- or trisubstituted), and which contain 1 to 6 (i.e. 1, 2, 3, 4, 5 or 6) and 1 to 4 (i.e. 1, 2, 3 or 4) carbon atoms, respectively. Preferably, $C_{1-6}$-alkyl and $C_{1-4}$-alkyl are saturated. Preferred $C_{1-6}$-alkyl groups are selected from the group consisting of methyl, ethyl, n-propyl, 2-propyl, n-butyl, isobutyl, sec-butyl, tert-butyl, n-pentyl, 2-pentyl, 3-pentyl, 2-methylbutyl, 3-methylbutyl, 3-methylbut-2-yl, 2-methylbut-2-yl, 2,2-dimethylpropyl, n-hexyl, 2-hexyl, 3-hexyl, 2-methylpentyl, 4-methylpentyl, 4-methylpent-2-yl, 2-methylpent-2-yl, 3,3-dimethylbutyl, 3,3-dimethylbut-2-yl, 3-methylpentyl, 3-methylpent-2-yl and 3-methylpent-3-yl; more preferably methyl, ethyl, n-propyl, 2-propyl, n-butyl, isobutyl, sec-butyl, tert-butyl, n-pentyl, 2-pentyl, 3-pentyl, 2-methylbutyl, 3-methylbutyl, 3-methylbut-2-yl, 2-methylbut-2-yl, 2,2-dimethylpropyl, n-hexyl. Particularly preferred $C_{1-6}$-alkyl groups are selected from $C_{1-4}$-alkyl groups. Preferred $C_{1-4}$-alkyl groups are selected from the group consisting of methyl, ethyl, n-propyl, 2-propyl, n-butyl, isobutyl, sec-butyl and tert-butyl.

According to the invention, the terms "$C_{1-6}$-alkylene" and "$C_{1-4}$-alkylene" relate to linear or branched and preferably saturated aliphatic residues which can be unsubstituted or mono- or polysubstituted (e.g. di- or trisubstituted) and which are preferably selected from the group consisting of $CH_2$, $CH(CH_3)$, $CH_2CH_2$, $CH_2CH(CH_3)$, $CH(CH_3)CH_2$, $CH_2CH_2CH_2$, $CH(CH_3)CH_2CH_2$, $CH_2CH(CH_3)CH_2$, $CH_2CH_2CH(CH_3)$, $CH_2CH_2CH_2CH_2$, $CH(CH_3)CH_2CH_2CH_2$, $CH_2CH(CH_3)CH_2CH_2$, $CH_2CH_2CH(CH_3)CH_2$, $CH_2CH_2CH_2CH(CH_3)$, $CH_2CH_2CH_2CH_2CH_2$, $CH(CH_3)CH_2CH_2CH_2CH_2$, $CH_2CH(CH_3)CH_2CH_2CH_2$, $CH_2CH_2CH(CH_3)CH_2CH_2$, $CH_2CH_2CH_2CH(CH_3)CH_2$, $CH_2CH_2CH_2CH_2CH(CH_3)$, and $CH_2CH_2CH_2CH_2CH_2CH_2$; more preferably $CH_2$, $CH(CH_3)$, $CH_2CH_2$, $CH_2CH(CH_3)$ and $CH(CH_3)CH_2$, most preferably $CH_2$ and $CH(CH_3)$, and in particular $CH_2$. Preferably, $C_{1-6}$-alkylene is selected from $C_{1-4}$-alkylene.

According to the invention, the terms "$C_{3-10}$-cycloalkyl" and "$C_{3-6}$-cycloalkyl" preferably mean monocyclic aliphatic hydrocarbons containing 3, 4, 5, 6, 7, 8, 9 or 10 carbon atoms as ring members and 3, 4, 5 or 6 carbon atoms as ring members, respectively, wherein the hydrocarbons in each case can be saturated or unsaturated (but not aromatic), unsubstituted or mono- or polysubstituted.

Preferably, $C_{3-10}$-cycloalkyl and $C_{3-6}$-cycloalkyl are saturated. The $C_{3-10}$-cycloalkyl and $C_{3-6}$-cycloalkyl can be bound to the respective superordinate general structure via any desired and possible ring member of the cycloalkyl group. The $C_{3-10}$-cycloalkyl and $C_{3-6}$-cycloalkyl are not condensed with further ring systems and are not bridged.

Preferred $C_{3-10}$-cycloalkyl groups are selected from the group consisting of cyclopropyl, cyclobutyl, cyclopentyl, cyclohexyl, cycloheptyl, cyclooctyl, cyclononyl, and cyclodecyl. Preferred $C_{3-10}$-cycloalkyl groups are selected from $C_{3-6}$-cycloalkyl groups.

According to the invention, the term "4 to 11-membered bicycloalkyl" preferably means bicyclic aliphatic hydrocarbons containing 4, 5, 6, 7, 8, 9, 10 or 11 carbon atoms as ring members, wherein the hydrocarbons in each case can be saturated or unsaturated (but not aromatic), unsubstituted or mono- or polysubstituted. The bicyclic system may

share a common bond (annealed rings) or may be bridged.

Preferably, 4 to 11-membered bicycloalkyl is saturated. The 4 to 11-membered bicycloalkyl can be bound to the respective superordinate general structure via any desired and possible ring member of the 4 to 11-membered bicycloalkyl group.

Preferred 4 to 11-membered bicycloalkyl groups are selected from the group consisting of bicyclo[1.1.0]butyl, bicylo[1.1.1]pentyl, bicyclo[2.1.0]pentyl, bicyclo[3.1.0]hexyl, bicyclo[2.2.0]hexyl, bicyclo[2.1.1]hexyl, bicyclo[4.1.0]heptyl, bicyclo[2.2.1]heptyl, and bicyclo[3.3.0]octyl.

According to the invention, the term "5 to 11-membered spiroalkyl or dispiroalkyl" means spirocyclic or dispirocyclic aliphatic hydrocarbons containing 5, 6, 7, 8, 9, 10 or 11 carbon atoms as ring members, wherein the hydrocarbons in each case can be saturated or unsaturated (but not aromatic), unsubstituted or mono- or polysubstituted. The spirocyclic system shares a common carbon atom connecting the rings. In case of a dispirocyclic system, there are three rings, whereas two of these three rings share a common carbon atom connecting these rings.

Preferably, 5 to 11-membered spiroalkyl or dispiroalkyl is saturated. The 5 to 11-membered spiroalkyl or dispiroalkyl can be bound to the respective superordinate general structure via any desired and possible ring member of the 5 to 11-membered spiroalkyl or dispiroalkyl group.

Preferred 5 to 11-membered spiroalkyl or dispiroalkyl groups are selected from the group consisting of spiro[2.2]pentyl, spiro[2.3]hexyl, spiro[3.3]heptyl, and dispiro[2.0.2.1]heptyl.

According to the invention, the terms "4 to 10-membered heterocycloalkyl" and "4 to 6-membered heterocycloalkyl" preferably mean monocyclic, heterocycloaliphatic saturated or unsaturated (but not aromatic) residues having 4 to 10, i.e. 4, 5, 6, 7, 8, 9 or 10 ring members and 4 to 6, i.e. 4, 5 or 6 ring members, respectively, wherein in each case at least one, if appropriate also two or three carbon atoms are replaced by a heteroatom or a heteroatom group each selected independently of one another from the group consisting of O, S, S(=O), $S(=O)_2$, N, NH and $N(C_{1-4}$-alkyl) such as $N(CH_3)$, wherein the carbon atoms of the ring can be unsubstituted or mono- or polysubstituted. Preferably, the 4 to 10-membered heterocycloalkyl and the 4 to 6-membered heterocycloalkyl contain only one heteroatom or heteroatom group within the ring.

Preferably, 4 to 10-membered heterocycloalkyl and 4 to 6-membered heterocycloalkyl are saturated. The 4 to 10-membered heterocycloalkyl and 4 to 6-membered heterocycloalkyl are not condensed with further ring systems and are not bridged.

The 4 to 10-membered heterocycloalkyl and the 4 to 6-membered heterocycloalkyl group can be bound to the superordinate general structure via any desired and possible ring member of the heterocycloaliphatic residue if not indicated otherwise. In a preferred embodiment, 4 to 10-membered heterocycloalkyl and 4 to 6-membered heterocycloalkyl are bound to the superordinate general structure via a carbon atom.

Preferred 4 to 10-membered heterocycloalkyl groups are selected from the group consisting of oxetanyl, tetrahydrofuranyl, tetrahydropyranyl, morpholinyl, thiomorpholinyl, tetrahydrothiopyranyl, tetrahydrothiopyranyl 1,1-dioxide, oxepanyl, piperidinyl, piperidinonyl, azetidinyl, pyrrolidinyl, pyrrolidinonyl, 4-methylpiperazinyl, morpholinonyl, dioxanyl, piperazinyl, tetrahydropyrrolyl, azepanyl, dioxepanyl, oxazepanyl, diazepanyl, thiazolidinyl, tetrahydrothiophenyl, tetrahydropyridinyl, dithiolanyl, dihydropyrrolyl, dioxolanyl, dihydropyridinyl, dihydrofuranyl, dihydroisoxazolyl, dihydrooxazolyl, imidazolidinyl, isoxazolidinyl, oxazolidinyl, piperazinyl, N-methylpyridinonyl, pyrazolidinyl, pyranyl; dihydroquinolinyl, dihydroisoquinolinyl, dihydroindolinyl, dihydroisoindolyl, tetrahydroquinolinyl, tetrahydroisoquinolinyl and tetrahydroindolinyl; more preferably oxetanyl, tetrahydrofuranyl, tetrahydropyranyl, morpholinyl, thiomorpholinyl, tetrahydrothiopyranyl, tetrahydrothiopyranyl 1,1-dioxide, oxepanyl, piperidinyl, piperidinonyl, azetidinyl, pyrrolidinyl, pyrrolidinonyl, 4-methylpiperazinyl, morpholinonyl, dioxanyl, piperazinyl, and tetrahydropyrrolyl.

Preferred 4 to 6-membered heterocycloalkyl groups are selected from the group consisting of oxetanyl, tetrahydrofuranyl, tetrahydropyranyl, morpholinyl, thiomorpholinyl, tetrahydrothiopyranyl, tetrahydrothiopyranyl 1,1-dioxide, piperidinyl, piperidinonyl, azetidinyl, pyrrolidinyl, pyrrolidinonyl, 4-methylpiperazinyl, morpholinonyl, dioxanyl, piperazinyl, tetrahydropyrrolyl, thiazolidinyl, tetrahydrothiophenyl, tetrahydropyridinyl, dithiolanyl, dihydropyrrolyl, dioxolanyl, dihydropyridinyl, dihydrofuranyl, dihydroisoxazolyl, dihydrooxazolyl, imidazolidinyl, isoxazolidinyl, oxazolidinyl, N-methylpyridinonyl, pyrazolidinyl, and pyranyl; more preferably oxetanyl, tetrahydrofuranyl, tetrahydropyranyl, morpholinyl, thiomorpholinyl, tetrahydrothiopyranyl, tetrahydrothiopyranyl 1,1-dioxide, piperidinyl, piperidinonyl, azetidinyl, pyrrolidinyl, pyrrolidinonyl, 4-methylpiperazinyl, morpholinonyl, dioxanyl, piperazinyl, and tetrahydropyrrolyl.

According to the invention, the term "5 to 11-membered heterobicycloalkyl" preferably means bicyclic heteroaliphatic saturated or unsaturated (but not aromatic) hydrocarbons containing 5, 6, 7, 8, 9, 10 or 11 ring members, wherein in each case at least one, if appropriate also two or three carbon atoms are replaced by a heteroatom or a heteroatom group each selected independently of one another from the group consisting of O, S, S(=O), $S(=O)_2$, N, NH and $N(C_{1-4}$-alkyl) such as $N(CH_3)$, wherein the carbon atoms of the ring can be unsubstituted or mono- or polysubstituted. The bicyclic system may share a common bond (annealed rings) or may be bridged.

Preferably, 5 to 11-membered heterobicycloalkyl is saturated. The 5 to 11-membered heterobicycloalkyl can be bound to the respective superordinate general structure via any desired and possible ring member of the 5 to 11-membered heterobicycloalkyl group.

A preferred 5 to 11-membered heterobicycloalkyl group is 2-oxa-bicyclo[2.1.1]hexyl.

According to the invention, the term "5- to 10-membered heteroaryl" and "5- or 6-membered heteroaryl", respectively, preferably means a 5 to 10-membered or 5- or 6-membered cyclic aromatic residue containing at least 1, if appropriate also 2, 3, 4 or 5 heteroatoms, wherein the heteroatoms are each selected independently of one another from the group S, N and O and the heteroaryl residue can be unsubstituted or mono- or polysubstituted, if not indicated otherwise. In the case of substitution on the heteroaryl, the substituents can be the same or different and be in any desired and possible position of the heteroaryl. The binding to the superordinate general structure can be carried out via any desired and possible ring member of the heteroaryl residue if not indicated otherwise. Preferably, the 5- to 10-membered heteroaryl and 5- or 6-membered heteroaryl is bound to the suprordinate general structure via a carbon atom of the heterocycle. The heteroaryl can also be condensed with a further ring system, e.g. saturated or (partially) unsaturated (hetero)cyclic, aromatic or heteroaromatic ring systems, which can in turn be unsubstituted or mono- or polysubstituted, if not indicated otherwise.

Preferably, the 5- to 10-membered heteroaryl is selected from the group consisting of pyridyl (i.e. 2-pyridyl, 3-pyridyl, 4-pyridyl), pyrazolyl, pyrrolo[2,3-b]pyridyl, pyridonyl (pyridinonyl), thienyl (thiophenyl), thiazolyl, 2,3-dihydrobenzo[d]isothiazolyl 1,1-dioxide, isoindolinonyl, pyrimidinyl, pyrazinyl, pyridazinyl, isoxazolyl, triazolyl, oxazolyl, oxadiazolyl, indazolyl, pyrazolopyridyl, pyrrolyl, imidazolyl, isothiazolyl, furanyl, thiadiazolyl, N-methylpyridinonyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, dibenzothienyl, imidazothiazolyl, indolizinyl, indolyl, isoquinolinyl, naphthyridinyl, phenazinyl, phenothiazinyl, phthalazinyl, purinyl, phenazinyl, tetrazolyl and triazinyl; more preferably pyridyl, pyrazolyl, pyrrolo[2,3-b]pyridyl, pyridonyl, thienyl, thiazolyl, 2,3-dihydrobenzo[d]isothiazolyl 1,1-dioxide, isoindolinonyl, pyrimidinyl, pyrazinyl, pyridazinyl, isoxazolyl, triazolyl, oxazolyl, oxadiazolyl, indazolyl, pyrazolopyridyl, pyrrolyl, imidazolyl, isothiazolyl, furanyl, and thiadiazolyl. Particularly preferably, 5 to 10-membered heteroaryl is selected from 5- or 6-membered heteroaryl.

Preferably, the 5- or 6-membered heteroaryl is selected from the group consisting of pyridyl, pyrazolyl, pyridonyl, thienyl, thiazolyl, pyrimidinyl, pyrazinyl, pyridazinyl, isoxazolyl, triazolyl, oxazolyl, oxadiazolyl, pyrrolyl, imidazolyl, isothiazolyl, furanyl, thiadiazolyl, N-methylpyridinonyl, tetrazolyl and triazinyl; more preferably pyridyl, pyrazolyl, pyridonyl, thienyl, thiazolyl, pyrimidinyl, pyrazinyl, pyridazinyl, isoxazolyl, triazolyl, oxazolyl, oxadiazolyl, pyrrolyl, imidazolyl, isothiazolyl, furanyl, and thiadiazolyl.

As pyridones and 2-hydroxypyridine are tautomers, for the purpose of the specification the definition of pyridines that may optionally be substituted with OH covers pyridones.

In connection with the terms "$C_{1-6}$-alkyl", "$C_{1-4}$-alkyl", "$C_{1-6}$-alkylene", "$C_{1-4}$-alkylene", "$C_{3-10}$-cycloalkyl", "$C_{3-6}$-cycloalkyl", "4 to 11-membered bicycloalkyl", "5 to 11-membered spiroalkyl or dispiroalkyl", "4 to 10-membered heterocycloalkyl", "4 to 6-membered heterocycloalkyl", and "5 to 11-membered heterobicycloalkyl", the term "substituted" refers in the sense of the invention, with respect to the corresponding residues or groups, to the single substitution (monosubstitution) or multiple substitution (polysubstitution), e.g. disubstitution, trisubstitution or tetrasubstitution; more preferably to monosubstitution, disubstitution or trisubstitution; of one or more hydrogen atoms each independently of one another by at least one substituent. In case of a multiple substitution, i.e. in case of polysubstituted residues, such as di- or trisubstituted residues, these residues may be polysubstituted either on different or on the same atoms, for example trisubstituted on the same carbon atom, as in the case of $CF_3$, $CH_2CF_3$ or disubstituted as in the case of 1,1-difluorocyclohexyl, or at various points, as in the case of 1-chloro-3-fluorocyclohexyl. The multiple substitution can be carried out using the same or using different substituents.

If a residue occurs multiply within a molecule, then this residue can have respectively different meanings for various substituents: if, for example, both **R4** and **R5** denote $C_{1-6}$-alkyl, then $C_{1-6}$-alkyl can e.g. represent methyl for **R4** and can represent 2-propyl for **R5.**

According to the invention, $C_{1-6}$-alkyl, $C_{1-4}$-alkyl, $C_{1-6}$-alkylene, $C_{1-4}$-alkylene, $C_{3-10}$-cycloalkyl, $C_{3-6}$-cycloalkyl, 4 to 11-membered bicycloalkyl, 5 to 11-membered spiroalkyl or dispiroalkyl, 4 to 10-membered heterocycloalkyl, 4 to 6-membered heterocycloalkyl, and 5 to 11-membered heterobicycloalkyl in each case independently from one another are unsubstituted or mono- or polysubstituted, more preferably unsubstituted or substituted with one, two, three, four or more substituents independently from one another selected from the group consisting of F, Cl, CN, $C_{1-6}$-alkyl, $C_{1-6}$-alkylene-OH, $C_{1-6}$-alkylene-$OCH_3$, $CF_3$, $CF_2H$, $CFH_2$, C(O)-$C_{1-6}$-alkyl, OH, =O, $OCF_3$, $OCF_2H$, $OCFH_2$, O-$C_{1-6}$-alkyl, $C_{1-4}$-alkylene-O-$C_{1-4}$-alkylene-O-$CH_3$, $C_{0-4}$-alkylene-O-($C_{1-4}$-alkylene-O)$_{1-4}$-$CH_3$, $NH_2$, NH-$C_{1-6}$-alkyl, or N($C_{1-6}$-alkyl)$_2$; preferably $CH_3$, $CF_3$, $CF_2H$, $CFH_2$, and $OCH_3$; more preferably $CF_3$, $CF_2H$, and $CFH_2$; and most preferably $CF_3$.

Preferably, $C_{1-6}$-alkylene groups are unsubstituted.

According to the invention, phenyl, 5 to 10-membered heteroaryl and 5 or 6-membered heteroaryl in each case independently from one another are unsubstituted or mono- or polysubstituted, more preferably unsubstituted or mono- or disubstituted, with one or more substituents selected from the group consisting of F, Cl, Br, CN, $C_{1-6}$-alkyl, $C_{3-6}$-cycloalkyl, $CF_3$, $CF_2H$, $CFH_2$, $C_{1-6}$-alkylene-$CF_3$, $C_{1-6}$-alkylene-$CF_2H$, $C_{1-6}$-alkylene-$CFH_2$, $C_{1-6}$-alkylene-OH,

$C_{1-6}$-alkylene-$OCH_3$, C(O)-$C_{1-6}$-alkyl, $OCF_3$, $OCF_2H$, $OCFH_2$, and O-$C_{1-6}$-alkyl; preferably F, $CH_3$, $CF_3$, $CF_2H$, $CFH_2$, and $OCH_3$; more preferably F, $CF_3$, $CF_2H$, and $CFH_2$; and most preferably F.

According to the invention,

(a) one of **A1**, **A2** and **A3** represents C-**R3**; another one of **A1**, **A2** and **A3** represents C-**R'**; and the remaining one of **A1**, **A2** and **A3** represents $N^+$-$O^-$ (pyridine N-oxides); or

(b) one of **A1**, **A2** and **A3** represents C-**R3'**; another one of **A1**, **A2** and **A3** represents C-**R'**; and the remaining one of **A1**, **A2** and **A3** represents N or C**R'** (pyridines or phenyls).

In preferred embodiments,

(i) **A1** represents C**R3**; **A2** represents $N^+$-$O^-$; and **A3** represents C**R'**, preferably CH;

(ii) **A1** represents C**R3**'; **A2** represents N or CH; and **A3** represents C**R'**, preferably CH; or

(iii) **A1** represents N or CH; **A2** represents C**R3'**; and **A3** represents C**R'**, preferably CH.

According to the invention, **R'** independently represents H, F, $CH_3$, $CH_2F$, $CHF_2$, $CF_3$, or $OCH_3$.

In preferred embodiments, **R'** represents H.

According to the invention, **L** represents $CH_2$, $CH(CH_3)$ or $CH(CH_2CH_3)$.

In preferred embodiments, **L** represents $CH_2$.

According to the invention, **R1** represents $C_{3-10}$-cycloalkyl, 5 to 11-membered spiroalkyl or dispiroalkyl, 4 to 11-membered bicycloalkyl, 4 to 10-membered heterocycloalkyl, or 5 to 11-membered heterobicycloalkyl.

In preferred embodiments, **R1** represents $C_{3-10}$-cycloalkyl, unsubstituted or substituted with one, two, three, four, or more substituents independently from one another selected from F, $CH_3$, $OCH_3$, CN, $CHF_2$ and $CF_3$.

Preferably, **R1** is selected from cyclopropyl, methyl cyclopropyl, dimethyl cyclopropyl, fluoro cyclopropyl, difluoro cyclopropyl, trifluoromethyl cyclopropyl, trifluoromethyl methyl cyclopropyl, methyl difluoro cyclopropyl, trifluoromethyl difluoro cyclopropyl, dimethyl difluoro cyclopropyl, cyclobutyl, methyl cyclobutyl, dimethyl cyclobutyl, fluoro cyclobutyl, difluoro cyclobutyl, trifluoromethyl cyclobutyl, trifluoromethyl methyl cyclobutyl, methyl difluoro cyclobutyl, trifluoromethyl difluoro cyclobutyl, dimethyl difluoro cyclobutyl, cyclopentyl, methyl cyclopentyl, dimethyl cyclopentyl, fluoro cyclopentyl, difluoro cyclopentyl, trifluoromethyl cyclopentyl, trifluoromethyl methyl cyclopentyl, methyl difluoro cyclopentyl, trifluoromethyl difluoro cyclopentyl, dimethyl difluoro cyclopentyl, cyclohexyl, methyl cyclohexyl, dimethyl cyclohexyl, fluoro cyclohexyl, difluoro cyclohexyl, trifluoromethyl cyclohexyl, trifluoromethyl methyl cyclohexyl, methyl difluoro cyclohexyl, trifluoromethyl difluoro cyclohexyl, dimethyl difluoro cyclohexyl, cycloheptyl, methyl cycloheptyl, dimethyl cycloheptyl, fluoro cycloheptyl, difluoro cycloheptyl, trifluoromethyl cycloheptyl, trifluoromethyl methyl cycloheptyl, methyl difluoro cycloheptyl, trifluoromethyl difluoro cycloheptyl, dimethyl difluoro cycloheptyl, cyclooctyl, methyl cyclooctyl, dimethyl cyclooctyl, fluoro cyclooctyl, difluoro cyclooctyl, trifluoromethyl cyclooctyl, trifluoromethyl methyl cyclooctyl, methyl difluoro cyclooctyl, trifluoromethyl difluoro cyclooctyl, dimethyl difluoro cyclooctyl, cyclononyl, methyl cyclononyl, dimethyl cyclononyl, fluoro cyclononyl, difluoro cyclononyl, trifluoromethyl cyclononyl, trifluoromethyl methyl cyclononyl, methyl difluoro cyclononyl, trifluoromethyl difluoro cyclononyl, dimethyl difluoro cyclononyl, cyclodecyl, methyl cyclodecyl, dimethyl cyclodecyl, fluoro cyclodecyl, difluoro cyclodecyl, trifluoromethyl cyclodecyl, trifluoromethyl methyl cyclodecyl, methyl difluoro cyclodecyl, trifluoromethyl difluoro cyclodecyl, and dimethyl difluoro cyclodecyl.

More preferably, **R1** is selected from dimethyl cyclopropyl, trifluoromethyl methyl cyclopropyl, trifluoromethyl cyclobutyl, difluoro cyclobutyl, methyl difluoro cyclobutyl, trifluoromethyl cyclopentyl, difluoro cyclopentyl, methyl difluoro cyclopentyl, and trifluoromethyl difluoro cyclopentyl.

In preferred embodiments, **R1** represents $C_{3-10}$-cycloalkyl, substituted with $CH_3$ and optionally substituted with one, two, three, four, or more substituents independently from one another selected from F, $OCH_3$, CN, $CHF_2$ and $CF_3$, wherein the carbon atom of $C_{3-10}$-cycloalkyl, which is substituted with $CH_3$, is linked to **L**. Preferred representatives include but are not limited to methyl difluoro cyclobutyl and methyl difluoro cyclopentyl.

In further preferred embodiments, **R1** represents 4 to 11-membered bicycloalkyl, unsubstituted or substituted with one, two, three, four, or more substituents independently from one another selected from F, $CH_3$, $OCH_3$, CN, $CHF_2$ and $CF_3$.

Preferably, **R1** is selected from bicylo[1.1.1]pentyl, methyl bicylo[1.1.1]pentyl, methoxy bicylo[1.1.1]pentyl, difluoromethyl bicylo[1.1.1]pentyl, trifluoromethyl bicylo[1.1.1]pentyl, fluoro bicylo[1.1.1]pentyl, difluoro bicylo[1.1.1]pentyl, methyl difluoro bicylo[1.1.1]pentyl, trifluoromethyl difluoro bicylo[1.1.1]pentyl, bicyclo[2.1.0]pentyl, methyl bicyclo[2.1.0]pentyl, trifluoromethyl bicyclo[2.1.0]pentyl, fluoro bicyclo[2.1.0]pentyl, difluoro bicyclo [2.1.0]pentyl, methyl difluoro bicyclo[2.1.0]pentyl, trifluoromethyl difluoro bicyclo[2.1.0]pentyl, bicyclo[3.1.0]hexyl, methyl bicyclo[3.1.0]hexyl,

trifluoromethyl bicyclo[3.1.0]hexyl, fluoro bicyclo[3.1.0]hexyl, difluoro bicyclo[3.1.0]hexyl, methyl difluoro bicyclo[3.1.0]hexyl, trifluoromethyl difluoro bicyclo[3.1.0]hexyl, bicyclo[2.2.0]hexyl, methyl bicyclo[2.2.0]hexyl, trifluoromethyl bicyclo[2.2.0]hexyl, fluoro bicyclo[2.2.0]hexyl, difluoro bicyclo[2.2.0]hexyl, methyl difluoro bicyclo[2.2.0]hexyl, trifluoromethyl difluoro bicyclo[2.2.0]hexyl, bicyclo[2.1.1]hexyl, methyl bicyclo[2.1.1]hexyl, trifluoromethyl bicyclo[2.1.1]hexyl, fluoro bicyclo[2.1.1]hexyl, difluoro bicyclo[2.1.1]hexyl, fluoro bicyclo[2.1.1]hexyl, difluoro bicyclo[2.1.1] hexyl, methyl difluoro bicyclo[2.1.1]hexyl, trifluoromethyl difluoro bicyclo[2.1.1]hexyl, bicyclo[4.1.0]heptyl, methyl bicyclo[4.1.0]heptyl, trifluoromethyl bicyclo[4.1.0]heptyl, fluoro bicyclo[4.1.0]heptyl, difluoro bicyclo[4.1.0]heptyl, methyl difluoro bicyclo[4.1.0]heptyl, trifluoromethyl difluoro bicyclo[4.1.0]heptyl, bicyclo[2.2.1]heptyl, methyl bicyclo[2.2.1]heptyl, trifluoromethyl bicyclo[2.2.1]heptyl, fluoro bicyclo[2.2.1]heptyl, difluoro bicyclo[2.2.1]heptyl, methyl difluoro bicyclo[2.2.1]heptyl, trifluoromethyl difluoro bicyclo[2.2.1]heptyl, bicyclo[3.3.0]octyl, methyl bicyclo[3.3.0]octyl, trifluoromethyl bicyclo[3.3.0]octyl, fluoro bicyclo[3.3.0]octyl, difluoro bicyclo[3.3.0]octyl, methyl difluoro bicyclo[3.3.0]octyl, and trifluoromethyl difluoro bicyclo[3.3.0]octyl.

More preferably, **R1** is selected from bicyclo[2.1.0]pentyl, difluoro bicyclo[2.1.0]pentyl, methyl bicyclo[2.2.0]hexyl, difluoro bicyclo[3.1.0]hexyl, methyl difluoro bicyclo[3.1.0]hexyl, trifluoromethyl difluoro bicyclo[3.1.0]hexyl, fluoro bicyclo[2.1.1]hexyl, difluoro bicyclo[2.1.1]hexyl, difluoro bicyclo[4.1.0]heptyl, fluoro bicyclo[2.2.1]heptyl, and fluoro bicyclo[3.3.0]octyl.

In other preferred embodiments, **R1** represents 5 to 11-membered spiroalkyl or dispiroalkyl, unsubstituted or substituted with one, two, three, four, or more substituents independently from one another selected from F, $CH_3$, $OCH_3$, CN, $CHF_2$ and $CF_3$.

Preferably, **R1** is selected from spiro[2.2]pentyl, methyl spiro[2.2]pentyl, fluoro spiro[2.2]pentyl, difluoro spiro[2.2]pentyl, methyl difluoro spiro[2.2]pentyl, spiro[2.3]hexyl, methyl spiro[2.3]hexyl, fluoro spiro[2.3]hexyl, difluoro spiro[2.3]hexyl, methyl difluoro spiro[2.3]hexyl, spiro[3.3]heptyl, methyl spiro[3.3]heptyl, fluoro spiro[3.3]heptyl, difluoro spiro[3.3]heptyl, methyl difluoro spiro[3.3]heptyl, and dispiro[2.0.2.1]heptyl.

More preferably, **R1** is selected from spiro[2.2]pentyl, difluoro spiro[2.2]pentyl, spiro[2.3]hexyl, methyl spiro[2.3]hexyl, fluoro spiro[2.3]hexyl, methyl difluoro spiro[2.3]hexyl, spiro[3.3]heptyl, and dispiro[2.02.1]heptyl.

In still further preferred embodiments, **R1** represents 4 to 10-membered heterocycloalkyl, unsubstituted or substituted with one, two, three, four, or more substituents independently from one another selected from F, $CH_3$, $OCH_3$, CN, $CHF_2$ and $CF_3$.

Preferably, **R1** is selected from oxetanyl, tetrahydrofuranyl, tetrahydropyranyl, morpholinyl, thiomorpholinyl, tetrahydrothiopyranyl, tetrahydrothiopyranyl 1,1-dioxide, oxepanyl, piperidinyl, piperidinonyl, azetidinyl, pyrrolidinyl, pyrrolidinonyl, 4-methylpiperazinyl, morpholinonyl, dioxanyl, piperazinyl, tetrahydropyrrolyl, azepanyl, dioxepanyl, oxazepanyl, diazepanyl, thiazolidinyl, tetrahydrothiophenyl, tetrahydropyridinyl, dithiolanyl, dihydropyrrolyl, dioxolanyl, dihydropyridinyl, dihydrofuranyl, dihydroisoxazolyl, dihydrooxazolyl, imidazolidinyl, isoxazolidinyl, oxazolidinyl, piperazinyl, N-methylpyridinonyl, pyrazolidinyl, pyranyl; dihydroquinolinyl, dihydroisoquinolinyl, dihydroindolinyl, dihydroisoindolyl, tetrahydroquinolinyl, tetrahydroisoquinolinyl and tetrahydroindolinyl.

More preferably, **R1** represents tetrahydropyranyl.

In yet further preferred embodiments, **R1** represents 5 to 11-membered heterobicycloalkyl, unsubstituted or substituted with one, two, three, four, or more substituents independently from one another selected from F, $CH_3$, $OCH_3$, CN, $CHF_2$ and $CF_3$.

Preferably, **R1** represents 2-oxa-bicyclo[2.1.1]hexyl or methyl 2-oxa-bicyclo[2.1.1]hexyl.

According to the invention, **R2** represents H or $C_{1-6}$-alkyl.

In preferred embodiments, **R2** represents H.

According to the invention, **R3** represents C(O)-$NH_2$, C(O)-N(H)($C_{1-6}$-alkyl), C(O)-N(H)($C_{3-6}$-cycloalkyl), C(O)-N($C_{1-6}$-alkyl)$_2$, C(O)-N($C_{1-6}$-alkyl)($C_{3-6}$-cycloalkyl), $S(O)_2$-$C_{1-6}$-alkyl, $S(O)_2$-($C_{3-6}$-cycloalkyl), $S(O)_2$-(4 to 6-membered heterocycloalkyl), $S(O)_2$-phenyl, $S(O)_2$-(5 or 6-membered heteroaryl), S(O)-$NH_2$, S(O)-N(H)($C_{1-6}$-alkyl), S(O)-N($C_{1-6}$-alkyl)$_2$, $S(O)_2$-$NH_2$, $S(O)_2$-N(H)($C_{1-6}$-alkyl), $S(O)_2$-N(H)($C_{3-6}$-cycloalkyl), $S(O)_2$-N($C_{1-6}$-alkyl)$_2$, S(O)-$C_{1-6}$-alkyl, S(O)-($C_{3-6}$-cycloalkyl), S(O)-(4 to 6-membered heterocycloalkyl), S(O)-phenyl, S(O)-(5 or 6-membered heteroaryl), $OCF_3$, $OCF_2H$, CN, OH, O-$C_{3-6}$-cycloalkyl, O-(4 to 6-membered heterocycloalkyl), or S(O)(N**R3a**)**R3b**, wherein **R3a** represents H, $C_{1-6}$-alkyl, C(O)$C_{1-5}$-alkyl, C(O)$C_{1-5}$-alkyl-$NH_2$, C(O)$C_{1-5}$-alkyl-NH-$CH_3$, C(O)$C_{1-5}$-alkyl-N($CH_3$)$_2$, $C_{3-10}$-cycloalkyl, $C_{1-6}$-alkylene-$C_{3-10}$-cycloalkyl, 4 to 10-membered heterocycloalkyl, or $C_{1-6}$-alkylene-(4 to 10-membered heterocycloalkyl); **R3b** represents $NH_2$, $C_{1-6}$-alkyl, $C_{3-10}$-cycloalkyl, $C_{1-6}$-alkylene-$C_{3-10}$-cycloalkyl, 4 to 10-membered heterocycloalkyl, or $C_{1-6}$-alkylene-(4 to 10-membered heterocycloalkyl); or **R3a** and **R3b** mean $(CH_2)_{3-5}$ and together with the atoms to which they are attached form a ring.

In preferred embodiments, **R3** represents C(O)-$NH_2$, $S(O)_2$-$C_{1-6}$-alkyl, $S(O)_2$-$NH_2$, or S(O)(N**R3a**)**R3b**.

Preferably, **R3** represents C(O)-$NH_2$, S(O)(NH)$CH_3$, or $S(O)_2$-$NH_2$.

According to the invention, **R3'** represents C(O)OH, C(O)O$C_{1-6}$-alkyl, or P(O)($C_{1-6}$-alkyl)$_2$.

Preferably, **R3'** represents C(O)OH, C(O)O$CH_3$, or P(O)($CH_3$)$_2$.

According to the invention, **R4** represents H, F, Cl, Br, CN, $CHF_2$, $CH_2F$, $CF_3$, $C_{1-6}$-alkyl, $C_{3-10}$-cycloalkyl,

$C_{1-6}$-alkylene-$C_{3-10}$-cycloalkyl, 4 to 11-membered bicycloalkyl, 5 to 11-membered spiroalkyl or bisspiroalkyl, 4 to 10-membered heterocycloalkyl, $C_{1-6}$-alkylene-(4 to 10-membered heterocycloalkyl), $NH_2$, N(H)($C_{1-6}$-alkyl), N($C_{1-6}$-alkyl)$_2$, O-$C_{1-6}$-alkyl, O-$C_{3-10}$-cycloalkyl, O-$C_{1-6}$-alkylene-$C_{3-10}$-cycloalkyl, O-(4 to 6-membered heterocycloalkyl), or O-$C_{1-6}$-alkylene-(4 to 6-membered heterocycloalkyl).

In preferred embodiments, **R4** represents H.

In further preferred embodiments, **R4** represents $C_{1-6}$-alkyl.

Preferably, **R4** represents $CHF_2$, $CH_2F$, $CF_3$, $CH_2CHF_2$, $CH_2CH_2F$, $CH_2CF_3$, $CF_2CH_3$, $CHFCH_3$, $CF_2CF_3$, $CHFCF_3$, $CH(CHF_2)(CH_3)$, $CH(CH_2F)(CH_3)$, $CH(CF_3)(CH_3)$, $CH_3$, $CH_2CH_3$, or $CH(CH_3)_2$.

More preferably, **R4** represents $CF_2CH_3$.

In other preferred embodiments, **R4** represents $C_{3-10}$-cycloalkyl, unsubstituted or substituted with one, two, three, four, or more substituents independently from one another selected from F, $CH_3$, $OCH_3$, CN, $CHF_2$ and $CF_3$.

Preferably, **R4** is selected from cyclopropyl, methyl cyclopropyl, difluoromethyl cyclopropyl, trifluoromethyl cyclopropyl, cyano cyclopropyl, methoxy cyclopropyl, fluoro cyclopropyl, difluoro cyclopropyl, trifluoro cyclopropyl, cyclobutyl, methyl cyclobutyl, difluoromethyl cyclobutyl, trifluoromethyl cyclobutyl, cyano cyclobutyl, methoxy cyclobutyl, fluoro cyclobutyl, difluoro cyclobutyl, trifluoro cyclobutyl, cyclopentyl, methyl cyclopentyl, difluoromethyl cyclopentyl, trifluoromethyl cyclopentyl, cyano cyclopentyl, methoxy cyclopentyl, fluoro cyclopentyl, difluoro cyclopentyl, trifluoro cyclopentyl, cyclohexyl, methyl cyclohexyl, difluoromethyl cyclohexyl, trifluoromethyl cyclohexyl, cyano cyclohexyl, methoxy cyclohexyl, fluoro cyclohexyl, difluoro cyclohexyl, and trifluoro cyclohexyl.

More preferably, **R4** is selected from cyclopropyl, methyl cyclopropyl, cyano cyclopropyl, methoxy cyclopropyl, difluoromethyl cyclopropyl, trifluoromethyl cyclopropyl, fluoro cyclopropyl, difluoro cclopropyl, trifluoro cyclopropyl, cyclobutyl, difluoro cyclobutyl, cyclopentyl, and difluorocyclopentyl.

In still further preferred embodiments, **R4** represents 4 to 11-membered bicycloalkyl, unsubstituted or substituted with one, two, three, four, or more substituents independently from one another selected from F, $CH_3$, $OCH_3$, CN, $CHF_2$ and $CF_3$.

Preferably, **R4** represents bicyclo[1.1.1]pentyl.

In yet further preferred embodiments, **R4** represents 5 to 11-membered spiroalkyl, unsubstituted or substituted with one, two, three, four, or more substituents independently from one another selected from F, $CH_3$, $OCH_3$, CN, $CHF_2$ and $CF_3$.

Preferably, **R4** represents spiro[2.2]pentyl or spiro[2.3]hexyl.

In other preferred embodiments, **R4** represents O-$C_{1-6}$-alkyl, unsubstituted or substituted with one, two, three, four, or more F.

Preferably, **R4** is selected from O-$CHF_2$, O-$CH_2F$, O-$CF_3$, O-$CH_2CHF_2$, O-$CH_2CH_2F$, O-$CH_2CF_3$, O-$CF_2CH_3$, O-$CHFCH_3$, O-$CF_2CF_3$, O-$CHFCF_3$, O-$CH_3$, O-$CH_2CH_3$, or O-$CH(CH_3)_2$.

According to the invention, **R5** represents H, F, Cl, Br, CN, $CHF_2$, $CH_2F$, $CF_3$, $C_{1-6}$-alkyl, $C_{3-10}$-cycloalkyl, $C_{1-6}$-alkylene-$C_{3-10}$-cycloalkyl, 4 to 10-membered heterocycloalkyl, $C_{1-6}$-alkylene-(4 to 10-membered heterocycloalkyl), $NH_2$, N(H)($C_{1-6}$-alkyl), N($C_{1-6}$-alkyl)$_2$, O-$C_{1-6}$-alkyl, O-$C_{3-10}$-cycloalkyl, O-$C_{1-6}$-alkylene-$C_{3-10}$-cycloalkyl, O-(4 to 6-membered heterocycloalkyl), or O-$C_{1-6}$-alkylene-(4 to 6-membered heterocycloalkyl).

In preferred embodiments, **R5** represents $C_{1-6}$-alkyl or Cl.

Preferably, **R5** is selected from $CHF_2$, $CH_2F$, $CF_3$, $CH_2CHF_2$, $CH_2CH_2F$, $CH_2CF_3$, $CF_2CH_3$, $CHFCH_3$, $CF_2CF_3$, $CHFCF_3$, $CH(CHF_2)(CH_3)$, $CH(CH_2F)(CH_3)$, $CH(CF_3)(CH_3)$, $CH_3$, $CH_2CH_3$, $CH(CH_3)_2$ or Cl.

More preferably, **R5** represents $CF_3$, $CHF_2$, $CH_3$ or Cl.

In a particularly preferred embodiment, the compound according to the invention is selected from the group consisting of

| Ex | Name |
|---|---|
| 1 | 2-carbamoyl-4-(1-((3,3 -difluorocyclopentyl)methyl)-3-(1,1-difluoroethyl)-4-methyl-1H-pyrazole-5-carboxamido)pyridine 1-oxide |
| 3 | 2-carbamoyl-4-(1-((3,3-difluorocyclobutyl)methyl)-3-(1,1-difluoroethyl)-4-methyl-1H-pyrazole-5-carboxamido) pyridine 1-oxide |
| 4 | 2-carbamoyl-4-(1-((3,3-difluoro-1-methylcyclobutyl)methyl)-3-(1,1-difluoroethyl)-4-methyl-1H-pyrazole-5-carboxamido)pyridine 1-oxide |
| 5 | 2-carbamoyl-4-(1-((3,3-difluoro-1-methylcyclopentyl)methyl)-3-(1,1-difluoroethyl)-4-methyl-1H-pyrazole-5-carboxamido)pyridine 1-oxide |
| 7 | 4-(1-((3,3-difluorocyclobutyl)methyl)-3-(1,1-difluoroethyl)-4-(trifluoromethyl)-1H-pyrazole-5-carboxamido) picolinic acid |
| 8 | 5-(1-((3,3-difluorocyclobutyl)methyl)-3-(1,1-difluoroethyl)-4-(trifluoromethyl)-1H-pyrazole-5-carboxamido) picolinic acid |

(continued)

| Ex | Name |
|---|---|
| 9 | 4-(3-cyclopropyl-1-((4,4-difluorocycloheayl)methyl)-4-(trifluoromethyl)-1H-pyrazole-5-carboxamido)picolinic acid |
| 10 | 1-(cyclohexylmethyl)-N-(2-(dimethylphosphoryl)pyridin-4-yl)-4-(trifluoromethyl)-1H-pyrazole-5-carboxamide |
| 11 | 4-(3-cyclopropyl-1-((3,3-difluoro-1-methylcyclobutyl)methyl)-4-(trifluoromethyl)-1H-pyrazole-5-carboxamido)-2-(S-methylsulfonimidoyl)pyridine 1-oxide |
| 12 | 4-(3-cyclopropyl-1-((3,3-difluoro-1-methylcyclopentyl)methyl)-4-(trifluoromethyl)-1H-pyrazole-5-carboxamido)-2-(S-methylsulfonimidoyl)pyridine 1-oxide |
| 13 | 4-(1-((3,3-difluoro-1-methylcyclobutyl)methyl)-3-(1,1-difluoroethyl)-4-methyl-1H-pyrazole-5-carboxamido)-2-(S-methylsulfonimidoyl)pyridine 1-oxide |
| 14 | 4-(1-((3,3-difluoro-1-methylcyclopentyl)methyl)-3-(1,1-difluoroethyl)-4-methyl-1H-pyrazole-5-carboxamido)-2-(S-methylsulfonimidoyl)pyridine 1-oxide |
| 15 | 4-(3-cyclobutyl-1-((3,3-difluoro-1-methylcyclobutyl)methyl)-4-(trifluoromethyl)-1H-pyrazole-5-carboxamido)-2-(S-methylsulfonimidoyl)pyridine 1-oxide |
| 16 | 4-(1-((3,3-difluoro-1-methylcyclobutyl)methyl)-3-(3,3-difluorocyclobutyl)-4-(trifluoromethyl)-1H-pyrazole-5-carboxamido)-2-(S-methylsulfonimidoyl)pyridine 1-oxide |
| 17 | 4-(3-(bicyclo[1.1.1]pentan-1yl)-1-((3,3-difluoro-1-methylcyclobutyl)methyl)-4-(trifluoromethyl)-1H-pyrazole-5-carboxamido)-2-(S-methylsulfonimidoyl)pyridine 1-oxide |
| 18 | 4-(3-cyclopentyl-1-((3,3-difluoro-1-methylcyclobutyl)methyl)-4-(trifluoromethyl)-1H-pyrazole-5-carboxamido)-2-(S-methylsulfonimidoyl)pyridine 1-oxide |
| 19 | 4-(1-((3,3-difluoro-1-methylcyclobutyl)methyl)-3-(3,3-difluorocyclopentyl)-4-(trifluoromethyl)-1H-pyrazole-5-carboxamido)-2-(S-methylsulfonimidoyl)pyridine 1-oxide |
| 20 | 4-(1-((3,3-difluoro-1-methylcyclobutyl)methyl)-3-(spiro[2.3]hexan-5-yl)-4-(trifluoromethyl)-1H-pyrazole-5-carboxamido)-2-(S-methylsulfonimidoyl)pyridine 1-oxide |
| 21 | 4-(1-((3,3-difluoro-1-methylcyclobutyl)methyl)-3-(2-fluorocyclopropyl)-4-(trifluoromethyl)-1H-pyrazole-5-carboxamido)-2-(S-methylsulfonimidoyl)pyridine 1-oxide |
| 22 | 4-(1-((3,3-difluoro-1-methylcyclobutyl)methyl)-3-(2,2-difluorocyclopropyl)-4-(trifluoromethyl)-1H-pyrazole-5-carboxamido)-2-(S-methylsulfonimidoyl)pyridine 1-oxide |
| 23 | 4-(1-((3,3-difluoro-1-methylcyclobutyl)methyl)-3-(spiro[2.2]pentan-1-yl)-4-(trifluoromethyl)-1H-pyrazole-5-carboxamido)-2-(S-methylsulfonimidoyl)pyridine 1-oxide |
| 24 | 4-(1-((3,3-difluoro-1-methylcyclobutyl)methyl)-3-(2-methylcyclopropyl)-4-(trifluoromethyl)-1H-pyrazole-5-carboxamido)-2-(S-methylsulfonimidoyl)pyridine 1-oxide |
| 25 | 4-(1-((3,3-difluoro-1-methylcyclobutyl)methyl)-3-(2-(difluoromethyl)cyclopropyl)-4-(trifluoromethyl)-1H-pyrazole-5-carboxamido)-2-(S-methylsulfonimidoyl)pyridine 1-oxide |
| 26 | 4-(1-((3,3-difluoro-1-methylcyclobutyl)methyl)-4-(trifluoromethyl)-3 -(2-(trifluoromethyl)cyclopropyl)-1H-pyrazole-5-carboxamido)-2-(S-methylsulfonimidoyl)pyridine 1-oxide |
| 27 | 4-(3-(1-cyanocyclopropyl)-1-((3,3-difluoro-1-methylcyclobutyl)methyl)-4-(trifluoromethyl)-1H-pyrazole-5-carboxamido)-2-(S-methylsulfonimidoyl)pyridine 1-oxide |
| 28 | 4-(1-((3,3-difluoro-1-methylcyclobutyl)methyl)-3-(1-methylcyclopropyl)-4-(trifluoromethyl)-1H-pyrazole-5-carboxamido)-2-(S-methylsulfonimidoyl)pyridine 1-oxide |
| 29 | 4-(1-((3,3-difluoro-1-methylcyclobutyl)methyl)-3-(1-fluorocyclopropyl)-4-(trifluoromethyl)-1H-pyrazole-5-carboxamido)-2-(S-methylsulfonimidoyl)pyridine 1-oxide |
| 30 | 4-(1-((3,3-difluoro-1-methylcyclobutyl)methyl)-3-(1-methoxycyclopropyl)-4-(trifluoromethyl)-1H-pyrazole-5-carboxamido)-2-(S-methylsulfonimidoyl)pyridine 1-oxide |
| 31 | 4-(1-((3,3-difluoro-1-methylcyclobutyl)methyl)-3-(2,3-difluorocyclopropyl)-4-(trifluoromethyl)-1H-pyrazole-5-carboxamido)-2-(S-methylsulfonimidoyl)pyridine 1-oxide |
| 32 | 4-(1-((3,3-difluoro-1-methylcyclobutyl)methyl)-3-(1,2,2-trifluorocyclopropyl)-4-(trifluoromethyl)-1H-pyrazole-5-carboxamido)-2-(S-methylsulfonimidoyl)pyridine 1-oxide |
| 33 | 4-(1-((3,3-difluoro-1-methylcyclobutyl)methyl)-3-(1,2-difluorocyclopropyl)-4-(trifluoromethyl)-1H-pyrazole-5-carboxamido)-2-(S-methylsulfonimidoyl)pyridine 1-oxide |
| 34 | 4-(1-((3,3-difluoro-1-methylcyclobutyl)methyl)-3-(1,1-difluoroethyl)-4-(trifluoromethyl)-1H-pyrazole-5-carboxamido)-2-(S-methylsulfonimidoyl)pyridine 1-oxide |

(continued)

| Ex | Name |
|---|---|
| 35 | 4-(4-chloro-1-((3,3-difluoro-1-methylcyclobutyl)methyl)-3-(1,1-difluoroethyl)-1H-pyrazole-5-carboxamido)-2-(S-methylsulfonimidoyl)pyridine 1-oxide |
| 36 | 4-(3-cyclopropyl-1-((3,3-difluoro-1-methylcyclobutyl)methyl)-4-(difluoromethyl)-1H-pyrazole-5-carboxamido)-2-(S-methylsulfonimidoyl)pyridine 1-oxide |
| 38 | 4-(3-cyclopropyl-1-(spiro[2.2]pentan-1ylmethyl)-4-(trifluoromethyl)-1H-pyrazole-5-carboxamido)-2-(S-methylsulfonimidoyl)pyridine1-oxide |
| 39 | 4-(3-cyclopropyl-1-((2,2-difluorospiro [22]pentan-1-yl)methyl)-4-(trifluoromethyl)-1H-pyrazole-5-carboxamido)-2-(S-methylsulfonimidoyl)pyridine 1-oxide |
| 40 | 4-(3-cyclopropyl-1-(dispiro[2.0.24.13]heptan-7-ylmethyl)-4-(trifluoromethyl)-1H-pyrazole-5-carboxamido)-2-(S-methylsulfonimidoyl)pyridine 1-oxide |
| 41 | 4-(3-cyclopropyl-1-((1,2-dimethylcyclopropyl)methyl)-4-(trifluoromethyl)-1H-pyrazole-5-carboxamido)-2-(S-methylsulfonimidoyl)pyridine 1-oxide |
| 42 | 4-(3-cyclopropyl-1-((1-methyl-2-(trifluoromethyl)cyclopropyl)methyl)-4-(trifluoromethyl)-1H-pyrazole-5-carboxamido)-2-(S-methylsulfonimidoyl)pyridine 1-oxide |
| 43 | 4-(1-(bicyclo[2.1.0]pentan-1-ylmethyl)-3-cyclopropyl-4-(trifluoromethyl)-1H-pyrazole-5-carboxamido)-2-(S-methylsulfonimidoyl)pyridine 1-oxide |
| 44 | 4-(3-cyclopropyl-1-(spiro[2.3]hexan-5-ylmethyl)-4-(trifluoromethyl)-1H-pyrazole-5-carboxamido)-2-(S-methylsulfonimidoyl)pyridine 1-oxide |
| 45 | 4-(3-cyclopropyl-1-((5-methylspiro[2.3]hexan-5-yl)methyl)-4-(trifluoromethyl)-1H-pyrazole-5-carboxamido)-2-(S-methylsulfonimidoyl)pyridine 1-oxide |
| 46 | 4-(3-cyclopropyl-1-((1-methyl-3-(trifluoromethyl)cyclobutyl)methyl)-4-(trifluoromethyl)-1H-pyrazole-5-carboxamido)-2-(S-methylsulfonimidoyl)pyridine 1-oxide |
| 47 | 4-(3-cyclopropyl-1-((1-fluorospiro[2.3]hexan-5-yl)methyl)-4-(trifluoromethyl)-1H-pyrazole-5-carboxamido)-2-(S-methylsulfonimidoyl)pyridine 1-oxide |
| 48 | 4-(3-cyclopropyl-4-(trifluoromethyl)-1-((3-(trifluoromethyl)cyclobutyl)methyl)-1H-pyrazole-5-carboxamido)-2-(S-methylsulfonimidoyl)pyridine 1-oxide |
| 49 | 4-(3-cyclopropyl-1-((3,3-difluorocyclobutyl)methyl)-4-(trifluoromethyl)-1H-pyrazole-5-carboxamido)-2-(S-methylsulfonimidoyl)pyridine 1-oxide |
| 50 | 4-(3-cyclopropyl-1-((5,5-difluorobicyclo[2.1.0]pentan-2-yl)methyl)-4-(trifluoromethyl)-1H-pyrazole-5-carboxamido)-2-(S-methylsulfonimidoyl)pyridine 1-oxide |
| 51 | 4-(3-cyclopropyl-1-(spiro[2.3]hexan-4-ylmethyl)-4-(trifluoromethyl)-1H-pyrazole-5-carboxamido)-2-(S-methylsulfonimidoyl)pyridine 1-oxide |
| 52 | 4-(3-cyclopropyl-1-((6,6-difluoro-4-methylspiro[2.3]hexan-4-yl)methyl)-4-(trifluoromethyl)-1H-pyrazole-5-carboxamido)-2-(S-methylsulfonimidoyl)pyridine 1-oxide |
| 53 | 4-(3-cyclopropyl-1-(spiro[3.3]heptan-1 ylmethyl)-4-(trifluoromethyl)-1H-pyrazole-5-carboxamido)-2-(S-methylsulfonimidoyl)pyridine 1-oxide |
| 54 | 4-(3-cyclopropyl-1-((2-methylbicyclo [2.2.0] hexan-2-yl)methyl)-4-(trifluoromethyl)-1H-pyrazole-5-carboxamido)-2-(S-methylsulfonimidoyl)pyridine 1-oxide |
| 55 | 4-(3-cyclopropyl-1-((3,3-difluorobicyclo[3.1.0]hexan-1-yl)methyl)-4-(trifluoromethyl)-1H-pyrazole-5-carboxamido)-2-(S-methylsulfonimidoyl)pyridine 1-oxide |
| 56 | 4-(3-cyclopropyl-1-((4,4-difluorobicyclo[3.1.0]hexan-1-yl)methyl)-4-(trifluoromethyl)-1H-pyrazole-5-carboxamido)-2-(S-methylsulfonimidoyl)pyridine 1-oxide |
| 57 | 4-(3-cyclopropyl-1-((5-fluorooctahydropentalen-2-yl)methyl)-4-(trifluoromethyl)-1H-pyrazole-5-carboxamido)-2-(S-methylsulfonimidoyl)pyridine 1-oxide |
| 58 | 4-(3-cyclopropyl-4-(trifluoromethyl)-1-((1-(trifluoromethyl)cyclopentyl)methyl)-1H-pyrazole-5-carboxamido)-2-(S-methylsulfonimidoyl)pyridine 1-oxide |
| 59 | 4-(3-cyclopropyl-1-((4,4-difluoro-2-methylcyclopentyl)methyl)-4-(trifluoromethyl)-1H-pyrazole-5-carboxamido)-2-(S-methylsulfonimidoyl)pyridine 1-oxide |
| 60 | 4-(3-cyclopropyl-1-((4,4-difluoro-2-(trifluoromethyl)cyclopentyl)methyl)-4-(trifluoromethyl)-1H-pyrazole-5-carboxamido)-2-(S-methylsulfonimidoyl)pyridine 1-oxide |
| 61 | 4-(3-cyclopropyl-1-((3,3-difluoro-5-methylbicyclo [3.1.0] hexan-1-yl)methyl)-4-(trifluoromethyl)-1H-pyrazole-5-carboxamido)-2-(S-methylsulfonimidoyl)pyridine 1-oxide |

(continued)

| Ex | Name |
|---|---|
| 62 | 4-(3-cyclopropyl-1-((3,3-difluoro-5-(trifluoromethyl)bicyclo[3 .1.0]hexan-1-yl)methyl)-4-(trifluoromethyl)-1H-pyrazole-5-carboxamido)-2-(S-methylsulfonimidoyl)pyridine 1-oxide |
| 63 | 4-(3-cyclopropyl-1-((2,2-difluorobicyclo[2.1.1]hexan-1-yl)methyl)-4-(trifluoromethyl)-1H-pyrazole-5-carboxamido)-2-(S-methylsulfonimidoyl)pyridine 1-oxide |
| 64 | 4-(3-cyclopropyl-1-((3,3-difluorobicyclo[2.1.1]hexan-1-yl)methyl)-4-(trifluoromethyl)-1H-pyrazole-5-carboxamido)-2-(S-methylsulfonimidoyl)pyridine 1-oxide |
| 65 | 4-(3-cyclopropyl-1-((4-fluorobicyclo[2.1.1]hexan-1-yl)methyl)-4-(trifluoromethyl)-1H-pyrazole-5-carboxamido)-2-(S-methylsulfonimidoyl)pyridine 1-oxide |
| 66 | 4-(3-cyclopropyl-1-((4-fluorobicyclo[22.1]heptan-1-yl)methyl)-4-(trifluoromethyl)-1H-pyrazole-5-carboxamido)-2-(S-methylsulfonimidoyl)pyridine 1-oxide |
| 67 | 4-(3-cyclopropyl-1-((4,4-difluorobicyclo[4.1.0]heptan-1-yl)methyl)-4-(trifluoromethyl)-1H-pyrazole-5-carboxamido)-2-(S-methylsulfonimidoyl)pyridine 1-oxide |
| 68 | 4-(3-cyclopropyl-1-((3,3-difluoro-1-methylcyclobutyl)methyl)-4-(trifluoromethyl)-1H-pyrazole-5-carboxamido)-2-sulfamoylpyridine 1-oxide |
| 69 | 4-(1-((3,3-difluoro-1-methylcyclobutyl)methyl)-3-(1,1-difluoroethyl)-4-methyl-1H-pyrazole-5-carboxamido)-2-sulfamoylpyridine 1-oxide |
| 70 | 4-(3-(bicyclo[1.1.1]pentan-1-yl)-1-((3,3-difluoro-1-methylcyclobutyl)methyl)-4-(trifluoromethyl)-1H-pyrazole-5-carboxamido)-2-sulfamoylpyridine 1-oxide |
| 71 | 2-carbamoyl-4-(3-cyclopropyl-1-((3,3-difluoro-1-methylcyclobutyl)methyl)-4-(trifluoromethyl)-1H-pyrazole-5-carboxamido)pyridine 1-oxide |
| 72 | 2-carbamoyl-4-(1-((3,3-difluoro-1-methylcyclobutyl)methyl)-3-(1,1-difluoroethyl)-4-methyl-1H-pyrazole-5-carboxamido)pyridine 1-oxide |
| 73 | 4-(3-(bicyclo[1.1.1]pentan-1yl)-1-((3,3-difluoro-1-methylcyclobutyl)methyl)-4-(trifluoromethyl)-1H-pyrazole-5-carboxamido)-2-carbamoylpyridine 1-oxide |
| 74 | 4-(3-cyclopropyl-1-((1-methyl-2-(trifluoromethyl)cyclopropyl)methyl)-4-(trifluoromethyl)-1H-pyrazole-5-carboxamido)-2-sulfamoylpyridine 1-oxide |
| 75 | 2-carbamoyl-4-(3-cyclopropyl-1-((1-methyl-2-(trifluoromethyl)cyclopropyl)methyl)-4-(trifluoromethyl)-1H-pyrazole-5-carboxamido)pyridine 1-oxide |
| 76 | 4-(3-cyclopropyl-1-((3,3-difluorobicyclo[3.1.0]hexan-1-yl)methyl)-4-(trifluoromethyl)-1H-pyrazole-5-carboxamido)-2-sulfamoylpyridine 1-oxide |
| 77 | 2-carbamoyl-4-(3-cyclopropyl-1-((3,3-difluorobicyclo[3.1.0]hexan-1-yl)methyl)-4-(trifluoromethyl)-1H-pyrazole-5-carboxamido)pyridine 1-oxide |
| 78 | 4-(3-cyclopropyl-1-((3,3-difluoro-1-methylcyclopentyl)methyl)-4-(trifluoromethyl)-1H-pyrazole-5-carboxamido)-2-sulfamoylpyridine 1-oxide |
| 79 | 4-(1-((3,3-difluoro-1-methylcyclopentyl)methyl)-3-(1,1-difluoroethyl)-4-methyl-1H-pyrazole-5-carboxamido)-2-sulfamoylpyridine 1-oxide |
| 80 | 2-carbamoyl-4-(3-cyclopropyl-1-((3,3-difluoro-1-methylcyclopentyl)methyl)-4-(trifluoromethyl)-1H-pyrazole-5-carboxamido)pyridine 1-oxide |
| 81 | 2-carbamoyl-4-(1-((3,3-difluoro-1-methylcyclopentyl)methyl)-3-(1,1-difluoroethyl)-4-methyl-1H-pyrazole-5-carboxamido)pyridine 1-oxide |
| 82 | 3-cyclopropyl-1-((3,3-difluoro-1-methylcyclobutyl)methyl)-N-(2-(dimethylphosphoryl)pyridin-4-yl)-4-(trifluoromethyl)-1H-pyrazole-5-carboxamide |
| 83 | methyl 4-(3-cyclopropyl-1-((tetrahydro-2H-pyran-4-yl)methyl)-4-(trifluoromethyl)-1H-pyrazole-5-carboxamido) picolinate |
| 84 | 4-(3-cyclopropyl-1-((tetrahydro-2H-pyran-4-yl)methyl)-4-(trifluoromethyl)-1H-pyrazole-5-carboxamido) picolinic acid |

in the form of the free compound or a physiologically acceptable salt thereof.

In a preferred embodiment, the compound according to the invention is an inhibitor of $Na_V1.8$. In the sense of the invention, the term "inhibitor of $Na_V1.8$" preferably means that the respective compound exhibits in a patch clamp assay an IC50 value on $Na_V1.8$ of at most 10 $\mu$M ($10 \cdot 10^{-6}$ mol/L); more preferably at most 1 $\mu$M; still more preferably at most 500 nM ($10^{-9}$ mol/L); even more preferably at most 100 nM; and most preferably at most 10 nM.

A preferred assay for testing compounds for their potency and method for determining an IC50 on $Na_V1.8$ is described in the experimental part down below.

In a preferred embodiment, the compound according to the invention is a selective inhibitor of $Na_V1.8$. In the sense of the invention, the term "selective inhibitor of $Na_V1.8$" preferably means that the respective compound preferably does not exhibit any inhibitory activity on $Na_V1.1$, $Na_V1.2$, $Na_V1.4$, $Na_V1.5$ and $Na_V1.6$. The skilled artisan knows suitable ways to determine whether a compound exhibits inhibitory effects on any of $Na_V1.1$, $Na_V1.2$, $Na_V1.4$, $Na_V1.5$ and $Na_V1.6$.

The invention therefore relates to a compound according to the invention for use in the inhibition of $Na_V1.8$.

Therefore, another aspect of the invention relates to a compound according to the invention for use in the treatment of pain. Still another aspect of the invention relates to a method of treatment of pain; comprising the administration of a therapeutically effective amount of a compound according to the invention to a subject in need thereof, preferably a human.

A further aspect of the invention relates to a compound according to the invention as medicament.

Another aspect of the invention relates to a pharmaceutical dosage form comprising a compound according to the invention. Preferably, the pharmaceutical dosage form comprises a compound according to the invention and one or more pharmaceutical excipients such as physiologically acceptable carriers, additives and/or auxiliary substances; and optionally one or more further pharmacologically active ingredient. Examples of suitable physiologically acceptable carriers, additives and/or auxiliary substances are fillers, solvents, diluents, colorings and/or binders. These substances are known to the person skilled in the art (see H. P. Fiedler, Lexikon der Hilfsstoffe fur Pharmazie, Kosmetik und angrenzende Gebiete, Editio Cantor Aulendoff).

The pharmaceutical dosage form according to the invention is preferably for systemic, topical or local administration, preferably for oral administration. Therefore, the pharmaceutical dosage form can be in form of a liquid, semisolid or solid, e.g. in the form of injection solutions, drops, juices, syrups, sprays, suspensions, tablets, patches, films, capsules, plasters, suppositories, ointments, creams, lotions, gels, emulsions, aerosols or in multiparticulate form, for example in the form of pellets or granules, if appropriate pressed into tablets, decanted in capsules or suspended in a liquid, and can also be administered as such.

The pharmaceutical dosage form according to the invention is preferably prepared with the aid of conventional means, devices, methods and processes known in the art. The amount of the compound according to the invention to be administered to the patient may vary and is e.g. dependent on the patient's weight or age and also on the type of administration, the indication and the severity of the disorder. Preferably 0.001 to 100 mg/kg, more preferably 0.05 to 75 mg/kg, most preferably 0.05 to 50 mg of a compound according to the invention are administered per kg of the patient's body weight.

Therefore, another aspect of the invention relates to the pharmaceutical dosage form according to the invention for use in the treatment of pain. Still another aspect of the invention relates to a method of treatment of pain; comprising the administration of a pharmaceutical dosage form according to the invention to a subject in need thereof, preferably a human.

## EXAMPLES

### Experimental protocols

The following abbreviations are used in the descriptions of the experimental protocols: ABPR = automatic back pressure regulator; aq. = aqueous; Boc = *tert*-butyloxycarbonyl; DAST = diethylaminosulfur trifluoride, DCM = dichloromethane; DIAD = diisopropylazodicarboxylate; DIPEA = N,*N*-diisopropylethylamine; DMF = *N*,*N*-dimethylformamide; DMSO = dimethylsulfoxide; EtOAc = ethyl acetate; $Et_2O$ = diethyl ether; EtOH = ethanol; h = hour; HATU = 1-[bis(dimethylamino)methylene]-1*H*,2,3-triazolo[4,5-b]pyridinium 3-oxide hexafluorophosphate; Hex = hexane; HPLC = high-performance liquid chromatography; LAH = lithium aluminium hydride, LCMS = liquid chromatography-mass spectrometry; LiHMDS = Lithium bis(trimethylsilyl)amide; MeCN = acetonitrile; MeI = methyl iodide; MeOH = methanol; $MeSO_2Cl$ = methanesulfonyl chloride; min = minute; Ms = methanesulfonyl; MTBE = methyl *tert*-butyl ether; NIS = N-iodosuccinimide; NMR = nuclear magnetic resonance; prep. = preparative; rt = room temperature; $R_t$ = retention time; sat. = saturated; SEM = 2-(trimethylsilyl)ethoxymethyl; SFC = supercritical fluid chromatography; SM = starting material; TEA = triethylamine; Tf = trifluoromethylsulfonyl; THF = tetrahydrofurane; $TMSCF_3$ = trifluoromethyltrimethylsilane; Ts = p-toluenesulfonyl.

### General synthesis schemes

As illustrated in Scheme 1, compounds of the invention can be prepared by *N*-alkylation of alkyl 1*H* pyrazole-5-carboxylates of the general formula (A) with appropriately functionalized alkylation reagents of the general formula (B) (where Y is leaving group, such as Br, Cl, OTs, OMs, OTf) under basic conditions to afford compounds of general

formula (C). Alternatively, intermediates of the general formula (C) can be prepared using a Mitsunobu reaction between alkyl 1*H*-pyrazole-5-carboxylates of type (A) and alcohols of the general formula (B) (where Y is OH) (Chem. Rev. 2009, 109, 6, 2551-2651). Intermediates of the general formula (C) can be hydrolized to carboxylic acids of the general formula (D).

**Scheme 1:** L, R1, R4, R5 are as defined in claim 1.

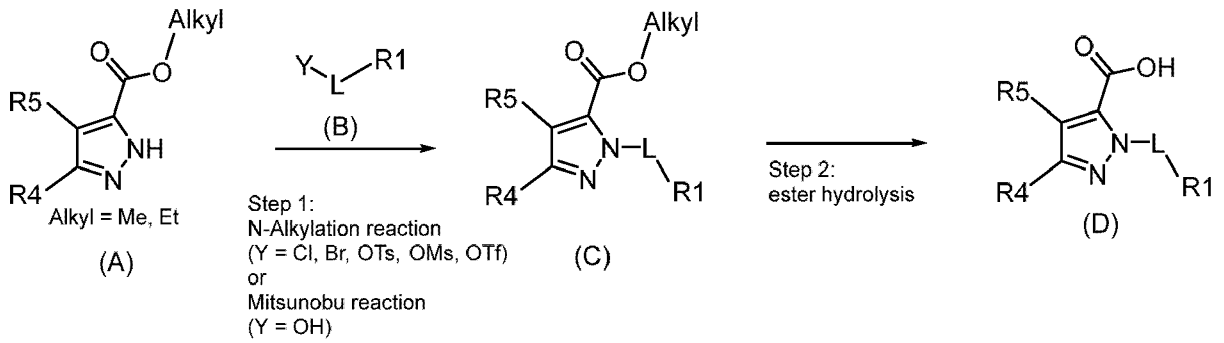


In some embodiments, intermediates need to bear one or more protecting groups, such as SEM (trimethylsilylethoxymethyl) or Bn (benzyl) which are deprotected after N-alkylation using standard deprotection protocols known in the art (T. W. Green, P. G. M. Wuts, Protective Groups in Organic Synthesis, Wiley-Interscience, New York, 1999). In some embodiments functional group interconversions are used such as fluorination using e.g. DAST (Synthesis, 2002, 2561-2578) or difluromethylation using sodium 2-chloro-2,2-difluoroacetate *(*Chem. Soc. Rev, 2021, 50, 8214-8247).

**Scheme 2:** L, R1, R4, R5 are as defined in claim 1.

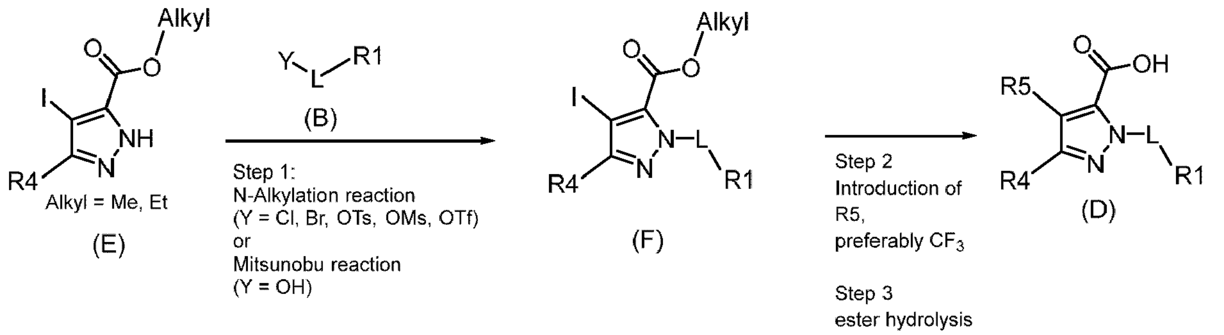


As illustrated in Scheme 2, intermediates of the general formula (D) can also be obtained by *N*-alkylation of alkyl 4-iodo-1*H*-pyrazole-5-carboxylates of the general formula (E) with appropriately functionalized alkylation reagents of the general formula (B) (Y is leaving group, such as Br, Cl, OTs, OMs, OTf) under basic conditions to give intermediates of the general formula (F). Introduction of the substituent R5 e.g. via trifluoromethylation with $TMSCF_3$ in the presence of CuI or Suzuki coupling followed by ester hydrolysis gives intermediates of the general formula (D). Alternatively, a Mitsunobu reaction between alkyl 4-iodo-1*H*-pyrazole-5-carboxylates of the general formula (E) and alcohols of the general formula (B) (where Y is OH) can be used to obtain intermediates of the general formula (F).

**Scheme 3:** R4, R5 are as defined in claim 1.

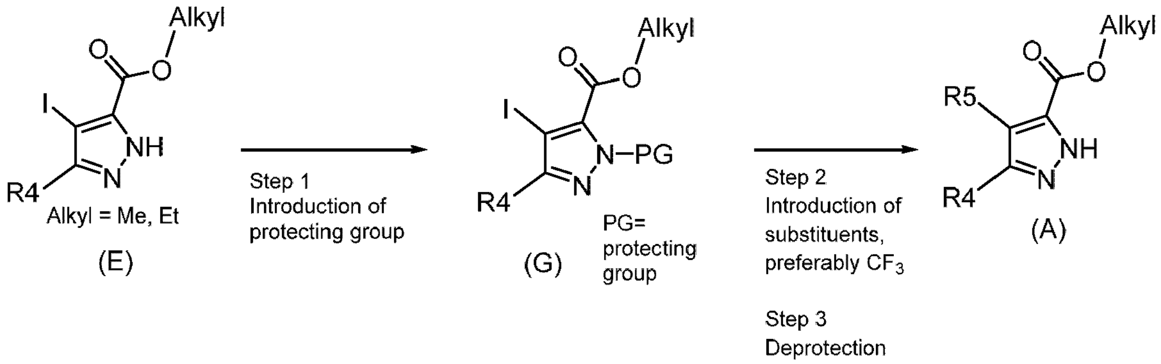


As illustrated in Scheme 3, alkyl 4-iodo-1*H*-pyrazole-5-carboxylates of the general formula (E) can also be used to obtain alkyl 1*H* pyrazole-5-carboaylates of the general formula (A) using a sequence consisting of introduction of a protecting group followed by introduction of the substituent R5 e.g. via trifluoromethylation with $TMSCF_3$ in the presence of CuI or Suzuki coupling followed by deprotection. Suitable protecting groups are e.g. SEM (trimethylsilylethoxymethyl). Standard protection/ deprotection protocols are known in the art (T. W. Green, P. G. M. Wuts, Protective Groups in Organic Synthesis, Wiley-Interscience, New York, 1999).

As illustrated in Scheme 4, intermediates of the general formula (D) can be converted to compounds of the general formula (H) by the use of an appropriate chlorinating reagent such as $POCl_3$. Intermediates of the general formula (H) can be converted to compounds of the general formula (J) using amide coupling with (hetero)arylamines of the general formula (I) (March's Advanced Organic Chemistry, 2007, 6th Edition, page 1427-1474). Compounds of the general formula (J), where R3' = $CO_2$-alkyl can be hydrolized to the corresponding carboxylic acids.

**Scheme 4:** L, R1, R2, R4, R5, R', A1, A2, A3 are as defined in claim 1.

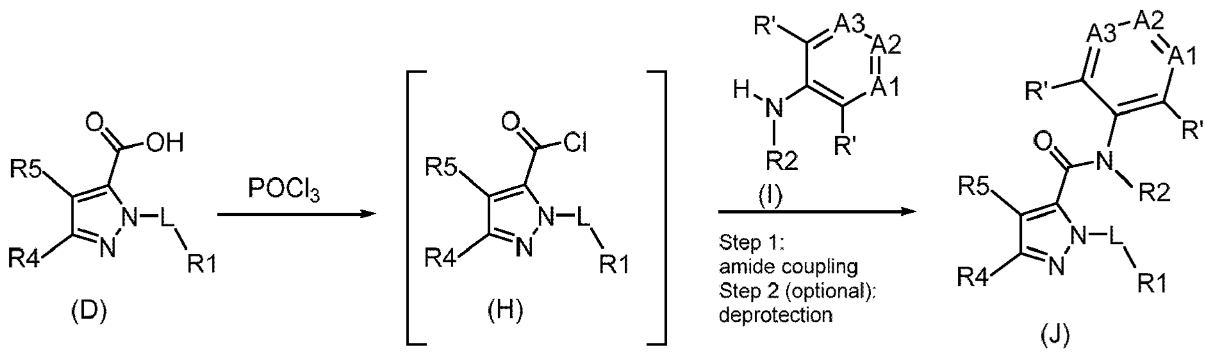


Alternatively and as illustrated in Scheme 5, intermediates of the general formula (D) can be converted to compounds of the general formula (K) using amide coupling with $NH_4Cl$ in the presence of a carboxylic acid activating reagent, preferably HATU and a base, preferably pyridine or DIPEA (March's Advanced Organic Chemistry, 2007, 6th Edition, page 1427-1474). Compounds of the general formula (K) can be converted to compounds of the general formula (J) using a amidation reaction with (hetero)arylhalides of general formula (L) in the presence of a suitable catalyst such as copper(I) trifluoromethanesulfonate benzene complex in the presence of an appropriate ligand such as trans-N,N-cyclohexyl-1,2-diamine and a base such as $Cs_2CO_3$.

**Scheme 5:** L, R1, R2, R4, R5, R', A1, A2, A3 are as defined in claim 1. Y=halogen

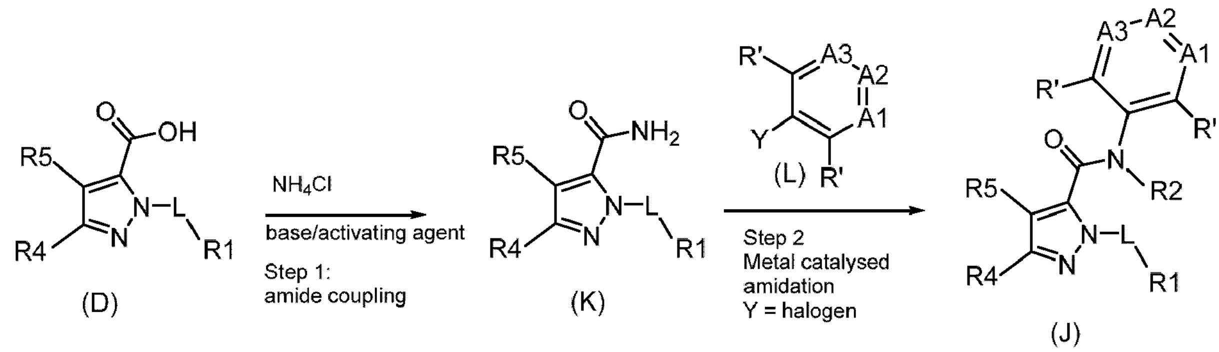


Alkyl 1*H*-pyrazole-5-carboxylates of the general formula (A), alkylation reagents of the general formula (B), (hetero)arylamines of the general formula (I) and (hetero)arylhalides of the general formula (L) can be either commercially available or prepared as described in the present invention or synthesized according to the standard procedures known to the person skilled in the art.

**Intermediate 1**

Ethyl 3-acetyl-1*H*-pyrazole-5-carboxylate

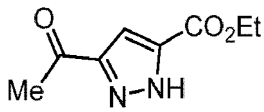

To a solution of $InCl_3$ (3.25 g, 14.7 mmol) in water (200 mL) were added but-3 yn-2-one (5.0 g, 73.5 mmol) and ethyl diazoacetate (9.31 mL, 88.2 mmol) at rt and the reaction mixture was stirred at rt for 16 h. The reaction mixture was diluted with ice water and extracted with EtOAc (3x 300 mL). The combined organics were washed with brine, dried ($Na_2SO_4$) and concentrated under reduced pressure. The residue was washed with hexane to afford the title compound (7.5 g, 56%). LCMS m/z = 183 $[M+H]^+$.

**Intermediate 2**

Ethyl 3-(1,1-difluoroethyl)-1*H*-pyrazole-5-carboxylate

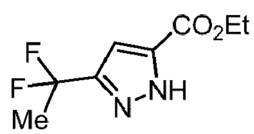

To a solution of ethyl 3-acetyl-1*H*-pyrazole-5-carboxylate (Intermediate 1, 6.0 g, 32.96 mmol) in DCM (150 mL) was added DAST (17.4 mL, 131.8 mmol) dropwise at 0 °C and the resulting mixture was stirred at rt for 16 h. The reaction was quenched with cold sat. $NaHCO_3$ solution and extracted with DCM (3x 100 mL). The combined organics were dried ($Na_2SO_4$) and evaporated to dryness under reduced pressure. The residue was purified by CombiFlash chromatography ($SiO_2$, 0-40% EtOAc/Hex) to afford the title compound (6 g, 89%). LCMS m/z = 205 $[M+H]^+$.

**Intermediate 3**

Ethyl 3-(1,1-difluoroethyl)-4-iodo-1*H*-pyrazole-5-carboxylate

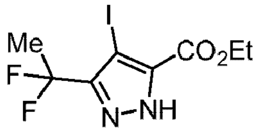

To a stirred solution of ethyl 3-(1,1-difluoroethyl)-1H-pyrazole-5-carboxylate (Intermediate 2, 6.0 g, 29.4 mmol) in DMF (60 mL) was added NIS (10.1 g, 44.1 mmol) at rt and the reaction mixture stirred at 90 °C for 16 h. The reaction mixture was diluted with ice water and extracted with EtOAc (3x 100 mL). The combined organics were washed with sat $Na_2S_2O_3$ solution, brine, dried ($Na_2SO_4$) and concentrated under reduced pressure. The residue was purified by combiflash chromatography ($SiO_2$, 0-50% EtOAc/Hex) to afford the title compound (9 g, 92%). LCMS m/z = 331 $[M+H]^+$.

**Intermediate 4**

(3,3-Difluorocyclobutyl)methyl methanesulfonate

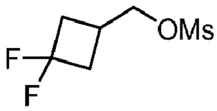

To a stirred solution of (3,3-difluorocyclobutyl)methanol (5.0 g, 41 mmol) in DCM (100 mL) were added $Et_3N$ (12 g, 123 mmol) and MsCl (12 g, 82 mmol) at 0 °C and the mixture was stirred for 2 h at rt. The reaction mixture was quenched with water (80 mL) and extracted with DCM (2x 50 mL). The combined organics were washed with water (50 mL), brine (50 mL), dried ($Na_2SO_4$) and concentrated under reduced pressure to afford the title compound (7.3 g, crude) which was used without further purification.

**Intermediate 5**

Ethyl 1-((3,3-difluorocyclobutyl)methyl)-3-(1,1-difluoroethyl)-4-iodo-1*H*-pyrazole-5-carboxylate

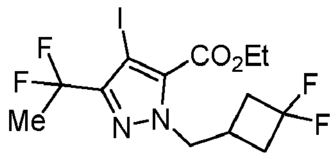

To a stirred solution of ethyl 3-(1,1-difluoroethyl)-4-iodo-1H-pyrazole-5-carboxylate (Intermediate 3, 1.0 g, 3.03 mmol) in acetonitrile (30 mL) were added $K_2CO_3$ (0.838 g, 6.07 mmol) and (3,3-difluorocyclobutyl)methyl methanesulfonate (Intermediate 4, 0.73 g, 364 mmol) at rt and the reaction mixture was heated at 80 °C for 16 h. The cooled mixture was diluted with cold water and extracted with EtOAc (3x 50 mL). The combined organics were washed with brine, dried ($Na_2SO_4$) and evaporated to dryness under reduced pressure. The residue was purified by combiflash chromatography ($SiO_2$, 0-20% EtOAc/Hex) to afford the title compound (0.8 g, 60%). LCMS m/z = 435 $[M+H]^+$.

**Intermediate 6**

Ethyl 1-((3,3-difluorocyclobutyl)methyl)-3-(1,1-difluoromethyl)-4-(trifluoromethyl)-1*H*-pyrazole-5-carboxylate

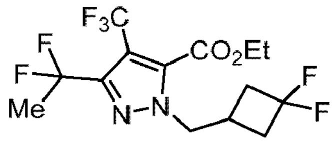

KF (0.2 g, 3.45 mmol) and CuI (0.43 g, 2.30 mmol) were added at rt to a solution of ethyl 1-((3,3-difluorocyclobutyl)methyl)-3-(1,1-difluoroethyl)-4-iodo-1H-pyrazole-5-carboxylate (Intermediate 5, 0.5 g, 1.15 mmol) in DMF (8 mL). To this was added dropwise $TMSCF_3$ (1.2 mL, 8.06 mmol) at 0 °C and the mixture was heated at 110 °C in sealed tube for 16 h. The cooled reaction mixture was diluted with cold-water (30 mL), filtered through celite and washed with EtOAc (300 mL). The filtrate was washed with water (50 mL), brine (30 mL), dried ($Na_2SO_4$) and concentrated under reduced pressure. The residue was purified by combiflash chromatography ($SiO_2$, 0-20% EtOAc/Hex) to yield the title compound (200 mg, 46%). LCMS m/z = 377 $[M+H]^+$.

**Intermediate 7**

1-((3,3-Difluorocyclobutyl)methyl)-3-(1,1-difluoroethyl)-4-(trifluoromethyl)-1H-pyrazole-5-carboxylic acid

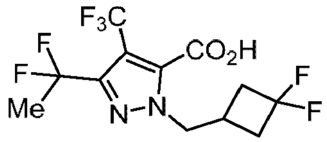

To a stirred solution of ethyl 1-((3,3-difluorocyclobutyl)methyl)-3-(1,1-difluoroethyl)-4-(trifluoromethyl)-1*H*-pyrazole-5-carboxylate (Intermediate 6, 0.35 g, 0.93 mmol) in a mixture of THF (8 mL) and $H_2O$ (2 mL) was added $LiOH \cdot H_2O$ (0.058 g, 1.39 mmol) at 0 °C and the mixture was stirred at rt 16 h. The reaction mixture was concentrated under reduced pressure and diluted with water. The aqueous phase was acidified with saturated $KHSO_4$ solution to pH 2 at 0°C and extracted with EtOAc (3x 30 mL). The combined organics were washed with brine, dried ($Na_2SO_4$) and evaporated to dryness under reduced pressure to afford the title compound (300 mg, 92%). LCMS m/z = 349 $[M+H]^+$.

**Intermediate** 8

Ethyl 3-cyclopropyl-4-iodo-1H-pyrazole-5-carboxylate

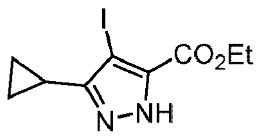

NIS (26.27 g, 100 mmol) was added portion wise to a solution of ethyl 3-cyclopropyl-1H-pyrazole-5-carboxylate (15 g, 83.2 mmol) in DCM (150 mL) at 0 °C and the reaction mixture was stirred for 2 h at rt. The reaction mixture was quenched with saturated sodium thiosulfate (200 mL) at 0 °C and extracted with DCM (2x 250 mL). The combined organics were washed with saturated $NaHCO_3$ (250 mL), brine (200 mL), dried ($Na_2SO_4$) and evaporated to dryness under reduced pressure. The residue was purified by combiflash chromatography (silica gel, 2-5% EtOAc/Hex) to afford the title compound (25 g, 90%). LCMS (Method G) m/z = 307 $[M+H]^+$.

**Intermediate 9**

Ethyl 3 -cyclopropyl-4-iodo-1-((2-(trimethylsilyl)ethoxy)methyl)-1H-pyrazole-5-carboxylate

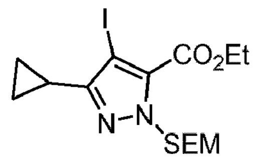

To a solution of ethyl 3-cyclopropyl-4-iodo-1H-pyrazole-5-carboxylate (Intermediate 8, 20 g, 65.6 mmol) in DCM (200 mL) was added DIPEA (34.4 mL, 536 mmol) at 0 °C and the mixture was stirred for 30 min before a solution of SEM-CL (15.6 mL, 131 mmol) in DCM (100 mL) was added drop wise at 0 °C. The reaction mixture was stirred at rt for 16 h. The reaction mixture was quenched with ice water and extracted with DCM (2x 250 mL). The combined organics were washed with brine (250 mL), dried ($Na_2SO_4$) and evaporated to dryness under reduced pressure. The residue was purified by column chromatography (silica gel, 5% EtOAc/Hex) to afford the title compound (22 g, 90%). $^1$H-NMR (400 MHz, DMSO-$d_6$): 5.61 (s, 2H), 4.33 (q, 2H), 3.45 (t, 2H), 1.89-1.85 (m, 1H), 1.34 (t, 3H), 0.96-0.90 (m, 2H), 0.78-0.73 (m, 4H), -0.09 (s, 9H).

**Intermediate 10**

Ethyl 3-cyclopropyl-4-(trifluoromethyl)-1-((2-(trimethylsilyl)ethoxy)methyl)-1H-pyrazole-5-carboxylate

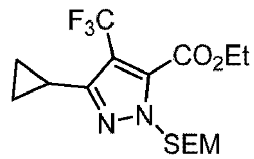

To a solution of ethyl 3-cyclopropyl-4-iodo-1-((2-(trimethylsilyl)ethoxy)methyl)-1H-pyrazole-5-carboxylate (Intermediate 9, 10 g, 23 mmol) in DMF (60 mL) at rt were added KF (4.0 g, 70 mmol), $TMSCF_3$ (23.8 mL, 161 mmol) and CuI (8.72 g, 45.9 mmol) and the reaction mixture was heated at 90 °C in sealed tube for 10 h. The reaction mixture was cooled to rt, diluted with cold-water (300 mL), filtered through celite and washed with EtOAc (800 mL). The filtrate was washed with water (3x 100 mL), brine (200 mL), dried ($Na_2SO_4$) and evaporated to dryness under reduced pressure. The residue was purified by combiflash chromatography (silica gel, 2-5% EtOAc/Hex) to give the title compound (7.8 g, 78%). $^1$H-NMR (400 MHz, DMSO-$d_6$): 5.57 (s, 2H), 4.35 (quin, 1H), 3.52-3.45 (m, 2H), 1.29 (q, 3H), 0.96-0.74 (m, 6H), -0.08 (s, 9H).

**Intermediate 11**

Ethyl 3-cyclopropyl-4-(trifluoromethyl)-1H-pyrazole-5-carboxylate

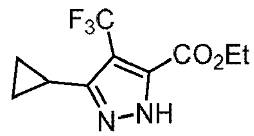

To a solution of ethyl 3-cyclopropyl-4-(trifluoromethyl)-1-((2-(trimethylsilyl)ethoxy)methyl)-1H-pyrazole-5-carboxylate (Intermediate 10, 4.7 g, 12.4 mmol) in EtOH (35 mL) was added 4M HCl in dioxane (25 mL) and the mixture was stirred at rt for 2 h. The reaction mixture was concentrated under reduced pressure and the residue diluted with EtOAc (800 mL), washed with sat.$NaHCO_3$ (200 mL), water (2x 100 mL), brine (250 mL), dried (Na2SO4) and evaporated

under reduced pressure. The residue was purified by combiflash chromatography (silica gel, 0-60% EtOAc/Hex) to afford the title compound (1.8 g, 58%). LCMS m/z = 249 [M+H]$^+$.

**Intermediate 12**

Ethyl 3-cyclopropyl-1-((4,4-difluorocyclohexyl)methyl)-4-(trifluoromethyl)-1H-pyrazole-5-carboxylate

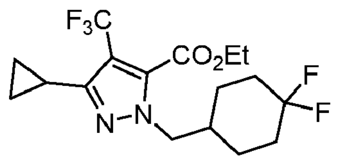

To a stirred solution of ethyl 3-cyclopropyl-4-(trifluoromethyl)-1H-pyrazole-5-carboxylate (Intermediate 11, 400 mg, 1.61 mmol) in THF (25 mL) were added (4,4-difluorocyclohexyl)methanol (266 mg, 1.77 mmol) and triphenylphospine (845 mg, 3.22 mmol) at 0 °C. DIAD (0.6 mL, 3.22 mmol) was added dropwise and the mixture was stirred at 90 °C for 16 h. The reaction mixture was concentrated under reduced pressure and the residue purified by combiflash chromatography (silica gel; 0-20% EtOAc/Hex) to afford the title compound (450 mg, 73%). LCMS m/z = 381 [M+H]$^+$.

**Intermediate 13**

(3,3-Difluoro-1-methylcyclopentyl)methanol

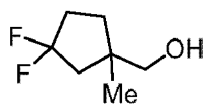

**Part 1.** To a stirred solution of ethyl 3-oxocyclopentane-1-carboxylate (7.0 g, 44.9 mmol) in DCM (175 mL) was added DAST (28.9 g, 179 mmol) at 0 °C under Ar and the resulting reaction mixture was stirred at rt for 24 h. The reaction mixture was quenched with sat. $NaHCO_3$ solution (200 mL) and extracted with DCM (2x 200 mL). The combined organics were washed with water (200 mL), brine (200 mL), dried over anhydrous $Na_2SO_4$ and concentrated under reduced pressure to afford ethyl 3,3-difluorocyclopentane-1-carboxylate (8.0 g, crude) which was directly used in Part 2 without further purification.

**Part 2.** To a stirred solution of ethyl 3,3-difluorocyclopentane-1-carboxylate (from Part 1, 4.0 g, 22.5 mmol) in THF (70 mL) was added LiHMDS in THF (1M, 29.2 mL, 29.2 mmol) at -5 °C and the mixture was stirred at 0 °C for 30 min under Ar. To the reaction mixture was added a solution of MeI (4.78 g, 33.7 mmol) in THF (10 mL) and the mixture was stirred at 0 °C-rt for 2 h. The reaction mixture was slowly quenched with saturated aq. $NH_4Cl$ solution (100 mL) and extracted with EtOAc (2x 100 mL). The combined organics were washed with water (100 mL), brine (100 mL), dried over anhydrous $Na_2SO_4$ and concentrated under reduced pressure to afford ethyl 3,3-difluoro-1-methylcyclopentane-1-carboxylate (3.5 g, crude).

**Part 3.** To a stirred solution of ethyl 3,3-difluoro-1-methylcyclopentane-1-carboxylate (from Part 2, 3.5 g, 18.2 mmol) in THF (87.5 mL) was added LAH in THF (1M, 27.3 mL, 27.3 mmol) at 0 °C under argon and the reaction mixture was stirred at 0 °C for 2 h. The reaction mixture was slowly poured into crushed ice (200 g), the pH was adjusted with 1N aq. HCl solution to ~6, the mixture was stirred well and extracted with ethyl acetate (2x 100 mL). The combined organic were washed with water (100 mL), brine (100 mL), dried over anhydrous $Na_2SO_4$ and concentrated under reduced pressure. The residue was purified by column chromatography ($SiO_2$, 15% EtOAc/PE) to afford the title compound (1.8 g, 53% over 3 steps).

**Intermediate 14**

(3,3-Difluoro-1-methylcyclopentyl)methyl trifluoromethanesulfonate

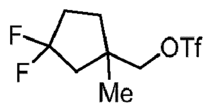

To a stirred solution of (3,3-difluoro-1-methylcyclopentyl)methanol (Intermediate 13, 1.3 g, 8.67 mmol) in DCM (32.5 mL) was added pyridine (1.37 g, 17.33 mmol) followed by trifluoromethane sulfonic anhydride (3.17 g, 11.3 mmol) drop wise at 0 °C under argon and the resulting reaction mixture was stirred at 0 °C for 2 h. The reaction mixture was quenched with water (50 mL) and extracted with DCM (2x 40 mL). The combined organics were washed with water (30 mL), brine (30 mL), dried over anhydrous $Na_2SO_4$ and concentrated under reduced pressure to afford the title compound (2.5 g, crude) which was directly used without further purification.

**Intermediate 15**

Ethyl 3-acetyl-4-methyl-1*H*-pyrazole-5-carboxylate

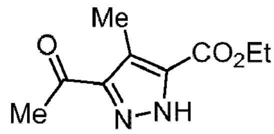

To a stirred solution of KOH (112 g, 200 mol) in ethanol (15 vol), was added pentane-2,4-dione (200 g, 200 mol) at reflux temperature followed by addition of ethyl diazoacetate (258 g, 200 mol) and the resulting reaction mixture was stirred at 80 °C for 1 h. The reaction mixture was concentrated under reduced pressure, diluted with water (50 vol), adjusted the pH to ~2 with 3N aq. HCl solution at 15-20°C. The precipitated solid was collected by filtration, washed with water and dried to afford the title compound (150 g, 38%) which was used without further purification. LCMS m/z = 197 $[M+H]^+$.

**Intermediate 16**

Ethyl 3-(1,1-difluoroethyl)-4-methyl-1*H*-pyrazole-5-carboxylate

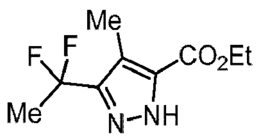

To a stirred solution of ethyl 3-acetyl-4-methyl-1*H*-pyrazole-5-carboxylate (Intermediate 15, 100 g, 510 mmol) in DCM (1L) was added DAST (164 g, 1020 mmol) at 0 °C and the reaction mixture was stirred at rt for 12 h. The reaction mixture was quenched with 50% aq. $NaHCO_3$ solution (500 mL), and extracted with DCM (2 x 200 mL). The combined organics were washed with water (150 mL), brine (150 mL), dried ($Na_2SO_4$) and evaporated to dryness *in vacuo.* The residue was purified by column chromatography ($SiO_2$, 10% EtOAc/PE) to afford the title compound (40 g, 35%). LCMS m/z = 219 $[M+H]^+$.

**Intermediate 17**

Ethyl 1-((3,3-difluoro-1-methylcyclopentyl)methyl)-3-(1,1-difluoroethyl)-4-methyl-1H-pyrazole-5-carboxylate

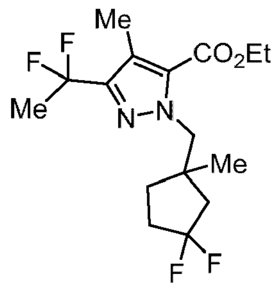

To a stirred solution of ethyl 3-(1,1-difluoroethyl)-4-methyl-1H-pyrazole-5-carboxylate (Intermediate 16, 1.6 g, 7.34 mmol) and (3,3-difluoro-1-methylcyclopentyl)methyl trifluoromethanesulfonate (Intermediate 14, 2.48 g, 8.81 mmol) in DMF (32 mL) was added $Cs_2CO_3$ (4.78 g, 14.68 mmol) at rt under argon atmosphere and the resulting reaction mixture was stirred at 85 °C for 6 h. The reaction mixture was quenched with water (75 mL) and extracted with EtOAc (2x 70 mL). The combined organics were washed with water (40 mL), brine (40 mL), dried over anhydrous $Na_2SO_4$ and concentrated under reduced pressure. The residue was purified by column chromatography ($SiO_2$, 5% EtOAc/PE) to afford the title compound (2.3 g, 90%). LCMS: m/z = 351 $[M+H]^+$.

**Intermediate 18**

Ethyl 1-(cyclohexylmethyl)-4-(trifluoromethyl)-1H-pyrazole-5-carboxylate

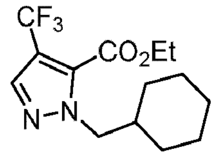

To a solution of methyl 3-(trifluoromethyl)-1H-pyrazole-5-carboxylate (3.5 g, 16.815 mmol) in DMF (70 mL) was added $Cs_2CO_3$ (16.4 g, 50.445 mmol) at rt under an argon atmosphere. The resulting reaction mixture was stirred for 15 min and then (bromomethyl)cyclohexane (2.58 mL, 18.497 mmol) was added. The mixture was stirred at rt for 18 h. The reaction mixture was diluted with water (150 mL) and extracted with ethyl acetate (2 x 100 mL). The combined organic layers were washed with brine (80 mL), dried over anhydrous sodium sulfate and concentrated under reduced pressure. The crude product was purified by GRACE flash column chromatography using 0-40% EtOAc/hexane as an eluent to obtain the title compound (2.4 g, 46%).

**Intermediate 19**

Ethyl (R)-1-((3,3-difluorocyclopentyl)methyl)-3-(1,1-difluoroethyl)-4-methyl-1H-pyrazole-5-carboxylate

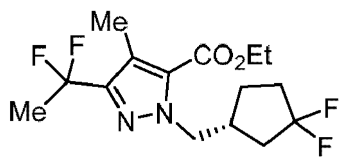

The reaction was carried out in duplicate. To a stirred solution of triphenylphosphine (12 g, 45.9 mmol) in THF (40 mL) was added DIAD (9.2 g, 45.9 mmol) drop wise at 0 °C and the mixture was stirred for 30 min at 0°C. To this mixture were added ethyl 3-(1,1-difluoroethyl)-4-methyl-1H-pyrazole-5-carboxylate (Intermediate 16, 4 g, 18.3 mmol) and (R)-(3,3-difluorocyclopentyl)methanol (2.7 g, 18.3 mmol) at 0 °C and the mixture was slowly warmed to rt and stirred for 16 h at rt.

The combined reaction mixtures were diluted with water (200 mL) and extracted with EtOAc (2x 400 mL). The combined organics were washed with cold water (100 mL), brine (100 mL), dried ($Na_2SO_4$) and concentrated under

reduced pressure. The residue was purified by column chromatography ($SiO_2$, 10% EtOAc/PE) to afford the title compound (8.0 g, 65%). LCMS m/z = 337 $[M+H]^+$.

**Intermediate 20**

Ethyl (S)-1-((3,3-difluorocyclopentyl)methyl)-3-(1,1-difluoroethyl)-4-methyl-1H-pyrazole-5-carboxylate

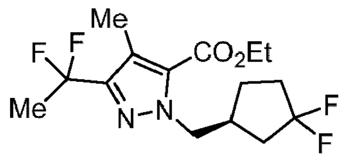

The title compound was prepared (4.5 g, 36.6%) from ethyl 3-(1,1-difluoroethyl)-4-methyl-1H-pyrazole-5-carboxylate (Intermediate 16) and (S)-(3,3-difluorocyclopentyl)methanol using an analogous method as described for Intermediate 19. LCMS m/z = 337 $[M+H]^+$.

**Intermediate 21**

Ethyl 1-((3,3-difluorocyclobutyl)methyl)-3-(1,1-difluoroethyl)-4-methyl-1H-pyrazole-5-carboxylate

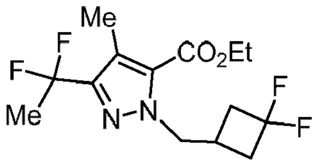

To a stirred solution of ethyl 1-((3,3-difluorocyclobutyl)methyl)-3-(1,1-difluoroethyl)-4-iodo-1H-pyrazole-5-carboxylate (Intermediate 5, 10 g, 23 mmol) in dioxane (100 mL) were added methyl boronic acid (18.5 g, 115 mmol) and $K_2CO_3$ (9.5 g, 63.1 mmol) at rt. The reaction mixture was degassed with Ar for 10 mins and $PdCl_2(dppf)\cdot DCM$ (3.7 mg, 4.61 mmol) was added at rt and the resulting mixture was heated to 100 °C 16 h. The reaction mixture was diluted with water (250 mL) and extracted with $Et_2O$ (2x 120 mL). The combined organics were dried over anhydrous $Na_2SO_4$ and concentrated under reduced pressure. The residue was purified by column chromatography ($SiO_2$, 12-16% EtOAc/PE) to afford the title compound (6 g, 80%). LCMS m/z = 217 $[M+H]^+$.

**Intermediate 22**

Ethyl 1-((3,3 -difluoro-1-methylcyclobutyl)methyl)-3-(1,1-difluoroethyl)-4-methyl-1H-pyrazole-5-carboxylate

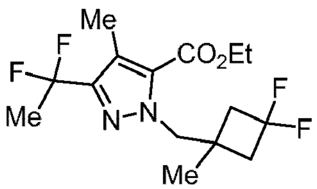

A solution of $PPh_3$ (19.4 g, 91.7 mmol) and DIAD (18.5 g, 91.7 mmol) in THF (250 mL) was stirred for 30 min at 0 °C before ethyl 3-(1,1-difluoroethyl)-4-methyl-1H-pyrazole-5-carboxylate (Intermediate 16, 10.0 g, 45.871 mmol) was added at 0 °C. The reaction mixture was stirred for 10 min and (3,3-difluoro-1-methylcyclobutyl)methanol (6.2 g, 45.9 mmol) was added at 0 °C and the mixture was stirred at 0 °C-rt for 16 h. The reaction mixture was quenched with water (150 mL) and extracted with EtOAc (100 mL). The combined organics were washed with water (50 mL), brine (50

mL), dried over anhydrous $Na_3SO_4$ and concentrated under reduced pressure. The reside was purified by column chromatography ($SiO_2$, 6% EtOAc/PE) to afford the title compound (15.0 g, 97%). LCMS m/z = 337 $[M+H]^+$.

**Intermediate 23-28 and 35 [LiOH hydrolysis]**

The title compounds were prepared from the appropriate ester using an analogous method to that described for Intermediate 7.

| Intermediate | Name/Structure/Data |
|---|---|
| 23 | 3-cyclopropyl-1-((4,4-difluorocyclohexyl)methyl)-4-(trifluoromethyl)-1H-pyrazole-5-carboxylic acid<br>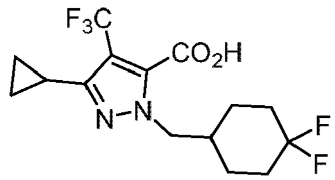<br>Ester: ethyl 3-cyclopropyl-1-((4,4-difluorocyclohexyl)methyl)-4-(trifluoromethyl)-1H-pyrazole-5-carboxylate (Intermediate 12)<br>Yield: 290 mg, 90%; LCMS: m/z = 353 $[M+H]^+$. |
| 24 | 1-((3,3-difluoro-1-methylcyclopentyl)methyl)-3-(1,1-difluoroethyl)-4-methyl-1H-pyrazole-5-carboxylic acid<br>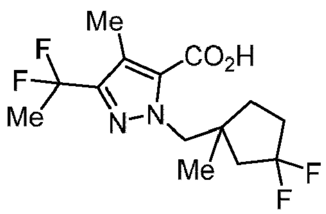<br>Ester: ethyl (R)-1-((3,3-difluorocyclopentyl)methyl)-3-(1,1-difluoroethyl)-4-methyl-1H-pyrazole-5-carboxylate (Intermediate 19)<br>Yield: 1.9 g, 95%; LCMS: m/z = 309 $[M+H]^+$. |
| 25 | (R)-1-((3,3-Difluorocyclopentyl)methyl)-3-(1,1-difluoroethyl)-4-methyl-1H-pyrazole-5-carboxylic acid<br>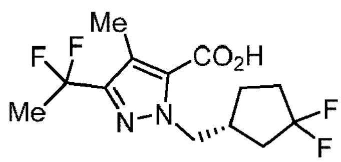<br>Ester: ethyl (R)-1-((3,3-difluorocyclopentyl)methyl)-3-(1,1-difluoroethyl)-4-methyl-1H-pyrazole-5-carboxylate (Intermediate 19)<br>Yield: 3 g, 68%; LCMS: m/z = 309 $[M+H]^+$ |
| 26 | (S)-1-((3,3-Difluorocyclopentyl)methyl)-3-(1,1-difluoroethyl)-4-methyl-1H-pyrazole-5-carboxylic acid<br>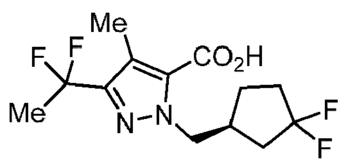<br>Ester: ethyl (S)-1-((3,3-difluorocyclopentyl)methyl)-3-(1,1-difluoroethyl)-4-methyl-1H-pyrazole-5-carboxylate (Intermediate 20)<br>Yield: 3 g, 68%; LCMS: m/z = 309 $[M+H]^+$. |

(continued)

| Intermediate | Name/Structure/Data |
|---|---|
| 27 | 1-((3,3-difluorocyclobutyl)methyl)-3-(1,1-difluoroethyl)-4-methyl-1H-pyrazole-5-carboxylic acid<br>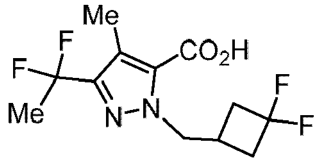<br>Ester: ethyl 1-((3,3-difluorocyclobutyl)methyl)-3-(1,1-difluoroethyl)-4-methyl-1H-pyrazole-5-carboxylate (Intermediate 21)<br>Yield: 7.0 g, 95%; LCMS m/z = 295 $[M+H]^+$. |
| 28 | 1-((3,3-difluoro-1-methylcyclobutyl)methyl)-3-(1,1-difluoroethyl)-4-methyl-1H-pyrazole-5-carboxylic acid<br>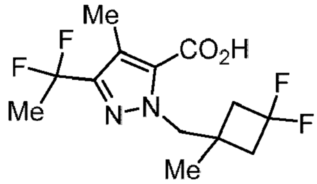<br>Ester: ethyl 1-((3,3-difluoro-1-methylcyclobutyl)methyl)-3-(1,-(1,1-difluoroethyl)-4-methyl-1H-pyrazole-5-carboxylate (Intermediate 22)<br>Yield: 7.0 g, 95%; LCMS m/z = 309 $[M+H]^+$. |
| 35 | 1-(Cyclohexylmethyl)-4-(trifluoromethyl)-1H-pyrazole-5-carboxylic acid<br>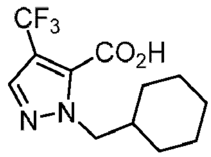<br>Ester: Ethyl 1-(cyclohexylmethyl)-4-(trifluoromethyl)-1H-pyrazole-5-carboxylate (Intermediate XX)<br>Yield: 0.2 g, 84%; LCMS m/z = 277 $[M+H]^+$. |

**Intermediate 29**

(R)-1-((3,3-Difluorocyclopentyl)methyl)-3-(1,1-difluoroethyl)-4-methyl-1H-pyrazole-5-carboxamide

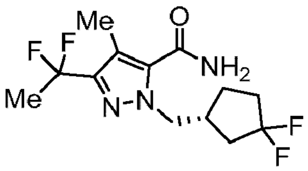

To a solution of (R)-1-((3,3-difluorocyclopentyl)methyl)-3-(1,1-difluoroethyl)-4-methyl-1H-pyrazole-5-carboxylic acid (Intermediate 25, 2.5 g, 8.11 mmol) in dry DMF (30 mL) were added HATU (4.62 g, 12.2 mmol), DIPEA (7 mL, 40.6 mmol) and $NH_4Cl$ (1.3 g, 24.4 mmol) and the resulting reaction mixture was stirred at rt for 2 h. The reaction mixture was diluted with ice cold water (100 mL) and stirred for 30 min. The precipitated solid was collected by filtration and dried under reduced pressure to afford the title compound (2.0 g) which was used without additional purification. LCMS m/z = 308 $[M+H]^+$.

**Intermediate 30-33 [HATU Amidation]**

The title compounds were prepared from the appropriate carboxylic acid using an analogous method to that described for Intermediate 29.

| Intermediate | Name/Structure/Data |
|---|---|
| 30 | (S)-1-((3,3-Difluorocyclopentyl)methyl)-3-(1,1-difluoroethyl)-4-methyl-1H-pyrazole-5-carboxamide 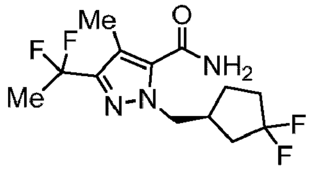 $RCO_2H$: (S)-1 -((3,3 -difluorocyclopentyl)methyl)-3 -(1, 1-difluoroethyl)-4-methyl-1H-pyrazole-5-carboxylic acid (Intermediate 26) Yield: 4.0 g, 64%. LCMS m/z = 308 $[M+H]^+$. |
| 31 | 1-((3,3-Difluoro-1-methylcyclopentyl)methyl)-3-(1,1-difluoroethyl)-4-methyl-1H-pyrazole-5-carboxamide 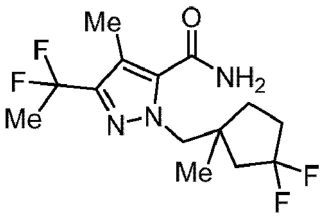 $RCO_2H$: 1-((3,3-difluoro-1-methylcyclopentyl)methyl)-3-(1,1-difluoroethyl)-4-methyl-1H-pyrazole-5-carboxylic acid (Intermediate 24) Yield: 1.75 g, 92%. LCMS m/z = 322 $[M+H]^+$. |
| 32 | 1-((3,3-difluoro-1-methylcyclobutyl)methyl)-3-(1,1-difluoroethyl)-4-methyl-1H-pyrazole-5-carboxamide 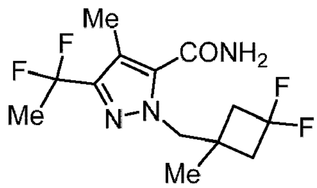 $RCO_2H$: 1-((3,3-difluoro-1-methylcyclobutyl)methyl)-3-(1,1-difluoroethyl)-4-methyl-1H-pyrazole-5-carboxylic acid (Intermediate 28) Yield: 6.5 g, 93%. LCMS m/z = 309 $[M+H]^+$. |
| 33 | 1-((3,3-difluorocyclobutyl)methyl)-3-(1,1-difluoroethyl)-4-methyl-1H-pyrazole-5-carboxamide 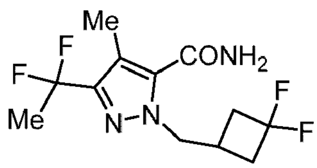 $RCO_2H$: 1-((3,3-difluorocyclobutyl)methyl)-3-(1,1-difluoroethyl)-4-methyl-1H-pyrazole-5 - carboxylic acid (Intermediate 27) Yield: 4.9 g, 98%. LCMS m/z = 295 $[M+H]^+$. |

**Intermediate 34**

4-Bromo-2-carbamoylpyridine 1-oxide

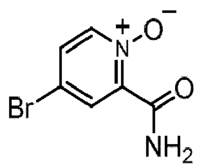

To a solution of 4-bromopicolinamide (300 mg, 1.49 mmol) in dry DCM (30 mL) was added m-CPBA (766 mg, 4.46 mmol) at rt and the suspension was stirred at rt for 48 h. The reaction mixture was concentrated under reduced pressure and the solids were collected by filtration, washed with DCM (10 mL) and dried under reduced pressure to afford the title compound (350 mg, crude). LCMS m/z = 217 $[M+H]^+$.

**Example Ia**

(R)-2-Carbamoyl-4-(1-((3,3-difluorocyclopentyl)methyl)-3-(1,1-difluoroethyl)-4-methyl-1H-pyrazole-5-carboxamido)pyridine 1-oxide

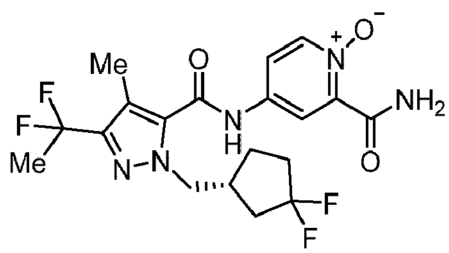

The reaction was carried out in duplicate.

A mixture of (R)-1-((3,3-difluorocyclopentyl)methyl)-3-(1,1-difluoroethyl)-4-methyl-1H-pyrazole-5-carboxamide (Intermediate 29, 100 mg, 0.33 mmol), 4-bromo-2-carbamoylpyridine 1-oxide (Intermediate 34, 84.9 mg, 0.39 mmol) and $Cs_2CO_3$ (211 mg, 0.65 mmol) in dioxane (10 mL) was degassed in a sealed tube with Ar for 20 min. To this were added copper(I) trifluoromethanesulfonate benzene complex (49 mg, 0.097 mmol) and trans-N,N-cyclohexyl-1,2-diamine (13.7 mg, 0.097 mmol) under Ar and the reaction mixture was heated at 90 °C for 16 h.

The two batches were combined and the reaction mixture was filtered through a pad of celite and the filtrate was concentrated under reduced pressure. The residue was purified by prep-HPLC (Sunfire C18, 150 x 19 mm, 5 $\mu$m; 0-100% MeCN/$H_2O$ (10 mM $NH_4HCO_3$)) to afford the title compound (24.1 mg, 10%). LCMS m/z = 444 $[M+H]^+$. $^1$H-NMR (500 MHz, DMSO-$d_6$) $\delta$: 11.17 (s, 1H), 10.61 (s, 1H) 8.60 (d, 1H), 8.39 (d, 1H), 8.27 (d, 1H), 7.87 (dd, 1H), 4.27 (d, 2H), 2.67-2.58 (m, 1H), 2.24 (s, 3H), 2.18-1.75 (m, 8H), 1.57-1.5 (m, 1H).

**Example 2-4**

The title compounds were prepared from the appropriate carboxamide ($RCONH_2$) and 4-bromo-2-carbamoylpyridine 1-oxide (Intermediate 34) using an analogous method to that described for Example 1

| Example | Name, Structure, HPLC, Data |
|---|---|
| 1b | (S)-2-Carbamoyl-4-(1-((3,3-difluorocyclopentyl)methyl)-3-(1,1-difluoroethyl)-4-methyl-1H-pyrazole-5-carboxamido)pyridine 1-oxide<br>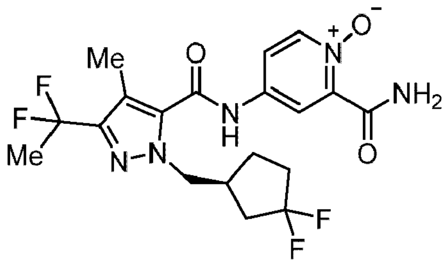<br>$RCONH_2$: (S)-1-((3,3-Difluorocyclopentyl)methyl)-3-(1,1-difluoroethyl)-4-methyl-1H-pyrazole-5-carboxamide (Intermediate 30)<br>Prep-HPLC (XSelect C18, 250 x 19 mm, 5 $\mu$m; 0-100% MeCN/$H_2O$ (10 mM $NH_4HCO_3$)).<br>Yield: 22.1 mg, 7%; LCMS m/z = 444 $[M+H]^+$. $^1$H-NMR (500 MHz, DMSO-$d_6$) $\delta$: 11.16 (s, 1H), 10.60 (d, 1H), 8.60 (d, 1H), 8.39 (d, 1H), 8.27 (d, 1H), 7.87 (dd, 1H), 4.27 (d, 2H), 2.67-2.58 (m, 1H), 2.24 (s, 3H), 2.18-1.75 (m, 8H), 1.57-1.5 (m, 1H). |
| 3 | 2-carbamoyl-4-(1-((3,3-difluorocyclobutyl)methyl)-3-(1,1-difluoroethyl)-4-methyl-1H-pyrazole-5-carboxamido)pyridine 1-oxide<br>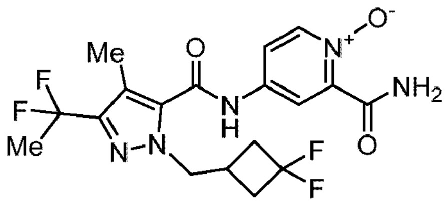<br>$RCONH_2$: 1-((3,3-difluorocyclobutyl)methyl)-3-(1,1-difluoroethyl)-4-methyl-1H-pyrazole-5-carboxamide (Intermediate 33)<br>Column Chromatography ($SiO_2$, 13-15% EtOAc/PE)<br>Yield: 50 mg, 15%; LCMS m/z = 430 $[M+H]^+$; $^1$H-NMR (500 MHz, DMSO-$d_6$) $\delta$: 11.18 (s, 1H), 10.60 (s, 1H), 8.62 (d, 1H), 8.40 (d, 1H), 8.28 (d, 1H), 7.89 (dd, 1H), 4.37 (d, 2H), 2.64-2.61 (m, 3H), 2.50-2.41 (m, 2H), 2.23 (s, 3H), 2.03 (t, 3H). |
| 4 | 2-carbamoyl-4-(1-((3,3-difluoro-1-methylcyclobutyl)methyl)-3-(1,1 -difluoroethyl)-4-methyl- 1H-pyrazole-5-carboxamido)pyridine 1-oxide<br>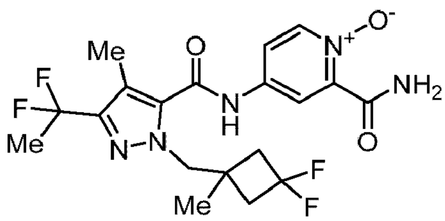<br>$RCONH_2$: 1-((3,3-difluoro-1-methylcyclobutyl)methyl)-3-(1,1-difluoroethyl)-4-methyl-1H-pyrazole-5-carboxamide (Intermediate 32)<br>Column Chromatography ($SiO_2$, 13-15% EtOAc/PE)<br>Yield: 50 mg, 15%; LCMS m/z = 444 $[M+H]^+$; $^1$H-NMR (500 MHz, DMSO-$d_6$) $\delta$: 11.20 (s, 1H), 10.59 (s, 1H), 8.60 (d, 1H), 8.39 (d, 1H), 8.27 (d, 1H), 7.89 (dd, 1H), 4.35 (s, 2H), 2.76-2.72 (m, 2H), 2.34-2.24 (m, 5H), 2.03 (t, 3H), 1.07 (s, 3H). |

**Example 5a/5b**

2-carbamoyl-4-(1-((3,3-difluoro-1-methylcyclopentyl)methyl)-3-(1,1-difluoroethyl)-4-methyl-1H-pyrazole-5-carboxamido)pvridine 1-oxide, synthesized in the form of enantiomer 1 of 2-carbamoyl-4-(1-((3,3-difluoro-1-methylcyclopentyl)methyl)-3-(1,1-difluoroethyl)-4-methyl-1H-pyrazole-5-carboxamido)pyridine 1-oxide (5a) and enantiomer 2 of 2-carbamoyl-4-(1-((3,3-difluoro-1-methylcyclopentyl)methyl)-3-(1,1-difluoroethyl)-4-methyl-1H-pyrazole-5-carboxamido)pyridine 1-oxide (5b)

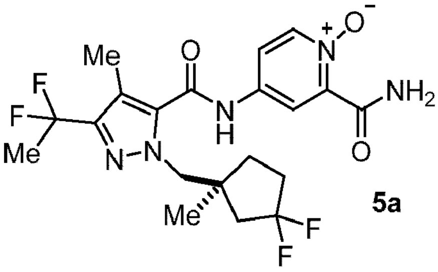

**5a**

and

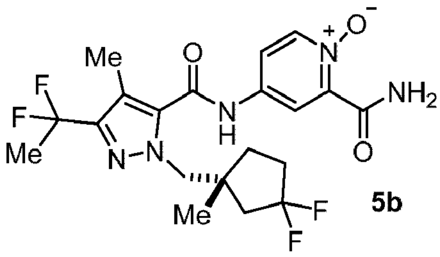

**5b**

To a stirred solution of 1-((3,3-difluoro-1-methylcyclopentyl)methyl)-3-(1,1-difluoroethyl)-4-methyl-1H-pyrazole-5-carboxamide (Intermediate 31, 500 mg, 1.56 mmol) in dioxane (10 mL) in a sealed tube were added 4-bromo-2-carbamoylpyridine 1-oxide (Intermediate 34, 408 mg, 1.87 mmol), $Cs_2CO_3$ (1.27 g, 3.90 mmol) at rt, and the reaction mixture degassed with argon for 10 min. To the reaction mixture were added trans N,N-dimethyl 1,2-diamino cyclohexane (66.4 mg, 0.47 mmol) and copper (I) trifluoromethane sulfonate benzene complex (196 mg, 0.39 mmol) at rt and the resulting reaction mixture was stirred at 85 °C for 16 h. The reaction mixture was cooled to rt, filtered through a pad of celite and washed with EtOAc (20 mL). The filtrate was washed with water (25 mL), dried ($Na_2SO_4$) and concentrated under reduced pressure. The residue was purified by Grace flash column chromatography (45% acetonitrile in 0.1% aq. formic acid) to afford 2-carbamoyl-4-(1-((3,3-difluoro-1-methylcyclopentyl)methyl)-3-(1,1-difluoroethyl)-4-methyl-1H-pyrazole-5-carboxamido)pyridine 1 -oxide which was further purified by prep-SFC (Lux Cellulose-4, 250 x 30 mm, 5 mm; 20% MeOH in $CO_2$) to afford:

**Peak 1, Example 5a:** (65 mg, 9%). LCMS m/z = 458 $[M+H]^+$. $^1H$-NMR (500 MHz, DMSO-$d_6$,) δ: 11.21 (s, 1H), 10.80-10.62 (m, 1H), 8.58-8.10 (m, 3H), 7.82 (s, 1H), 4.34 (brs, 2H), 2.37-2.35 (m, 1H), 2.32-2.25 (m, 3H), 2.13-2.08 (m, 2H), 2.01 (t, 3H), 1.85-1.81 (m, 2H), 1.52-1.45 (m, 2H), 1.23 (s, 1H), 0.98 (s, 3H). Chiral SFC: column: two Chiracel OX-H (4.6 mm x 250 mm), 5 μm in sequence; co-solvent: MeOH, flow : 3 mL/min; % of co-solvent : 15%; ABPR: 1500 psi; T: 30 °C; $R_t$ = 45.68 min (first eluting).

**Peak 2, Example 5b:** (70 mg, 10%). LCMS m/z = 458 $[M+H]^+$. $^1H$-NMR (500 MHz, DMSO-$d_6$,) δ: 11.20 (s, 1H), 10.62 (s, 1H), 8.57 (s, 1H), 8.36-8.25 (m, 2H), 7.86-7.85 (m, 1H), 4.29 (s, 2H), 2.36-2.32 (m, 1H), 2.25 (s, 3H), 1.19-1.12 (m, 2H), 2.08-2.01 (m,3H), 1.87-1.79 (m, 1H), 1.25 (s, 1H), 0.95 (s, 3H). Chiral SFC: column: two Chiracel OX-H (4.6 mm x 250 mm), 5 μm in sequence; co-solvent: MeOH, flow : 3 mL/min; % of co-solvent : 15%; ABPR: 1500 psi; T: 30 °C; $R_t$ = 49.76 min (second eluting).

**Example 7**

4-(1-((3,3-difluorocyclobutyl)methyl)-3-(1,1-difluoroethyl)-4-(trifluoromethyl)-1H-pyrazole-5-carboxamido)picolinic acid

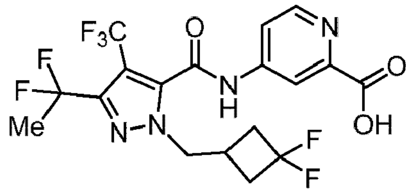

Part 1: To a solution of 1-((3,3-difluorocyclobutyl)methyl)-3-(1,1-difluoroethyl)-4-(trifluoromethyl)-1H-pyrazole-5-carboxylic acid (Intermediate 7, 0.08 g, 0.23 mmol) in pyridine (3 mL) were added $POCl_3$ (0.07 mL, 0.69 mmol), methyl 4-aminopyridine-2-carboxylate (0.04 g, 0.34 mmol) at 0 °C and the reaction mixture was stirred at rt for 2 h. The reaction mixture was concentrated under reduced pressure and diluted with cold water and the aqueous layer was extracted with EtOAc (3x 50 mL). The combined organics were washed with brine, dried ($Na_2SO_4$) and concentrated under reduced pressure. The residue was taken up in THF (6 mL) and 1M NaOH (3 mL) was added at 0 °C and the reaction mixture was stirred at rt for 16 h. The reaction mixture was concentrated under reduced pressure, diluted with water and the aqueous phase extracted with EtOAc (3x 60 mL). The combined organics were washed with brine, dried ($Na_2SO_4$) and evaporated to dryness *in vacuo*. The residue was purified by reverse phase prep-HPLC (XBridge C18, 250 x 19 mm, 10 μm; 20-55% MeCN/$H_2O$ (20 mM $NH_4HCO_3$)) to afford the title compound (10 mg, 12%). LCMS m/z = 469 $[M+H]^+$; $^1$H-NMR (400 MHz, DMSO-$d_6$,) δ: 8.63 (s, 1H), 8.29 (s, 1H), 7.76 (d, 1H), 4.38 (d, 2H), 2.66-2.50 (m, 5H), 2.13-2.03 (m, 3H).

**Example 8**

5-(1-((3,3-difluorocyclobutyl)methyl)-3-(1,1-difluoroethyl)-4-(trifluoromethyl)-1H-pyrazole-5-carboxamido)picolinic acid

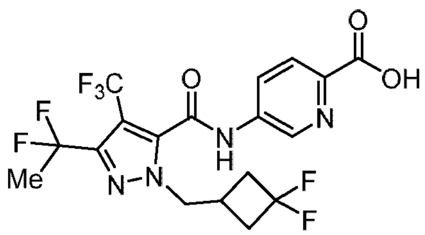

The title compound was prepared (20 mg, 18%) from 1-((3,3-difluorocyclobutyl)methyl)-3-(1,1-difluoroethyl)-4-(trifluoromethyl)-1H-pyrazole-5-carboxylic acid (Intermediate 7) and methyl 5-aminopicolinate using an analogous method to that described for Example 7. Prep-HPLC (Kinetex EVO C18, 250 x 21.2 mm, 5 μm; 20-45% MeCN/$H_2O$ (0.1% $HCO_2H$); LCMS: m/z = 469 $[M+H]^+$; $^1$H-NMR (400 MHz, DMSO-$d_6$) δ: 13.11 (s, 1H), 11.72 (s, 1 H), 8.88 (s, 1 H), 8.52-8.28 (m, 1 H), 8.20-8.11 (m, 1 H), 4.38 (d, 2 H), 2.75-2.50 (m, 5 H), 2.13-2.03 (m, 3 H).

**Example 9**

4-(3-cyclopropyl-1-((4,4-difluorocyclohexyl)methyl)-4-(trifluoromethyl)-1H-pyrazole-5-carboxamido)picolinic acid

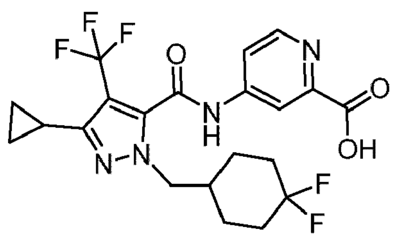

The title compound was prepared (55 mg, 45%) from 3-cyclopropyl-1-((4,4-difluorocyclohexyl)methyl)-4-(trifluoromethyl)-1H-pyrazole-5-carboxylic acid (Intermediate 23) and methyl 5-aminopicolinate using an analogous method to that described for Example 7. LCMS m/z = 473 $[M+H]^+$; $^1$H-NMR (400 MHz, DMSO-$d_6$) δ: 13.23 (brs, 1H), 11.57 (s, 1H), 8.64 (d, 1H), 8.35 (s, 1H),7.81-7.79 (m, 1H), 3.99 (d, 2H), 1.95-1.82 (m, 4H), 1.79-1.67 (m, 2H), 1.58-1.55 (m, 2H), 1.23-1.14 (m, 2H),1.00-0.98 (m, 2H), 0.97-0.96 (m, 2H).

**Example 10**

1-(cyclohexylmethyl)-N-(2-(dimethylphosphoryl)pyridin-4-yl)-4-(trifluoromethyl)-1H-pyrazole-5-carboxamide

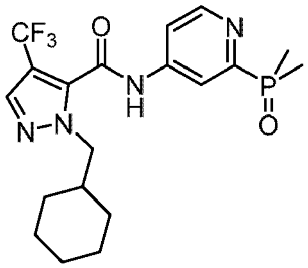

To a solution of 1-(cyclohexylmethyl)-4-(trifluoromethyl)-1H-pyrazole-5-carboxylic acid (Intermediate 35, 100 mg, 0.363 mmol) in pyridine (2 mL) was added $POCl_3$ (0.068 mL, 0.726 mmol, 2 eq) drop wise at 0 °C under an argon atmosphere. The reaction mixture was stirred for 10 min and then (4-aminopyridin-2-yl)dimethylphosphine oxide (92.7 mg, 0.545 mmol, 1.5 eq) was added at the same temperature. The reaction mixture was allowed to warm to rt and was stirred for 3 h. The reaction mixture was cooled to 0 °C, ice-water (10 mL) was added and acidified with 1N HCl up to pH = ~3-4. The crude product was extracted with ethyl acetate (2x 30 mL). The combined organic layers were dried over anhydrous $Na_2SO_4$ and concentrated under reduced pressure. The crude product was purified by GRACE flash column chromatography using 0-10% MeOH in dichloromethane as an eluent to afford the title compound (39 mg, 25%). LCMS: m/z = 429 $[M+H]^+$; $^1$H-NMR (400 MHz, DMSO-$d_6$) $\delta$: 11.53 (s, 1H), 8.70 (d, 1H), 8.26 (dd, 1H), 8.06 (s, 1H), 7.75-7.73 (m, 1H), 4.06 (d, 2H), 1.87-1.81 (m, 1H), 1.67-1.48 (m, 1H), 1.23-1.08 (m, 3H), 0.97-0.89 (m, 2H).

The following prophetic examples could be synthesized according to the general schemes and by analogy to the synthetic procedures described above:

| Structure | Prophetic example number | Chemical name |
|---|---|---|
| 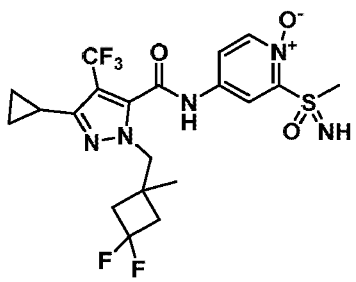 | 11 | 4-(3-cyclopropyl-1-((3,3-difluoro-1-methylcyclobutyl) methyl)-4-(trifluoromethyl)-1H-pyrazole-5-carboxamido)-2-(S-methylsulfonimidoyl)pyridine 1-oxide |
| 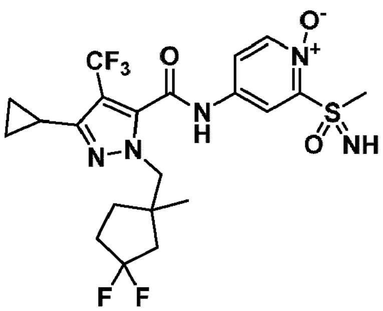 | 12 | 4-(3-cyclopropyl-1-((3,3-difluoro-1-methylcyclopentyl)methyl)-4-(trifluoromethyl)-1H-pyrazole-5-carboxamido)-2-(S-methylsulfonimidoyl) pyridine 1-oxide |

(continued)

| Structure | Prophetic example number | Chemical name |
|---|---|---|
| 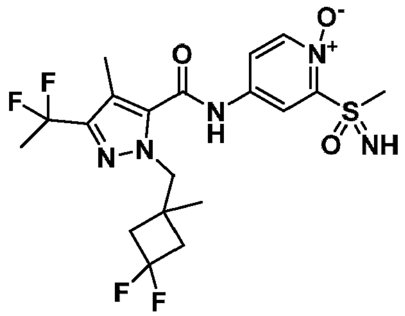 | 13 | 4-(1-((3,3-difluoro-1-methylcyclobutyl)methyl)-3 -(1,1-difluoroethyl)-4-methyl-1H-pyrazole-5-carboxamido)-2-(S-methylsulfonimidoyl)pyridine 1-oxide |
| 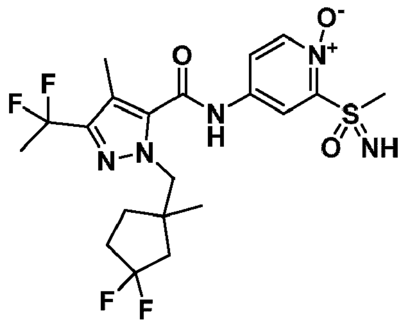 | 14 | 4-(1-((3,3-difluoro-1-methylcyclopentyl)methyl)-3-(1,1-difluoroethyl)-4-methyl-1H-pyrazole-5-carboxamido)-2-(S-methylsulfonimidoyl)pyridine 1-oxide |
| 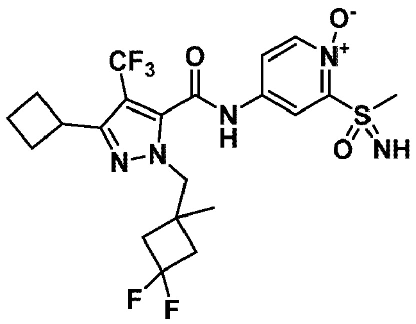 | 15 | 4-(3-cyclobutyl-1-((3,3-difluoro-1-methylcyclobutyl) methyl)-4-(trifluoromethyl)-1H-pyrazole-5-carboxamido)-2-(S-methylsulfonimidoyl)pyridine 1-oxide |
| 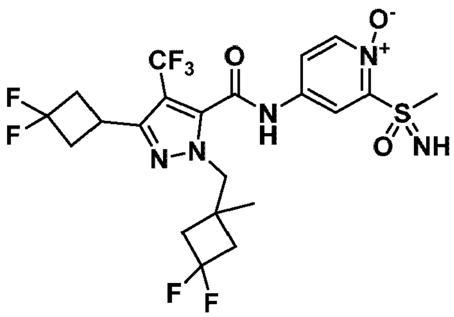 | 16 | 4-(1-((3,3-difluoro-1-methylcyclobutyl)methyl)-3 -(3,3-difluorocyclobutyl)-4-(trifluoromethyl)-1H-pyrazole-5-carboxamido)-2-(S-methylsulfonimidoyl) pyridine 1-oxide |

(continued)

| Structure | Prophetic example number | Chemical name |
|---|---|---|
| 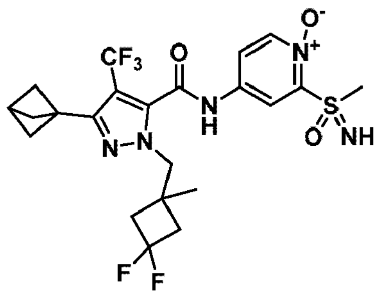 | 17 | 4-(3-(bicyclo[1.1.1]pentan-1-yl)-1-((3,3-difluoro-1-methylcyclobutyl)methyl)-4-(trifluoromethyl)-1H-pyrazole-5-carboxamido)-2-(S-methylsulfonimidoyl) pyridine 1-oxide |
| 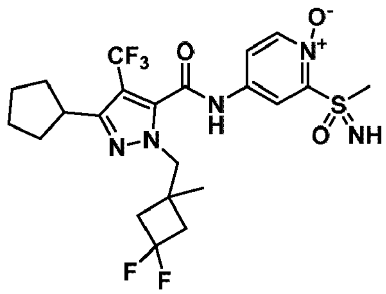 | 18 | 4-(3-cyclopentyl-1-((3,3-difluoro-1-methylcyclobutyl) methyl)-4-(trifluoromethyl)-1H-pyrazole-5-carboxamido)-2-(S-methylsulfonimidoyl)pyridine 1-oxide |
| 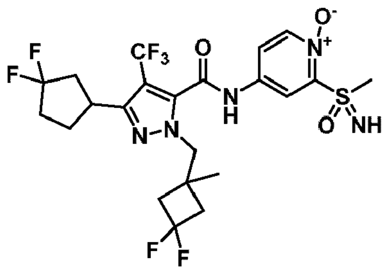 | 19 | 4-(1-((3,3-difluoro-1-methylcyclobutyl)methyl)-3-(3,3-difluorocyclopentyl)-4-(trifluoromethyl)-1H-pyrazole-5-carboxamido)-2-(S-methylsulfonimidoyl) pyridine 1-oxide |
| 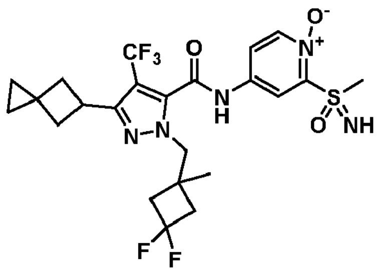 | 20 | 4-(1-((3,3-difluoro-1-methylcyclobutyl)methyl)-3-(spiro[2.3]hexan-5-yl)-4-(trifluoromethyl)-1H-pyrazole-5-carboxamido)-2-(S-methylsulfonimidoyl) pyridine 1-oxide |
| 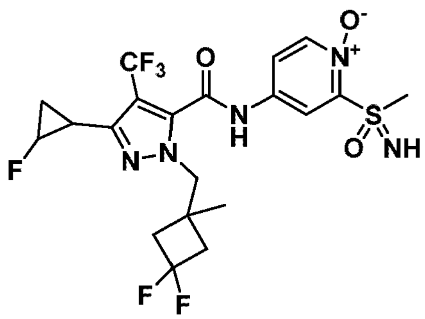 | 21 | 4-(1-((3,3-difluoro-1-methylcyclobutyl)methyl)-3-(2-fluorocyclopropyl)-4-(trifluoromethyl)-1H-pyrazole-5-carboxamido)-2-(S-methylsulfonimidoyl)pyridine 1-oxide |

(continued)

| Structure | Prophetic example number | Chemical name |
|---|---|---|
| 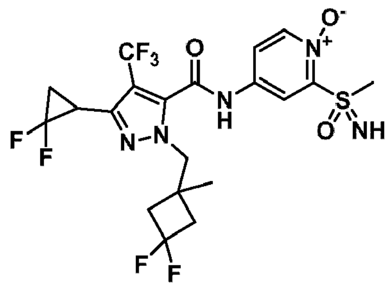 | 22 | 4-(1-((3,3-difluoro-1-methylcyclobutyl)methyl)-3-(2,2-difluorocyclopropyl)-4-(trifluoromethyl)-1H-pyrazole-5-carboxamido)-2-(S-methylsulfonimidoyl) pyridine 1-oxide |
| 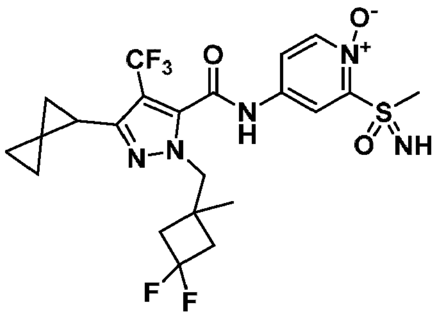 | 23 | 4-(1-((3,3-difluoro-1-methylcyclobutyl)methyl)-3 -(spiro[22]pentan-1-yl)-4-(trifluoromethyl)-1H-pyrazole-5-carboxamido)-2-(S-methylsulfonimidoyl) pyridine 1-oxide |
| 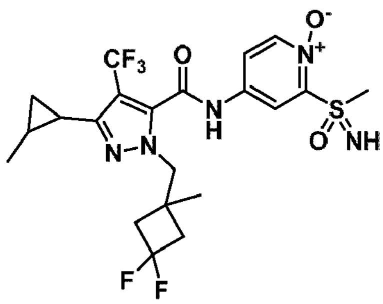 | 24 | 4-(1-((3,3-difluoro-1-methylcyclobutyl)methyl)-3-(2-methylcyclopropyl)-4-(trifluoromethyl)-1H-pyrazole-5-carboxamido)-2-(S-methylsulfonimidoyl)pyridine 1-oxide |
| 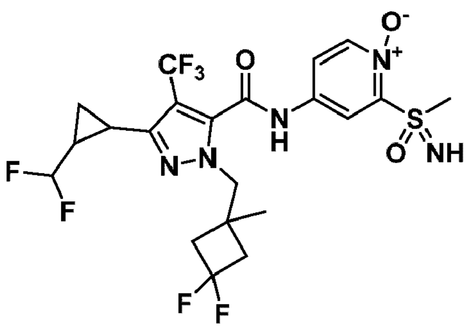 | 25 | 4-(1-((3,3-difluoro-1-methylcyclobutyl)methyl)-3-(2-(difluoromethyl)cyclopropyl)-4-(trifluoromethyl)-1H-pyrazole-5-carboxamido)-2-(S-methylsulfonimidoyl)pyridine 1-oxide |
| 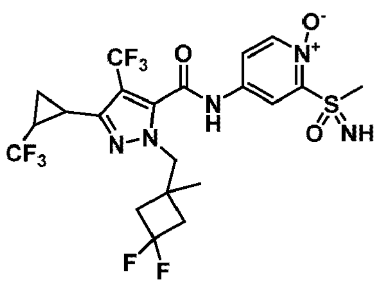 | 26 | 4-(1-((3,3-difluoro-1-methylcyclobutyl)methyl)-4-(trifluoromethyl)-3-(2-(trifluoromethyl)cyclopropyl)-1H-pyrazole-5-carboxamido)-2-(S-methylsulfonimidoyl)pyridine 1-oxide |

(continued)

| Structure | Prophetic example number | Chemical name |
|---|---|---|
| 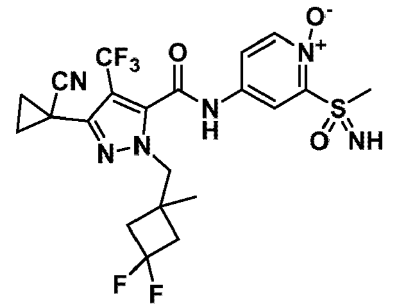 | 27 | 4-(3-(1-cyanocyclopropyl)-1-((3,3-difluoro-1-methylcyclobutyl)methyl)-4-(trifluoromethyl)-1H-pyrazole-5-carboxamido)-2-(S-methylsulfonimidoyl) pyridine 1-oxide |
| 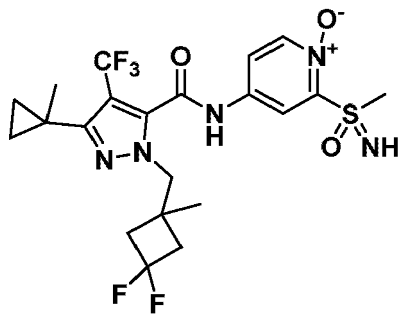 | 28 | 4-(1-((3,3-difluoro-1-methylcyclobutyl)methyl)-3-(1-methylcyclopropyl)-4-(trifluoromethyl)-1H-pyrazole-5-carboxamido)-2-(S-methylsulfonimidoyl)pyridine 1-oxide |
| 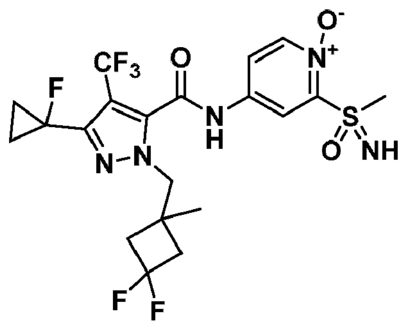 | 29 | 4-(1-((3,3-difluoro-1-methylcyclobutyl)methyl)-3-(1-fluorocyclopropyl)-4-(trifluoromethyl)-1H-pyrazole-5-carboxamido)-2-(S-methylsulfonimidoyl)pyridine 1-oxide |
| 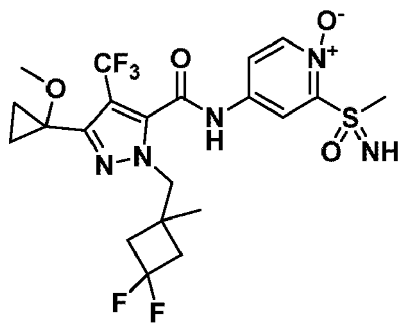 | 30 | 4-(1-((3,3-difluoro-1-methylcyclobutyl)methyl)-3-(1-methoxycyclopropyl)-4-(trifluoromethyl)-1H-pyrazole-5-carboxamido)-2-(S-methylsulfonimidoyl) pyridine 1-oxide |

(continued)

| Structure | Prophetic example number | Chemical name |
|---|---|---|
| 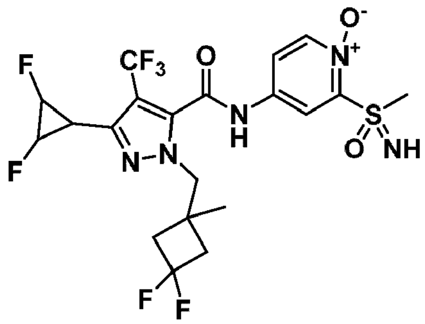 | 31 | 4-(1-((3,3-difluoro-1-methylcyclobutyl)methyl)-3-(2,3 - difluorocyclopropyl)-4-(trifluoromethyl)-1H-pyrazole-5-carboxamido)-2-(S-methylsulfonimidoyl) pyridine 1-oxide |
| 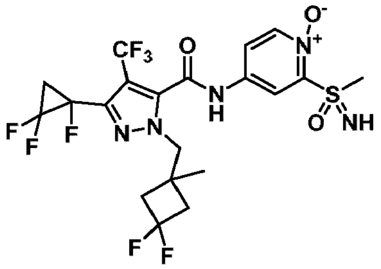 | 32 | 4-(1-((3,3-difluoro-1-methylcyclobutyl)methyl)-3-(1,2,2-trifluorocyclopropyl)-4-(trifluoromethyl)-1H-pyrazole-5-carboxamido)-2-(S-methylsulfonimidoyl) pyridine 1-oxide |
| 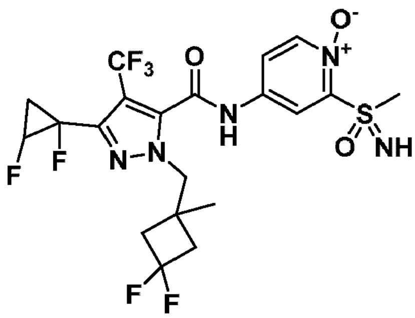 | 33 | 4-(1-((3,3-difluoro-1-methylcyclobutyl)methyl)-3-(1,2-difluorocyclopropyl)-4-(trifluoromethyl)-1H-pyrazole-5-carboxamido)-2-(S-methylsulfonimidoyl) pyridine 1-oxide |
| 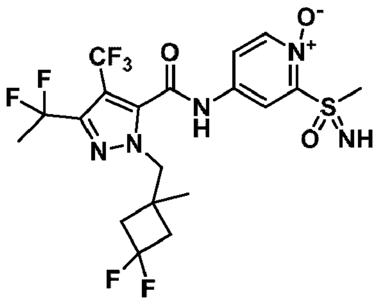 | 34 | 4-(1-((3,3-difluoro-1-methylcyclobutyl)methyl)-3-(1,1-difluoroethyl)-4-(trifluoromethyl)-1H-pyrazole-5-carboxamido)-2-(S-methylsulfonimidoyl)pyridine 1-oxide |
| 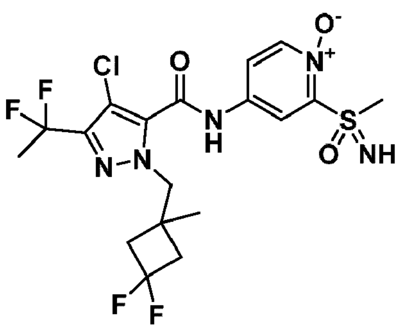 | 35 | 4-(4-chloro-1-((3,3-difluoro-1-methylcyclobutyl) methyl)-3-(1,1-difluoroethyl)-1H-pyrazole-5-carboxamido)-2-(S-methylsulfonimidoyl)pyridine 1-oxide |

(continued)

| Structure | Prophetic example number | Chemical name |
|---|---|---|
| 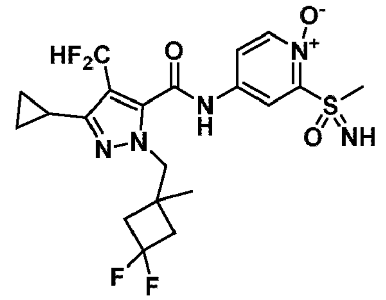 | 36 | 4-(3-cyclopropyl-1-((3,3-difluoro-1-methylcyclobutyl) methyl)-4-(difluoromethyl)-1H-pyrazole-5-carboxamido)-2-(S-methylsulfonimidoyl)pyridine 1-oxide |
| 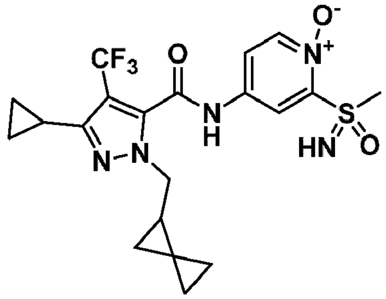 | 38 | 4-(3-cyclopropyl-1-(spiro[2.2]pentan-1-ylmethyl)-4-(trifluoromethyl)-1H-pyrazole-5-carboxamido)-2-(S-methylsulfonimidoyl)pyridine 1-oxide |
| 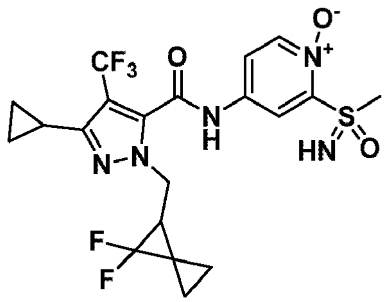 | 39 | 4-(3-cyclopropyl-1-((2,2-difluorospiro[2.2]pentan-1-yl)methyl)-4-(trifluoromethyl)-1H-pyrazole-5-carboxamido)-2-(S-methylsulfonimidoyl)pyridine 1-oxide |
| 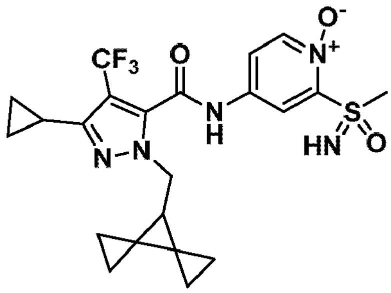 | 40 | 4-(3-cyclopropyl-1-(dispiro[2.0.24.13]heptan-7-ylmethyl)-4-(trifluoromethyl)-1H-pyrazole-5-carboxamido)-2-(S-methylsulfonimidoyl)pyridine 1-oxide |
| 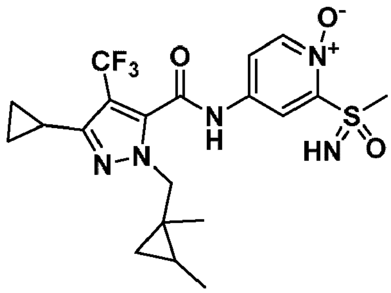 | 41 | 4-(3-cyclopropyl-1-((1,2-dimethylcyclopropyl) methyl)-4-(trifluoromethyl)-1H-pyrazole-5-carboxamido)-2-(S-methylsulfonimidoyl)pyridine 1-oxide |

(continued)

| Structure | Prophetic example number | Chemical name |
|---|---|---|
| 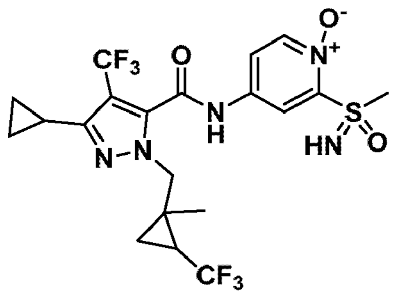 | 42 | 4-(3-cyclopropyl-1-((1-methyl-2-(trifluoromethyl) cyclopropyl)methyl)-4-(trifluoromethyl)-1H-pyrazole-5-carboxamido)-2-(S-methylsulfonimidoyl)pyridine 1-oxide |
| 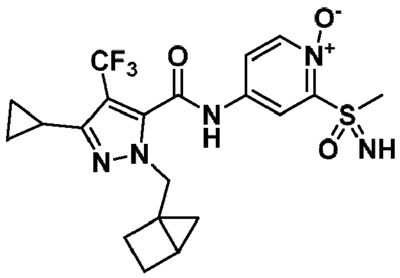 | 43 | 4-(1-(bicyclo[2.1.0]pentan-1-ylmethyl)-3-cyclopropyl-4-(trifluoromethyl)-1H-pyrazole-5-carboxamido)-2-(S-methylsulfonimidoyl)pyridine 1-oxide |
| 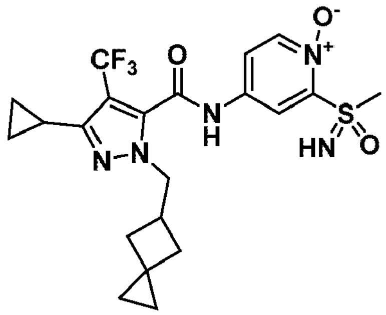 | 44 | 4-(3-cyclopropyl-1-(spiro[2.3]hexan-5-ylmethyl)-4-(trifluoromethyl)-1H-pyrazole-5-carboxamido)-2-(S-methylsulfonimidoyl)pyridine 1-oxide |
| 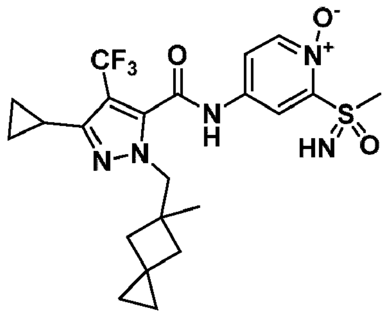 | 45 | 4-(3-cyclopropyl-1-((5-methylspiro[2.3]hexan-5-yl) methyl)-4-(trifluoromethyl)-1H-pyrazole-5-carboxamido)-2-(S-methylsulfonimidoyl)pyridine 1-oxide |

(continued)

| Structure | Prophetic example number | Chemical name |
|---|---|---|
| 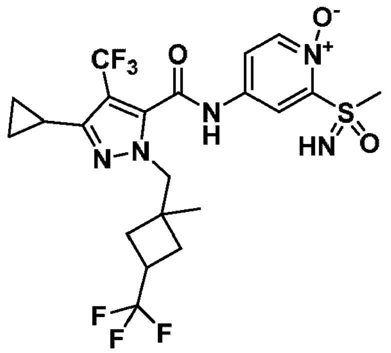 | 46 | 4-(3-cyclopropyl-1-((1-methyl-3-(trifluoromethyl) cyclobutyl)methyl)-4-(trifluoromethyl)-1H-pyrazole-5-carboxamido)-2-(S-methylsulfonimidoyl)pyridine 1-oxide |
| 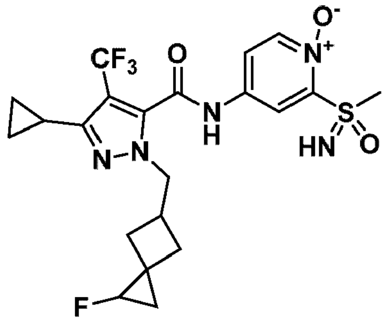 | 47 | 4-(3-cyclopropyl-1-((1-fluorospiro[2.3]hexan-5-yl) methyl)-4-(trifluoromethyl)-1H-pyrazole-5-carboxamido)-2-(S-methylsulfonimidoyl)pyridine 1-oxide |
| 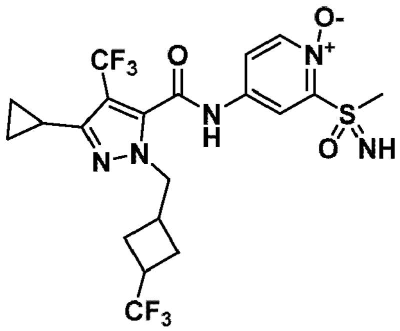 | 48 | 4-(3-cyclopropyl-4-(trifluoromethyl)-1-((3-(trifluoromethyl)cyclobutyl)methyl)-1H-pyrazole-5-carboxamido)-2-(S-methylsulfonimidoyl) pyridine 1-oxide |
| 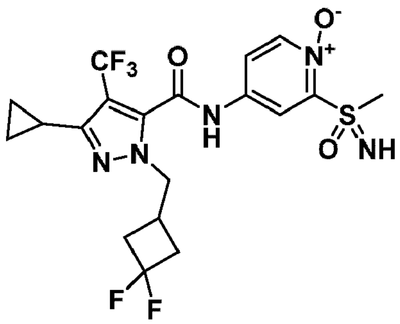 | 49 | 4-(3-cyclopropyl-1-((3,3-difluorocyclobutyl)methyl)-4-(trifluoromethyl)-1H-pyrazole-5-carboxamido)-2-(S-methylsulfonimidoyl)pyridine 1-oxide |

(continued)

| Structure | Prophetic example number | Chemical name |
|---|---|---|
| 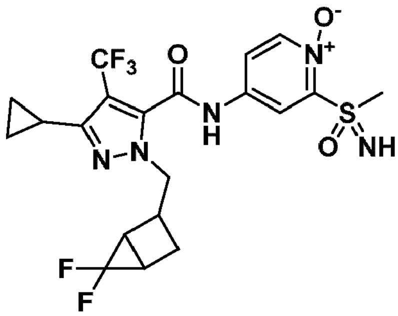 | 50 | 4-(3-cyclopropyl-1-((5,5-difluorobicyclo[2.1.0] pentan-2-yl)methyl)-4-(trifluoromethyl)-1H-pyrazole-5-carboxamido)-2-(S-methylsulfonimidoyl)pyridine 1-oxide |
| 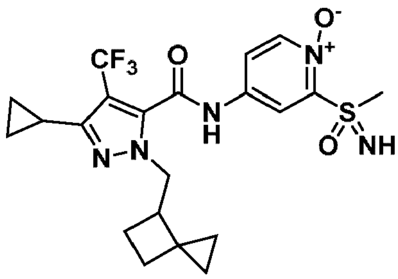 | 51 | 4-(3-cyclopropyl-1-(spiro[2.3]hexan-4-ylmethyl)-4-(trifluoromethyl)-1H-pyrazole-5-carboxamido)-2-(S-methylsulfonimidoyl)pyridine 1-oxide |
| 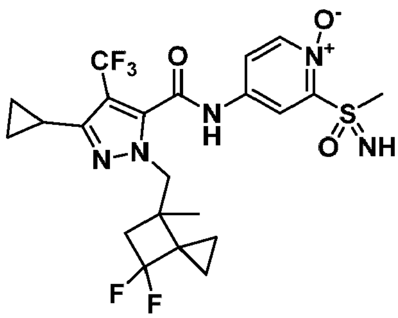 | 52 | 4-(3-cyclopropyl-1-((6,6-difluoro-4-methylspiro[2.3] hexan-4-yl)methyl)-4-(trifluoromethyl)-1H-pyrazole-5-carboxamido)-2-(S-methylsulfonimidoyl)pyridine 1-oxide |
| 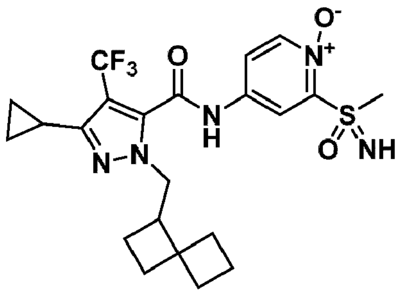 | 53 | 4-(3-cyclopropyl-1-(spiro[3.3]heptan-1-ylmethyl)-4-(trifluoromethyl)-1H-pyrazole-5-carboxamido)-2-(S-methylsulfonimidoyl)pyridine 1-oxide |
| 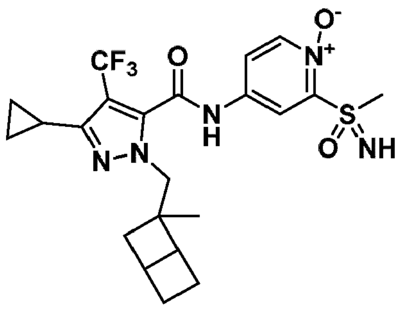 | 54 | 4-(3-cyclopropyl-1-((2-methylbicyclo[2.2.0]hexan-2-yl)methyl)-4-(trifluoromethyl)-1H-pyrazole-5-carboxamido)-2-(S-methylsulfonimidoyl)pyridine 1-oxide |

(continued)

| Structure | Prophetic example number | Chemical name |
|---|---|---|
| 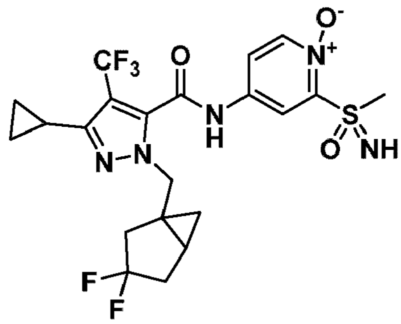 | 55 | 4-(3-cyclopropyl-1-((3,3-difluorobicyclo[3.1.0]hexan-1-yl)methyl)-4-(trifluoromethyl)-1H-pyrazole-5-carboxamido)-2-(S-methylsulfonimidoyl)pyridine 1-oxide |
| 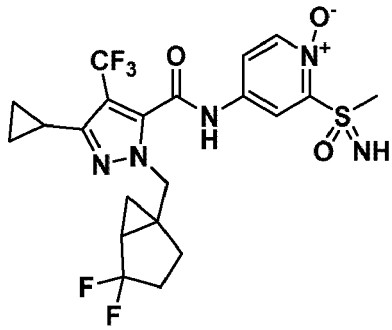 | 56 | 4-(3-cyclopropyl-1-((4,4-difluorobicyclo[3.1.0]hexan-1-yl)methyl)-4-(trifluoromethyl)-1H-pyrazole-5-carboxamido)-2-(S-methylsulfonimidoyl)pyridine 1-oxide |
| 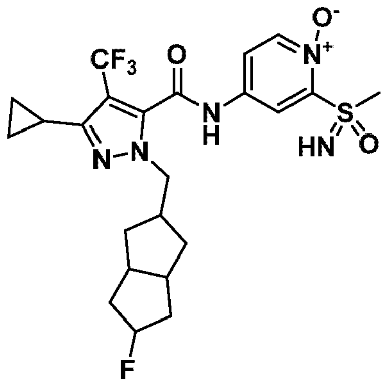 | 57 | 4-(3-cyclopropyl-1-((5-fluorooctahydropentalen-2-yl)methyl)-4-(trifluoromethyl)-1H-pyrazole-5-carboxamido)-2-(S-methylsulfonimidoyl)pyridine 1-oxide |
| 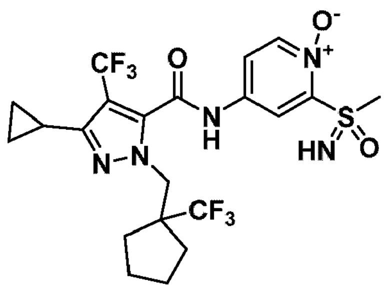 | 58 | 4-(3-cyclopropyl-4-(trifluoromethyl)-1-((1-(trifluoromethyl)cyclopentyl)methyl)-1H-pyrazole-5-carboxamido)-2-(S-methylsulfonimidoyl)pyridine 1-oxide |

(continued)

| Structure | Prophetic example number | Chemical name |
|---|---|---|
| 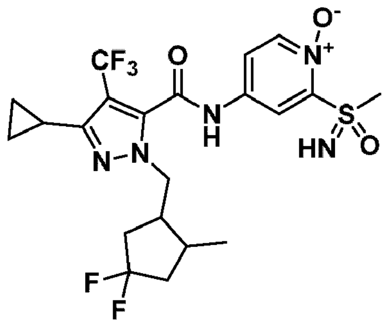 | 59 | 4-(3-cyclopropyl-1-((4,4-difluoro-2-methylcyclopentyl)methyl)-4-(trifluoromethyl)-1H-pyrazole-5-carboxamido)-2-(S-methylsulfonimidoyl) pyridine 1-oxide |
| 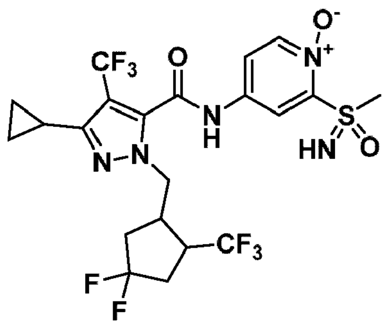 | 60 | 4-(3-cyclopropyl-1-((4,4-difluoro-2-(trifluoromethyl) cyclopentyl)methyl)-4-(trifluoromethyl)-1H-pyrazole-5-carboxamido)-2-(S-methylsulfonimidoyl)pyridine 1-oxide |
| 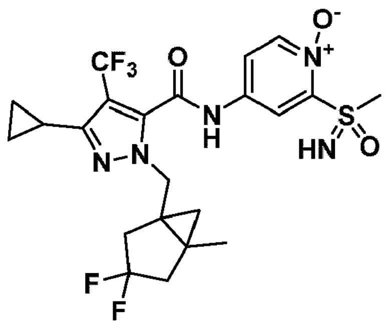 | 61 | 4-(3-cyclopropyl-1-((3,3-difluoro-5-methylbicyclo [3.1.0]hexan-1-yl)methyl)-4-(trifluoromethyl)-1H-pyrazole-5-carboxamido)-2-(S-methylsulfonimidoyl) pyridine 1-oxide |
| 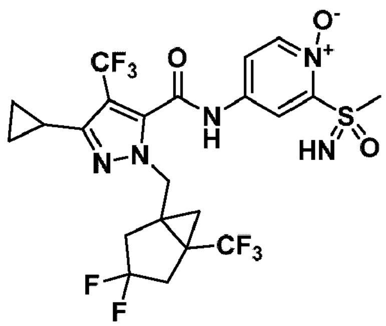 | 62 | 4-(3-cyclopropyl-1-((3,3-difluoro-5-(trifluoromethyl) bicyclo[3.1.0]hexan-1-yl)methyl)-4-(trifluoromethyl)-1H-pyrazole-5-carboxamido)-2-(S-methylsulfonimidoyl)pyridine 1-oxide |

(continued)

| Structure | Prophetic example number | Chemical name |
|---|---|---|
| 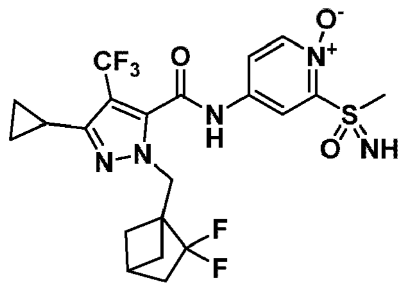 | 63 | 4-(3-cyclopropyl-1-((2,2-difluorobicyclo[2.1.1]hexan-1-yl)methyl)-4-(trifluoromethyl)-1H-pyrazole-5-carboxamido)-2-(S-methylsulfonimidoyl)pyridine 1-oxide |
| 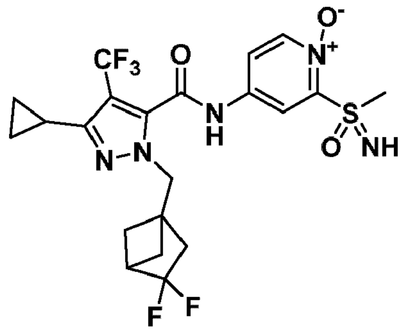 | 64 | 4-(3-cyclopropyl-1-((3,3-difluorobicyclo[2.1.1]hexan-1-yl)methyl)-4-(trifluoromethyl)-1H-pyrazole-5-carboxamido)-2-(S-methylsulfonimidoyl)pyridine 1-oxide |
| 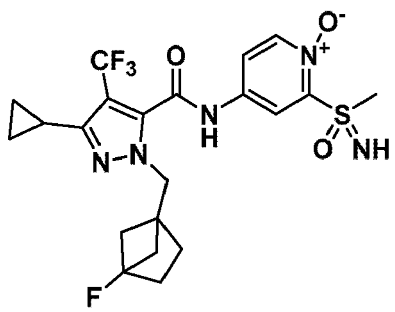 | 65 | 4-(3-cyclopropyl-1-((4-fluorobicyclo[2.1.1]hexan-1-yl)methyl)-4-(trifluoromethyl)-1H-pyrazole-5-carboxamido)-2-(S-methylsulfonimidoyl)pyridine 1-oxide |
| 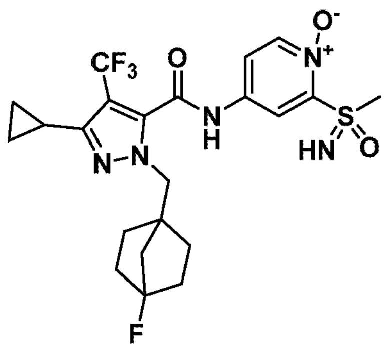 | 66 | 4-(3-cyclopropyl-1-((4-fluorobicyclo[2.2.1]heptan-1-yl)methyl)-4-(trifluoromethyl)-1H-pyrazole-5-carboxamido)-2-(S-methylsulfonimidoyl)pyridine 1-oxide |

(continued)

| Structure | Prophetic example number | Chemical name |
|---|---|---|
| 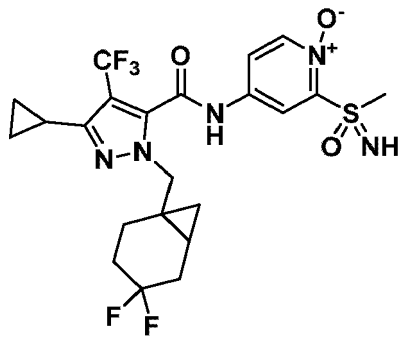 | 67 | 4-(3-cyclopropyl-1-((4,4-difluorobicyclo[4.1.0] heptan-1-yl)methyl)-4-(trifluoromethyl)-1H-pyrazole-5-carboxamido)-2-(S-methylsulfonimidoyl)pyridine 1-oxide |
| 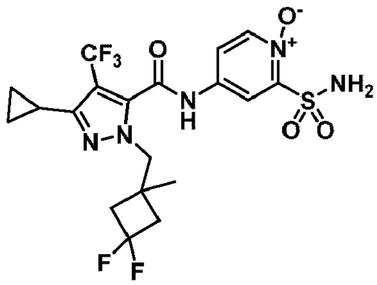 | 68 | 4-(3-cyclopropyl-1-((3,3-difluoro-1-methylcyclobutyl) methyl)-4-(trifluoromethyl)-1H-pyrazole-5-carboxamido)-2-sulfamoylpyridine 1-oxide |
| 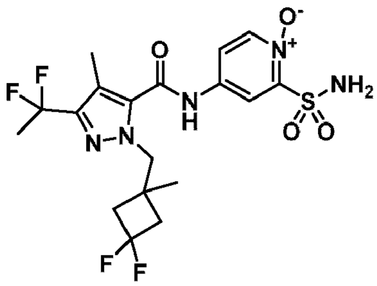 | 69 | 4-(1-((3,3-difluoro-1-methylcyclobutyl)methyl)-3-(1,1-difluoroethyl)-4-methyl-1H-pyrazole-5-carboxamido)-2-sulfamoylpyridine 1-oxide |
| 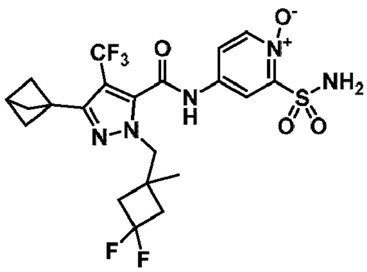 | 70 | 4-(3-(bicyclo[1.1.1]pentan-1-yl)-1-((3,3-difluoro-1-methylcyclobutyl)methyl)-4-(trifluoromethyl)-1H-pyrazole-5-carboxamido)-2-sulfamoylpyridine 1-oxide |
| 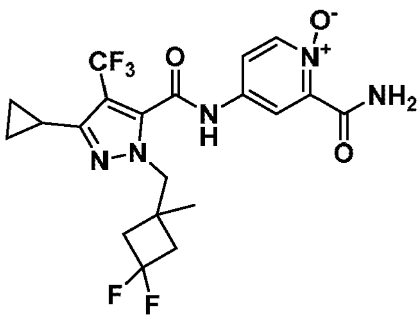 | 71 | 2-carbamoyl-4-(3-cyclopropyl-1-((3,3-difluoro-1-methylcyclobutyl)methyl)-4-(trifluoromethyl)-1H-pyrazole-5-carboxamido)pyridine 1-oxide |

(continued)

| Structure | Prophetic example number | Chemical name |
|---|---|---|
| 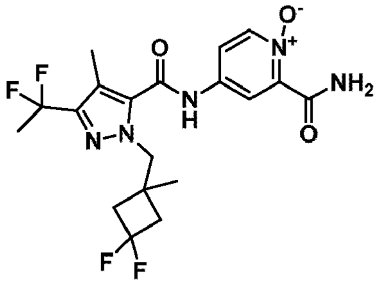 | 72 | 2-carbamoyl-4-(1-((3,3-difluoro-1-methylcyclobutyl) methyl)-3-(1,1-difluoroethyl)-4-methyl-1H-pyrazole-5-carboxamido)pyridine 1-oxide |
| 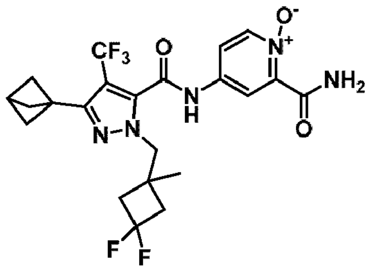 | 73 | 4-(3-(bicyclo[1.1.1]pentan-1-yl)-1-((3,3-difluoro-1-methylcyclobutyl)methyl)-4-(trifluoromethyl)-1H-pyrazole-5-carboxamido)-2-carbamoylpyridine 1-oxide |
| 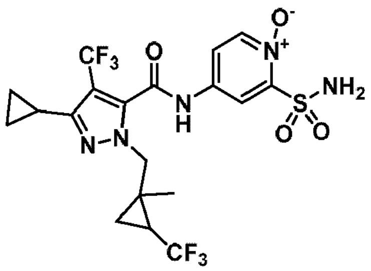 | 74 | 4-(3-cyclopropyl-1-((1-methyl-2-(trifluoromethyl) cyclopropyl)methyl)-4-(trifluoromethyl)-1H-pyrazole-5-carboxamido)-2-sulfamoylpyridine 1-oxide |
| 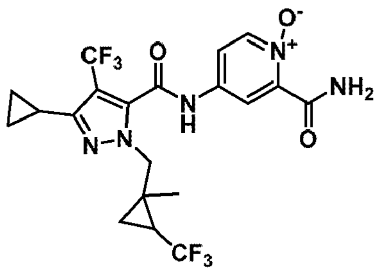 | 75 | 2-carbamoyl-4-(3-cyclopropyl-1-((1-methyl-2-(trifluoromethyl)cyclopropyl)methyl)-4-(trifluoromethyl)-1H-pyrazole-5-carboxamido) pyridine 1-oxide |
| 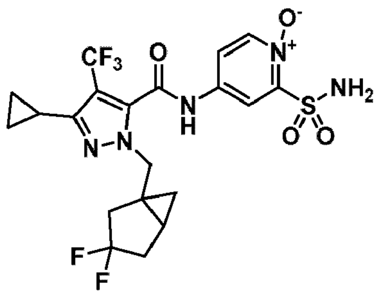 | 76 | 4-(3-cyclopropyl-1-((3,3-difluorobicyclo[3.1.0]hexan-1-yl)methyl)-4-(trifluoromethyl)-1H-pyrazole-5-carboxamido)-2-sulfamoylpyridine 1-oxide |

(continued)

| Structure | Prophetic example number | Chemical name |
|---|---|---|
| 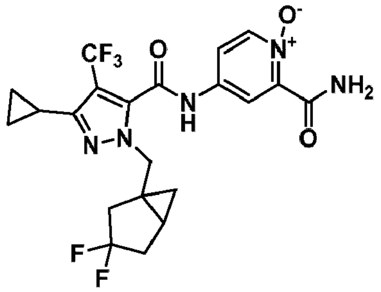 | 77 | 2-carbamoyl-4-(3-cyclopropyl-1-((3,3-difluorobicyclo [3.1.0]hexan-1-yl)methyl)-4-(trifluoromethyl)-1H-pyrazole-5-carboxamido)pyridine 1-oxide |
| 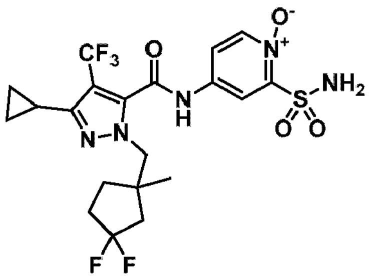 | 78 | 4-(3-cyclopropyl-1-((3,3-difluoro-1-methylcyclopentyl)methyl)-4-(trifluoromethyl)-1H-pyrazole-5-carboxamido)-2-sulfamoylpyridine 1-oxide |
| 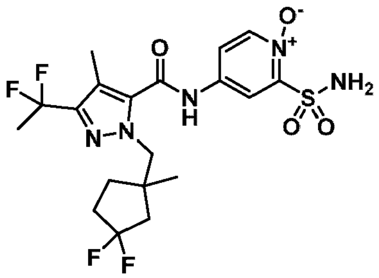 | 79 | 4-(1-((3,3-difluoro-1-methylcyclopentyl)methyl)-3-(1,1-difluoroethyl)-4-methyl-1H-pyrazole-5-carboxamido)-2-sulfamoylpyridine 1-oxide |
| 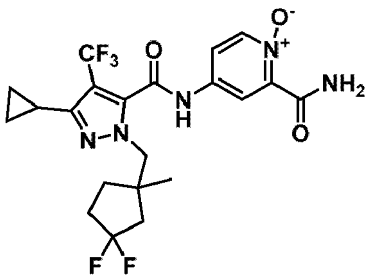 | 80 | 2-carbamoyl-4-(3-cyclopropyl-1-((3,3-difluoro-1-methylcyclopentyl)methyl)-4-(trifluoromethyl)-1H-pyrazole-5-carboxamido)pyridine 1-oxide |
| 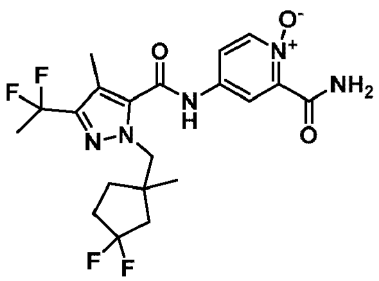 | 81 | 2-carbamoyl-4-(1-((3,3-difluoro-1-methylcyclopentyl) methyl)-3-(1,1-difluoroethyl)-4-methyl-1H-pyrazole-5-carboxamido)pyridine 1-oxide |

(continued)

| Structure | Prophetic example number | Chemical name |
|---|---|---|
| 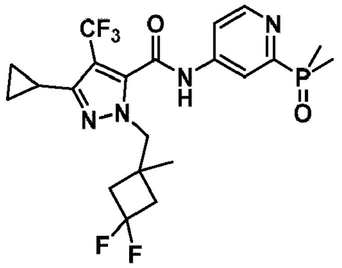 | 82 | 3-cyclopropyl-1-((3,3-difluoro-1-methylcyclobutyl) methyl)-N-(2-(dimethylphosphoryl)pyridin-4-yl)-4-(trifluoromethyl)-1H-pyrazole-5-carboxamide |
| 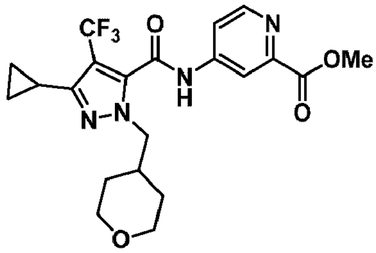 | 83 | methyl 4-(3-cyclopropyl-1-((tetrahydro-2H-pyran-4-yl)methyl)-4-(trifluoromethyl)-1H-pyrazole-5-carboxamido)picolinate |
| 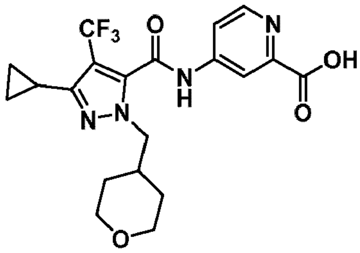 | 84 | 4-(3-cyclopropyl-1-((tetrahydro-2H-pyran-4-yl) methyl)-4-(trifluoromethyl)-1H-pyrazole-5-carboxamido)picolinic acid |

**Electrophysiology: Voltage-Clamp recordings**

The following $Na_V1.8$ recombinant cell line was used for recordings: HEK-$Na_V1.8$ (NM 006514.1) with b3 (NM_018400.3).

Sodium currents were measured using the patch clamp technique in the whole-cell configuration using the Qube384 (Sophion A/S, Copenhagen, Denmark) automated voltage clamp platform. "Multi hole" plates were used for the cell line expressing $Na_V1.8$ while "single hole" plates were used for the recombinant cell lines expressing the other subtypes. Appropriate filters (for minimum seal resistance and minimum current size) and series resistance compensation (for high quality sodium channel recordings) were applied. Data was collected at ambient room temperature.

The recording intracellular solution contained (in mM): NaCl 145 mM, KCl 4 mM, $CaCl_2$ 2 mM, MgCh 1 mM, HEPES 10 mM, Glucose 10 mM, pH 7.4 (NaOH). The extracellular recording solution contained (in mM): CsF 120 mM, CsCl 20 mM, NaCl 10 mM, EGTA 10 mM, HEPES 10 mM, pH 7.2 (CsOH). Currents were recorded at 25 kHz sampling frequency and filtered at 5 kHz. Series resistance compensation was applied at 65%. Vehicle (VEH) is the control condition where cells are exposed to 0.3 % DMSO without compound. All runs include VEH controls being exposed to the same voltage protocols to assess non-compound related phenomena such as run down and then used to isolate compound dependent effects on currents.

To check for *state-dependence inhibition*, the following voltage-sequence was applied every 20 seconds: From a resting membrane potential of-120 mV, the first test pulse (P1; 20 ms to -10 mV) was applied to check for channels in the Resting State followed by a brief recovery (20 ms to -120 mV), then holding the membrane voltage to $V_{1/2}$ (4 seconds at voltage to obtain half the channel at Rest and half Inactivated) with a subsequent second test pulse (P2; 20 ms to -10 mV) to check for channels in the Inactivated State, followed by another brief recovery (20 ms to -120 mV) and a final third test pulse (P3; 20 ms to -10 mV) to check for recovered channels.

To check for *frequency-dependent inhibition*, a 10 Hz protocol and a 20 Hz protocol were applied; namely,

From a resting membrane potential of -120 mV, 40-pulses (10 ms to -10 mV) was applied at 10 Hz (at 100 ms

between pulses) and then at 20 Hz (at 50 ms between pulses).

Each parameter was recorded (P1, P2, P3, P40 from 10Hz and P40 from 20Hz) during a control period (lasting ~ 5 minutes) when establishing the baseline and during compound period (lasting ~ 12 minutes) when test compound (or vehicle) was applied. For each parameter, the value at the end of the compound period was normalized to the vehicle baseline; as follows

$$Normalized\ Inhibition\ (Norm_{CPD}) = \frac{CPD\ Value_{end\ CPD\ period}}{VEH\ Value_{end\ Control\ Period}}$$

To adjust for any variance in the $Na^+$ current signal during the compound period (owing to cumulative inactivation independent of compound or shifts in biophysics over time), a dedicated segment of the recording wells in each 384 plate were dedicated to having only vehicle exposure. These vehicle-only recordings were used to correct for any apparent "run-up" or "run-down" in the experiment.

$$Normalized\ Inhibition\ (Norm_{VEH}) = \frac{VEH\ Value_{end\ CPD\ period}}{VEH\ Value_{end\ Control\ Period}}$$

The adjusted inhibition was calculated as follows:

$$\%\ Inhibition_{corrected} = 100 \times \frac{Norm_{CPD} - Norm_{VEH}}{100 - Norm_{VEH}}$$

Percent inhibition was determined and $IC_{50}$ values were calculated using a 4 parameter logistic model within XLFit Software (IDBS, Boston MA):

$$\%\ Inhibition_{corrected} = A + \frac{(B - A)}{(1 + \left(\frac{x}{C}\right)^{D}}$$

where A and B are the maximal and minimum inhibition respectively, C is the $IC_{50}$ concentration and D is the (Hill) slope.

The potency data of the example compounds are summarized in the table below (category **A**: human NaV1.8 $IC_{50} \leq 0.1$ µM; category **B**: 0.1 µM < human NaV1.8 $IC_{50}$ < 1 µM; category **C**: 1 µM < human NaV1.8 $IC_{50}$ <_ 10 µM; "n.d.": not determined):

| Example number | Potency category | Example number | Potency category |
|---|---|---|---|
| **1a** | B | **5b** | A |
| **1b** | B | 7 | C |
| **3** | B | **8** | B |
| **4** | A | **9** | B |
| **5a** | A | **10** | B |

## Claims

**1.** A compound according to general formula (I)

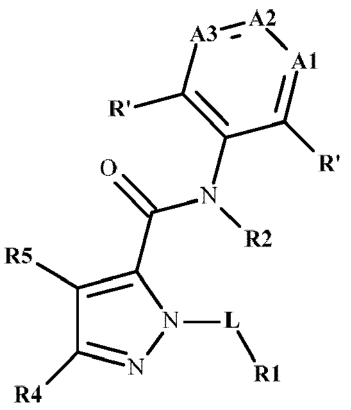

(I)

wherein

(a) one of **A1**, **A2** and **A3** represents C-**R3**; another one of **A1**, **A2** and **A3** represents C-**R'**; and the remaining one of **A1**, **A2** and **A3** represents $N^+$-$O^-$; or
(b) one of **A1**, **A2** and **A3** represents C-**R3'**; another one of **A1**, **A2** and **A3** represents C-**R'**; and the remaining one of **A1**, **A2** and **A3** represents N or C**R'**;

**R'** represents H, F, $CH_3$, $CH_2F$, $CHF_2$, $CF_3$, or $OCH_3$;
**L** represents $CH_2$, $CH(CH_3)$ or $CH(CH_2CH_3)$;
**R1** represents $C_{3-10}$-cycloalkyl, 5 to 11-membered spiroalkyl or dispiroalkyl, 4 to 11-membered bicycloalkyl, 4 to 10-membered heterocycloalkyl, or 5 to 11-membered heterobicycloalkyl;
**R2** represents H or $C_{1-6}$-alkyl;
**R3** represents C(O)-$NH_2$, C(O)-N(H)($C_{1-6}$-alkyl), C(O)-N(H)($C_{3-6}$-cycloalkyl), C(O)-N($C_{1-6}$-alkyl)$_2$, C(O)-N($C_{1-6}$-alkyl)($C_{3-6}$-cycloalkyl), $S(O)_2$-$C_{1-6}$-alkyl, $S(O)_2$-($C_{3-6}$-cycloalkyl), $S(O)_2$-(4 to 6-membered heterocycloalkyl), $S(O)_2$-phenyl, $S(O)_2$-(5 or 6-membered heteroaryl), S(O)-$NH_2$, S(O)-N(H)($C_{1-6}$-alkyl), S(O)-N($C_{1-6}$-alkyl)$_2$, $S(O)_2$-$NH_2$, $S(O)_2$-N(H)($C_{1-6}$-alkyl), $S(O)_2$-N(H)($C_{3-6}$-cycloalkyl), $S(O)_2$-N($C_{1-6}$-alkyl)$_2$, S(O)-$C_{1-6}$-alkyl, S(O)-($C_{3-6}$-cycloalkyl), S(O)-(4 to 6-membered heterocycloalkyl), S(O)-phenyl, S(O)-(5 or 6-membered heteroaryl), $OCF_3$, $OCF_2H$, CN, OH, O-$C_{3-6}$-cycloalkyl, O-(4 to 6-membered heterocycloalkyl), or S(O)(N**R3a**)**R3b**;
**R3'** represents C(O)OH, C(O)O$C_{1-6}$-alkyl, or P(O)($C_{1-6}$-alkyl)$_2$;
wherein

**R3a** represents H, $C_{1-6}$-alkyl, C(O)$C_{1-5}$-alkyl, C(O)$C_{1-5}$-alkyl-$NH_2$, C(O)$C_{1-5}$-alkyl-NH-$CH_3$, C(O)$C_{1-5}$-alkyl-N($CH_3$)$_2$, $C_{3-10}$-cycloalkyl, $C_{1-6}$-alkylene-$C_{3-10}$-cycloalkyl, 4 to 10-membered heterocycloalkyl, or $C_{1-6}$-alkylene-(4 to 10-membered heterocycloalkyl);
**R3b** represents $NH_2$, $C_{1-6}$-alkyl, $C_{3-10}$-cycloalkyl, $C_{1-6}$-alkylene-$C_{3-10}$-cycloalkyl, 4 to 10-membered heterocycloalkyl, or $C_{1-6}$-alkylene-(4 to 10-membered heterocycloalkyl);
or **R3a** and **R3b** mean $(CH_2)_{3-5}$ and together with the atoms to which they are attached form a ring;

**R4** represents H, F, Cl, Br, CN, $CHF_2$, $CH_2F$, $CF_3$, $C_{1-6}$-alkyl, $C_{3-10}$-cycloalkyl, $C_{1-6}$-alkylene-$C_{3-10}$-cycloalkyl, 4 to 11-membered bicycloalkyl, 5 to 11-membered spiroalkyl or bisspiroalkyl, 4 to 10-membered heterocycloalkyl, $C_{1-6}$-alkylene-(4 to 10-membered heterocycloalkyl), $NH_2$, N(H)($C_{1-6}$-alkyl), N($C_{1-6}$-alkyl)$_2$, O-$C_{1-6}$-alkyl, O-$C_{3-10}$-cycloalkyl, O-$C_{1-6}$-alkylene-$C_{3-10}$-cycloalkyl, O-(4 to 6-membered heterocycloalkyl), or O-$C_{1-6}$-alkylene-(4 to 6-membered heterocycloalkyl);
**R5** represents H, F, Cl, Br, CN, $CHF_2$, $CH_2F$, $CF_3$, $C_{1-6}$-alkyl, $C_{3-10}$-cycloalkyl, $C_{1-6}$-alkylene-$C_{3-10}$-cycloalkyl, 4 to 10-membered heterocycloalkyl, $C_{1-6}$-alkylene-(4 to 10-membered heterocycloalkyl), $NH_2$, N(H)($C_{1-6}$-alkyl), N($C_{1-6}$-alkyl)$_2$, O-$C_{1-6}$-alkyl, O-$C_{3-10}$-cycloalkyl, O-$C_{1-6}$-alkylene-$C_{3-10}$-cycloalkyl, O-(4 to 6-membered heterocycloalkyl), or O-$C_{1-6}$-alkylene-(4 to 6-membered heterocycloalkyl);
wherein $C_{1-6}$-alkyl and $C_{1-6}$-alkylene in each case independently from one another is linear or branched;
wherein $C_{1-6}$-alkyl, $C_{1-6}$-alkylene, $C_{3-10}$-cycloalkyl, $C_{3-6}$-cycloalkyl, 4 to 11-membered bicycloalkyl, 5 to 11-membered spiroalkyl or dispiroalkyl, 4 to 10-membered heterocycloalkyl, 4 to 6-membered heterocycloalkyl,

and 5 to 11-membered heterobicycloalkyl in each case independently from one another are unsubstituted or substituted with one, two, three, four or more substituents independently from one another selected from the group consisting of F, Cl, CN, $C_{1-6}$-alkyl, $C_{1-6}$-alkylene-OH, $C_{1-6}$-alkylene-$OCH_3$, $CF_3$, $CF_2H$, $CFH_2$, C(O)-$C_{1-6}$-alkyl, OH, =O, $OCF_3$, $OCF_2H$, $OCFH_2$, O-$C_{1-6}$-alkyl, $C_{1-4}$-alkylene-O-$C_{1-4}$-alkylene-O-$CH_3$, $C_{0-4}$-alkylene-O-($C_{1-4}$-alkylene-O)$_{1-4}$-$CH_3$, $NH_2$, NH-$C_{1-6}$-alkyl, or N($C_{1-6}$-alkyl)$_2$;
wherein phenyl, 5 to 10-membered heteroaryl and 5 or 6-membered heteroaryl in each case independently from one another are unsubstituted or mono- or polysubstituted with one or more substituents selected from the group consisting of F, Cl, Br, CN, $C_{1-6}$-alkyl, $C_{3-6}$-cycloalkyl, $CF_3$, $CF_2H$, $CFH_2$, $C_{1-6}$-alkylene-$CF_3$, $C_{1-6}$-alkylene-$CF_2H$, $C_{1-6}$-alkylene-$CFH_2$, $C_{1-6}$-alkylene-OH, $C_{1-6}$-alkylene-$OCH_3$, C(O)-$C_{1-6}$-alkyl, $OCF_3$, $OCF_2H$, $OCFH_2$, and O-$C_{1-6}$-alkyl;
in the form of the free compound or a physiologically acceptable salt thereof.

**2.** The compound according to claim 1, wherein

(i) **A1** represents C**R3**; **A2** represents $N^+$-$O^-$; and **A3** represents C**R'**, preferably CH;
(ii) **A1** represents C**R3'**; **A2** represents N or CH; and **A3** represents C**R'**, preferably CH; or
(iii) **A1** represents N or CH; **A2** represents C**R3'**; and **A3** represents C**R'**, preferably CH.

**3.** The compound according to claim 1 or 2, wherein **L** represents $CH_2$.

**4.** The compound according to any of the preceding claims, wherein **R1** represents

- $C_{3-10}$-cycloalkyl, unsubstituted or substituted with one, two, three, four, or more substituents independently from one another selected from F, $CH_3$, $OCH_3$, CN, $CHF_2$ and $CF_3$; preferably selected from cyclopropyl, methyl cyclopropyl, dimethyl cyclopropyl, fluoro cyclopropyl, difluoro cyclopropyl, trifluoromethyl cyclopropyl, trifluoromethyl methyl cyclopropyl, methyl difluoro cyclopropyl, trifluoromethyl difluoro cyclopropyl, dimethyl difluoro cyclopropyl, cyclobutyl, methyl cyclobutyl, dimethyl cyclobutyl, fluoro cyclobutyl, difluoro cyclobutyl, trifluoromethyl cyclobutyl, trifluoromethyl methyl cyclobutyl, methyl difluoro cyclobutyl, trifluoromethyl difluoro cyclobutyl, dimethyl difluoro cyclobutyl, cyclopentyl, methyl cyclopentyl, dimethyl cyclopentyl, fluoro cyclopentyl, difluoro cyclopentyl, trifluoromethyl cyclopentyl, trifluoromethyl methyl cyclopentyl, methyl difluoro cyclopentyl, trifluoromethyl difluoro cyclopentyl, dimethyl difluoro cyclopentyl, cyclohexyl, methyl cyclohexyl, dimethyl cyclohexyl, fluoro cyclohexyl, difluoro cyclohexyl, trifluoromethyl cyclohexyl, trifluoromethyl methyl cyclohexyl, methyl difluoro cyclohexyl, trifluoromethyl difluoro cyclohexyl, dimethyl difluoro cyclohexyl, cycloheptyl, methyl cycloheptyl, dimethyl cycloheptyl, fluoro cycloheptyl, difluoro cycloheptyl, trifluoromethyl cycloheptyl, trifluoromethyl methyl cycloheptyl, methyl difluoro cycloheptyl, trifluoromethyl difluoro cycloheptyl, dimethyl difluoro cycloheptyl, cyclooctyl, methyl cyclooctyl, dimethyl cyclooctyl, fluoro cyclooctyl, difluoro cyclooctyl, trifluoromethyl cyclooctyl, trifluoromethyl methyl cyclooctyl, methyl difluoro cyclooctyl, trifluoromethyl difluoro cyclooctyl, dimethyl difluoro cyclooctyl, cyclononyl, methyl cyclononyl, dimethyl cyclononyl, fluoro cyclononyl, difluoro cyclononyl, trifluoromethyl cyclononyl, trifluoromethyl methyl cyclononyl, methyl difluoro cyclononyl, trifluoromethyl difluoro cyclononyl, dimethyl difluoro cyclononyl, cyclodecyl, methyl cyclodecyl, dimethyl cyclodecyl, fluoro cyclodecyl, difluoro cyclodecyl, trifluoromethyl cyclodecyl, trifluoromethyl methyl cyclodecyl, methyl difluoro cyclodecyl, trifluoromethyl difluoro cyclodecyl, and dimethyl difluoro cyclodecyl;
more preferably selected from dimethyl cyclopropyl, trifluoromethyl methyl cyclopropyl, trifluoromethyl cyclobutyl, difluoro cyclobutyl, methyl difluoro cyclobutyl, trifluoromethyl cyclopentyl, difluoro cyclopentyl, methyl difluoro cyclopentyl, and trifluoromethyl difluoro cyclopentyl; or
- 4 to 11-membered bicycloalkyl, unsubstituted or substituted with one, two, three, four, or more substituents independently from one another selected from F, $CH_3$, $OCH_3$, CN, $CHF_2$ and $CF_3$;
preferably selected from bicylo[1.1.1]pentyl, methyl bicylo[1.1.1]pentyl, methoxy bicylo[1.1.1]pentyl, difluoromethyl bicylo[1.1.1]pentyl, trifluoromethyl bicylo[1.1.1]pentyl, fluoro bicylo[1.1.1]pentyl, difluoro bicylo[1.1.1]pentyl, methyl difluoro bicylo[1.1.1]pentyl, trifluoromethyl difluoro bicylo[1.1.1]pentyl, bicyclo[2.1.0]pentyl, methyl bicyclo[2.1.0]pentyl, trifluoromethyl bicyclo[2.1.0]pentyl, fluoro bicyclo[2.1.0]pentyl, difluoro bicyclo [2.1.0]pentyl, methyl difluoro bicyclo[2.1.0]pentyl, trifluoromethyl difluoro bicyclo[2.1.0]pentyl, bicyclo[3.1.0]hexyl, methyl bicyclo[3.1.0]hexyl, trifluoromethyl bicyclo[3.1.0]hexyl, fluoro bicyclo[3.1.0]hexyl, difluoro bicyclo[3.1.0]hexyl, methyl difluoro bicyclo[3.1.0]hexyl, trifluoromethyl difluoro bicyclo[3.1.0]hexyl, bicyclo[2.2.0]hexyl, methyl bicyclo[2.2.0]hexyl, trifluoromethyl bicyclo[2.2.0]hexyl, fluoro bicyclo[2.2.0]hexyl, difluoro bicyclo[2.2.0]hexyl, methyl difluoro bicyclo[2.2.0]hexyl, trifluoromethyl difluoro bicyclo[2.2.0]hexyl, bicyclo[2.1.1]hexyl, methyl bicyclo[2.1.1]hexyl, trifluoromethyl bicyclo[2.1.1]hexyl, fluoro bicyclo[2.1.1]hexyl, difluoro bicyclo[2.1.1]hexyl, fluoro bicyclo[2.1.1]hexyl, difluoro bicyclo[2.1.1]hexyl, methyl difluoro bicyclo[2.1.1]hexyl, trifluoromethyl difluoro bicyclo[2.1.1]hexyl, bicyclo[4.1.0]heptyl, methyl bicyclo[4.1.0]heptyl, trifluoromethyl bicyclo[4.1.0]heptyl, fluoro bicyclo[4.1.0]heptyl, difluoro bicyclo[4.1.0]heptyl, methyl difluoro bicyclo[4.1.0]heptyl, trifluoromethyl difluoro bicyclo[4.1.0]heptyl, bicyclo[2.2.1]heptyl, methyl bicyclo[2.2.1]heptyl, trifluoromethyl bicyclo[2.2.1]heptyl, fluoro bicyclo[2.2.1]heptyl, difluoro bicyclo[2.2.1]heptyl, methyl difluoro bicyclo[2.2.1]heptyl, trifluoromethyl difluoro bicyclo[2.2.1]heptyl, bicyclo[3.3.0]octyl, methyl bicyclo[3.3.0]octyl, trifluoromethyl bicyclo[3.3.0]octyl, fluoro bicyclo[3.3.0]octyl, difluoro bicyclo[3.3.0]octyl, methyl difluoro bicyclo[3.3.0]octyl, and trifluoromethyl difluoro bicyclo[3.3.0]octyl;

more preferably selected from bicyclo[2.1.0]pentyl, difluoro bicyclo[2.1.0]pentyl, methyl bicyclo[2.2.0]hexyl, difluoro bicyclo[3.1.0]hexyl, methyl difluoro bicyclo[3.1.0]hexyl, trifluoromethyl difluoro bicyclo[3.1.0]hexyl, fluoro bicyclo[2.1.1]hexyl, difluoro bicyclo[2.1.1]hexyl, difluoro bicyclo[4.1.0]heptyl, fluoro bicyclo[2.2.1]heptyl, and fluoro bicyclo[3.3.0]octyl;

- 5 to 11-membered spiroalkyl or dispiroalkyl, unsubstituted or substituted with one, two, three, four, or more substituents independently from one another selected from F, $CH_3$, $OCH_3$, CN, $CHF_2$ and $CF_3$;

preferably selected from spiro[2.2]pentyl, methyl spiro[2.2]pentyl, fluoro spiro[2.2]pentyl, difluoro spiro[2.2]pentyl, methyl difluoro spiro[2.2]pentyl, spiro[2.3]hexyl, methyl spiro[2.3]hexyl, fluoro spiro[2.3]hexyl, difluoro spiro[2.3]hexyl, methyl difluoro spiro[2.3]hexyl, spiro[3.3]heptyl, methyl spiro[3.3]heptyl, fluoro spiro[3.3]heptyl, difluoro spiro[3.3]heptyl, methyl difluoro spiro[3.3]heptyl, and dispiro[2.02.1]heptyl;

more preferably selected from spiro[2.2]pentyl, difluoro spiro[2.2]pentyl, spiro[2.3]hexyl, methyl spiro[2.3]hexyl, fluoro spiro[2.3]hexyl, methyl difluoro spiro[2.3]hexyl, spiro[3.3]heptyl, and dispiro[2.0.2.1]heptyl;

- 4 to 10-membered heterocycloalkyl, unsubstituted or substituted with one, two, three, four, or more substituents independently from one another selected from F, $CH_3$, $OCH_3$, CN, $CHF_2$ and $CF_3$;

preferably selected from oxetanyl, tetrahydrofuranyl, tetrahydropyranyl, morpholinyl, thiomorpholinyl, tetrahydrothiopyranyl, tetrahydrothiopyranyl 1,1-dioxide, oxepanyl, piperidinyl, piperidinonyl, azetidinyl, pyrrolidinyl, pyrrolidinonyl, 4-methylpiperazinyl, morpholinonyl, dioxanyl, piperazinyl, tetrahydropyrrolyl, azepanyl, dioxepanyl, oxazepanyl, diazepanyl, thiazolidinyl, tetrahydrothiophenyl, tetrahydropyridinyl, dithiolanyl, dihydropyrrolyl, dioxolanyl, dihydropyridinyl, dihydrofuranyl, dihydroisoxazolyl, dihydrooxazolyl, imidazolidinyl, isoxazolidinyl, oxazolidinyl, piperazinyl, N-methylpyridinonyl, pyrazolidinyl, pyranyl; dihydroquinolinyl, dihydroisoquinolinyl, dihydroindolinyl, dihydroisoindolyl, tetrahydroquinolinyl, tetrahydroisoquinolinyl and tetrahydroindolinyl;

more preferably tetrahydropyranyl; or

- 5 to 11-membered heterobicycloalkyl, unsubstituted or substituted with one, two, three, four, or more substituents independently from one another selected from F, $CH_3$, $OCH_3$, CN, $CHF_2$ and $CF_3$; preferably 2-oxa-bicyclo[2.1.1]hexyl or methyl 2-oxa-bicyclo[2.1.1]hexyl.

**5.** The compound according to any of the preceding claims, wherein **R2** represents H.

**6.** The compound according to any of the preceding claims, wherein **R3** represents C(O)-$NH_2$, $S(O)_2$-$C_{1\text{-}6}$-alkyl, $S(O)_2$-$NH_2$, or S(O)(N**R3a**)**R3b**; preferably C(O)-$NH_2$, S(O)(NH)$CH_3$, $S(O)_2$-$NH_2$,.

**7.** The compound according to any of the preceding claims, wherein **R3'** represents C(O)OH, C(O)O$C_{1\text{-}6}$-alkyl, or P(O)($C_{1\text{-}6}$-alkyl)$_2$; preferably C(O)OH, C(O)O$CH_3$, or P(O)$(CH_3)_2$.

**8.** The compound according to any of the preceding claims, wherein **R4** represents

- H;

- $C_{1\text{-}6}$-alkyl;

preferably $CHF_2$, $CH_2F$, $CF_3$, $CH_2CHF_2$, $CH_2CH_2F$, $CH_2CF_3$, $CF_2CH_3$, $CHFCH_3$, $CF_2CF_3$, $CHFCF_3$, $CH(CHF_2)(CH_3)$, $CH(CH_2F)(CH_3)$, $CH(CF_3)(CH_3)$, $CH_3$, $CH_2CH_3$, $CH(CH_3)_2$;

more preferably $CF_2CH_3$;

- $C_{3\text{-}10}$-cycloalkyl, unsubstituted or substituted with one, two, three, four, or more substituents independently from one another selected from F, $CH_3$, $OCH_3$, CN, $CHF_2$ and $CF_3$;

preferably selected from cyclopropyl, methyl cyclopropyl, difluoromethyl cyclopropyl, trifluoromethyl cyclopropyl, cyano cyclopropyl, methoxy cyclopropyl, fluoro cyclopropyl, difluoro cyclopropyl, trifluoro cyclopropyl, cyclobutyl, methyl cyclobutyl, difluoromethyl cyclobutyl, trifluoromethyl cyclobutyl, cyano cyclobutyl, methoxy cyclobutyl, fluoro cyclobutyl, difluoro cyclobutyl, trifluoro cyclobutyl, cyclopentyl, methyl cyclopentyl, difluoromethyl cyclopentyl, trifluoromethyl cyclopentyl, cyano cyclopentyl, methoxy cyclopentyl, fluoro cyclopentyl, difluoro cyclopentyl, trifluoro cyclopentyl, cyclohexyl, methyl cyclohexyl, difluoromethyl cyclohexyl, trifluoromethyl cyclohexyl, cyano cyclohexyl, methoxy cyclohexyl, fluoro cyclohexyl, difluoro cyclohexyl, and trifluoro cyclohexyl;

more preferably cyclopropyl, methyl cyclopropyl, cyano cyclopropyl, methoxy cyclopropyl, difluoromethyl cyclopropyl, trifluoromethyl cyclopropyl, fluoro cyclopropyl, difluoro cclopropyl, trifluoro cyclopropyl, cyclobutyl, difluoro cyclobutyl, cyclopentyl, difluorocyclopentyl;
- 4 to 11-membered bicycloalkyl, unsubstituted or substituted with one, two, three, four, or more substituents independently from one another selected from F, $CH_3$, $OCH_3$, CN, $CHF_2$ and $CF_3$;
preferably bicyclo[1.1.1]pentyl;
- 5 to 11-membered spiroalkyl, unsubstituted or substituted with one, two, three, four, or more substituents independently from one another selected from F, $CH_3$, $OCH_3$, CN, $CHF_2$ and $CF_3$;
preferably spiro[2.2]pentyl or spiro[2.3]hexyl; or
- O-$C_{1-6}$-alkyl, unsubstituted or substituted with one, two, three, four, or more F;
preferably selected from O-$CHF_2$, O-$CH_2F$, O-$CF_3$, O-$CH_2CHF_2$, O-$CH_2CH_2F$, O-$CH_2CF_3$, O-$CF_2CH_3$, O-$CHFCH_3$, O-$CF_2CF_3$, O-$CHFCF_3$, O-$CH_3$, O-$CH_2CH_3$, or O-$CH(CH_3)_2$.

**9.** The compound according to any of the preceding claims, wherein **R5** represents $C_{1-6}$-alkyl or Cl; preferably $CHF_2$, $CH_2F$, $CF_3$, $CH_2CHF_2$, $CH_2CH_2F$, $CH_2CF_3$, $CF_2CH_3$, $CHFCH_3$, $CF_2CF_3$, $CHFCF_3$, $CH(CHF_2)(CH_3)$, $CH(CH_2F)(CH_3)$, $CH(CF_3)(CH_3)$, $CH_3$, $CH_2CH_3$, $CH(CH_3)_2$ or Cl; more preferably $CF_3$, $CHF_2$, $CH_3$ or Cl.

**10.** The compound according to any of the preceding claims, which is selected from the group consisting of

• 2-carbamoyl-4-(1-((3,3-difluorocyclopentyl)methyl)-3-(1,1-difluoroethyl)-4-methyl-1H-pyrazole-5-carboxamido)pyridine 1-oxide;
• 2-carbamoyl-4-(1-((3,3-difluorocyclobutyl)methyl)-3-(1,1-difluoroethyl)-4-methyl-1H-pyrazole-5-carboxamido)pyridine 1-oxide;
• 2-carbamoyl-4-(1-((3,3-difluoro-1-methylcyclobutyl)methyl)-3-(1,1-difluoroethyl)-4-methyl-1H-pyrazole-5-carboxamido)pyridine 1-oxide;
• 2-carbamoyl-4-(1-((3,3-difluoro-1-methylcyclopentyl)methyl)-3-(1,1-difluoroethyl)-4-methyl-1H-pyrazole-5-carboxamido)pyridine 1-oxide;
• 4-(1-((3,3 -difluorocyclobutyl)methyl)-3-(1,1-difluoroethyl)-4-(trifluoromethyl)-1H-pyrazole-5-carboxamido)picolinic acid;
• 5-(1-((3,3 -difluorocyclobutyl)methyl)-3 -(1,1-difluoroethyl)-4-(trifluoromethyl)-1H-pyrazole-5-carboxamido)picolinic acid;
• 4-(3-cyclopropyl-1-((4,4-difluorocycloheayl)methyl)-4-(trifluoromethyl)-1H-pyrazole-5-carboxamido)picolinic acid;
• 1-(cyclohexylmethyl)-N-(2-(dimethylphosphoryl)pyridin-4-yl)-4-(trifluoromethyl)-1H-pyrazole-5-carboxamide;
• 4-(3-cyclopropyl-1-((3,3-difluoro-1-methylcyclobutyl)methyl)-4-(trifluoromethyl)-1H-pyrazole-5-carboxamido)-2-(S-methylsulfonimidoyl)pyridine 1-oxide;
• 4-(3-cyclopropyl-1-((3,3 -difluoro-1-methylcyclopentyl)methyl)-4-(trifluoromethyl)-1H-pyrazole-5-carboxamido)-2-(S-methylsulfonimidoyl)pyridine 1-oxide;
• 4-(1-((3,3-difluoro-1-methylcyclobutyl)methyl)-3-(1,1-difluoroethyl)-4-methyl-1H-pyrazole-5-carboxamido)-2-(S-methylsulfonimidoyl)pyridine 1-oxide;
• 4-(1-((3,3-difluoro-1-methylcyclopentyl)methyl)-3-(1,1-difluoroethyl)-4-methyl-1H-pyrazole-5-carboxamido)-2-(S-methylsulfonimidoyl)pyridine 1-oxide;
• 4-(3-cyclobutyl-1-((3,3-difluoro-1-methylcyclobutyl)methyl)-4-(trifluoromethyl)-1H-pyrazole-5-carboxamido)-2-(S-methylsulfonimidoyl)pyridine 1-oxide;
• 4-(1-((3,3-difluoro-1-methylcyclobutyl)methyl)-3-(3,3-difluorocyclobutyl)-4-(trifluoromethyl)-1H-pyrazole-5-carboxamido)-2-(S-methylsulfonimidoyl)pyridine 1-oxide;
• 4-(3-(bicyclo [1.1.1]pentan-1-yl)-1-((3,3 -difluoro-1-methylcyclobutyl)methyl)-4-(trifluoromethyl)-1H-pyrazole-5-carboxamido)-2-(S-methylsulfonimidoyl)pyridine 1-oxide;
• 4-(3-cyclopentyl-1-((3,3-difluoro-1-methylcyclobutyl)methyl)-4-(trifluoromethyl)-1H-pyrazole-5-carboxamido)-2-(S-methylsulfonimidoyl)pyridine 1-oxide;
• 4-(1-((3,3 -difluoro-1-methylcyclobutyl)methyl)-3-(3,3-difluorocyclopentyl)-4-(trifluoromethyl)-1H-pyrazole-5-carboxamido)-2-(S-methylsulfonimidoyl)pyridine 1-oxide;
• 4-(1-((3,3-difluoro-1-methylcyclobutyl)methyl)-3-(spiro[2.3]hexan-5-yl)-4-(trifluoromethyl)-1H-pyrazole-5-carboxamido)-2-(S-methylsulfonimidoyl)pyridine 1-oxide;
• 4-(1-((3,3 -difluoro-1-methylcyclobutyl)methyl)-3-(2-fluorocyclopropyl)-4-(trifluoromethyl)-1H-pyrazole-5-carboxamido)-2-(S-methylsulfonimidoyl)pyridine 1-oxide;
• 4-(1-((3,3 -difluoro-1-methylcyclobutyl)methyl)-3-(2,2-difluorocyclopropyl)-4-(trifluoromethyl)-1H-pyrazole-5-carboxamido)-2-(S-methylsulfonimidoyl)pyridine 1-oxide;
• 4-(1-((3,3 -difluoro-1-methylcyclobutyl)methyl)-3-(spiro[2.2]pentan-1-yl)-4-(trifluoromethyl)-1H-pyrazole-5-carboxamido)-2-(S-methylsulfonimidoyl)pyridine 1-oxide;

• 4-(1-((3,3 -difluoro-1-methylcyclobutyl)methyl)-3-(2-methylcyclopropyl)-4-(trifluoromethyl)-1H-pyrazole-5-carboxamido)-2-(S-methylsulfonimidoyl)pyridine 1-oxide;

• 4-(1-((3,3 -difluoro-1-methylcyclobutyl)methyl)-3-(2-(difluoromethyl)cyclopropyl)-4-(trifluoromethyl)-1H-pyrazole-5-carboxamido)-2-(S-methylsulfonimidoyl)pyridine 1-oxide;

• 4-(1-((3,3 -difluoro-1-methylcyclobutyl)methyl)-4-(trifluoromethyl)-3 -(2-(trifluoromethyl)cyclopropyl)-1H-pyrazole-5-carboxamido)-2-(S-methylsulfonimidoyl)pyridine 1-oxide;

• 4-(3-(1-cyanocyclopropyl)-1-((3,3-difluoro-1-methylcyclobutyl)methyl)-4-(trifluoromethyl)-1H-pyrazole-5-carboxamido)-2-(S-methylsulfonimidoyl)pyridine 1-oxide;

• 4-(1-((3,3-difluoro-1-methylcyclobutyl)methyl)-3-(1-methylcyclopropyl)-4-(trifluoromethyl)-1H-pyrazole-5-carboxamido)-2-(S-methylsulfonimidoyl)pyridine 1-oxide;

• 4-(1-((3,3 -difluoro-1-methylcyclobutyl)methyl)-3-(1-fluorocyclopropyl)-4-(trifluoromethyl)-1H-pyrazole-5-carboxamido)-2-(S-methylsulfonimidoyl)pyridine 1-oxide;

• 4-(1-((3,3-difluoro-1-methylcyclobutyl)methyl)-3-(1-methoxycyclopropyl)-4-(trifluoromethyl)-1H-pyrazole-5-carboxamido)-2-(S-methylsulfonimidoyl)pyridine 1-oxide;

• 4-(1-((3,3 -difluoro-1-methylcyclobutyl)methyl)-3-(2,3-difluorocyclopropyl)-4-(trifluoromethyl)-1H-pyrazole-5-carboxamido)-2-(S-methylsulfonimidoyl)pyridine 1-oxide;

• 4-(1-((3,3 -difluoro-1-methylcyclobutyl)methyl)-3 -(1,2,2-trifluorocyclopropyl)-4-(trifluoromethyl)-1H-pyrazole-5-carboxamido)-2-(S-methylsulfonimidoyl)pyridine 1-oxide;

• 4-(1-((3,3 -difluoro-1-methylcyclobutyl)methyl)-3 -(1,2-difluorocyclopropyl)-4-(trifluoromethyl)-1H-pyrazole-5-carboxamido)-2-(S-methylsulfonimidoyl)pyridine 1-oxide;

• 4-(1-((3,3-difluoro-1-methylcyclobutyl)methyl)-3-(1,1-difluoroethyl)-4-(trifluoromethyl)-1H-pyrazole-5-carboxamido)-2-(S-methylsulfonimidoyl)pyridine 1-oxide;

• 4-(4-chloro-1-((3,3 -difluoro-1-methylcyclobutyl)methyl)-3 -(1,1-difluoroethyl)- 1H-pyrazole-5-carboxamido)-2-(S-methylsulfonimidoyl)pyridine 1-oxide;

• 4-(3-cyclopropyl-1-((3,3-difluoro-1-methylcyclobutyl)methyl)-4-(difluoromethyl)-1H-pyrazole-5-carboxamido)-2-(S-methylsulfonimidoyl)pyridine 1-oxide;

• 4-(3-cyclopropyl-1-(spiro[2.2]pentan-1-ylmethyl)-4-(trifluoromethyl)-1H-pyrazole-5-carboxamido)-2-(S-methylsulfonimidoyl)pyridine 1-oxide;

• 4-(3-cyclopropyl-1-((2,2-difluorospiro[22]pentan-1-yl)methyl)-4-(trifluoromethyl)-1H-pyrazole-5-carboxamido)-2-(S-methylsulfonimidoyl)pyridine 1-oxide;

• 4-(3-cyclopropyl-1-(dispiro[2.024.13]heptan-7-ylmethyl)-4-(trifluoromethyl)-1H-pyrazole-5-carboxamido)-2-(S-methylsulfonimidoyl)pyridine 1-oxide;

• 4-(3-cyclopropyl-1-((1,2-dimethylcyclopropyl)methyl)-4-(trifluoromethyl)-1H-pyrazole-5-carboxamido)-2-(S-methylsulfonimidoyl)pyridine 1-oxide;

• 4-(3-cyclopropyl-1-((1-methyl-2-(trifluoromethyl)cyclopropyl)methyl)-4-(trifluoromethyl)-1H-pyrazole-5-carboxamido)-2-(S-methylsulfonimidoyl)pyridine 1-oxide;

• 4-(1-(bicyclo[2.1.0]pentan-1-ylmethyl)-3-cyclopropyl-4-(trifluoromethyl)-1H-pyrazole-5-carboxamido)-2-(S-methylsulfonimidoyl)pyridine 1-oxide;

• 4-(3-cyclopropyl-1-(spiro[2.3]hexan-5-ylmethyl)-4-(trifluoromethyl)-1H-pyrazole-5-carboxamido)-2-(S-methylsulfonimidoyl)pyridine 1-oxide;

• 4-(3-cyclopropyl-1-((5-methylspiro[2.3]hexan-5-yl)methyl)-4-(trifluoromethyl)-1H-pyrazole-5-carboxamido)-2-(S-methylsulfonimidoyl)pyridine 1-oxide;

• 4-(3-cyclopropyl-1-((1-methyl-3-(trifluoromethyl)cyclobutyl)methyl)-4-(trifluoromethyl)-1H-pyrazole-5-carboxamido)-2-(S-methylsulfonimidoyl)pyridine 1-oxide;

• 4-(3-cyclopropyl-1-((1-fluorospiro[2.3]hexan-5-yl)methyl)-4-(trifluoromethyl)-1H-pyrazole-5-carboxamido)-2-(S-methylsulfonimidoyl)pyridine 1-oxide;

• 4-(3-cyclopropyl-4-(trifluoromethyl)-1-((3-(trifluoromethyl)cyclobutyl)methyl)-1H-pyrazole-5-carboxamido)-2-(S-methylsulfonimidoyl)pyridine 1-oxide;

• 4-(3-cyclopropyl-1-((3,3-difluorocyclobutyl)methyl)-4-(trifluoromethyl)-1H-pyrazole-5-carboxamido)-2-(S-methylsulfonimidoyl)pyridine 1-oxide;

• 4-(3-cyclopropyl-1-((5,5-difluorobicyclo[2.1.0]pentan-2-yl)methyl)-4-(trifluoromethyl)-1H-pyrazole-5-carboxamido)-2-(S-methylsulfonimidoyl)pyridine 1-oxide;

• 4-(3-cyclopropyl-1-(spiro[2.3]hexan-4-ylmethyl)-4-(trifluoromethyl)-1H-pyrazole-5-carboxamido)-2-(S-methylsulfonimidoyl)pyridine 1-oxide;

• 4-(3-cyclopropyl-1-((6,6-difluoro-4-methylspiro[2.3]hexan-4-yl)methyl)-4-(trifluoromethyl)-1H-pyrazole-5-carboxamido)-2-(S-methylsulfonimidoyl)pyridine 1-oxide;

• 4-(3-cyclopropyl-1-(spiro[3.3]heptan-1-ylmethyl)-4-(trifluoromethyl)-1H-pyrazole-5-carboxamido)-2-(S-methylsulfonimidoyl)pyridine 1-oxide;

- 4-(3-cyclopropyl-1-((2-methylbicyclo[2.2.0]hexan-2-yl)methyl)-4-(trifluoromethyl)-1H-pyrazole-5-carboxamido)-2-(S-methylsulfonimidoyl)pyridine 1-oxide;
- 4-(3-cyclopropyl-1-((3,3-difluorobicyclo[3.1.0]hexan-1-yl)methyl)-4-(trifluoromethyl)-1H-pyrazole-5-carboxamido)-2-(S-methylsulfonimidoyl)pyridine 1-oxide;
- 4-(3-cyclopropyl-1-((4,4-difluorobicyclo[3.1.0]hexan-1-yl)methyl)-4-(trifluoromethyl)-1H-pyrazole-5-carboxamido)-2-(S-methylsulfonimidoyl)pyridine 1-oxide;
- 4-(3-cyclopropyl-1-((5-fluorooctahydropentalen-2-yl)methyl)-4-(trifluoromethyl)-1H-pyrazole-5-carboxamido)-2-(S-methylsulfonimidoyl)pyridine 1-oxide;
- 4-(3-cyclopropyl-4-(trifluoromethyl)-1-((1-(trifluoromethyl)cyclopentyl)methyl)-1H-pyrazole-5-carboxamido)-2-(S-methylsulfonimidoyl)pyridine 1-oxide;
- 4-(3-cyclopropyl-1-((4,4-difluoro-2-methylcyclopentyl)methyl)-4-(trifluoromethyl)-1H-pyrazole-5-carboxamido)-2-(S-methylsulfonimidoyl)pyridine 1-oxide;
- 4-(3-cyclopropyl-1-((4,4-difluoro-2-(trifluoromethyl)cyclopentyl)methyl)-4-(trifluoromethyl)-1H-pyrazole-5-carboxamido)-2-(S-methylsulfonimidoyl)pyridine 1-oxide;
- 4-(3-cyclopropyl-1-((3,3-difluoro-5-methylbicyclo[3.1.0]hexan-1-yl)methyl)-4-(trifluoromethyl)-1H-pyrazole-5-carboxamido)-2-(S-methylsulfonimidoyl)pyridine 1-oxide;
- 4-(3-cyclopropyl-1-((3,3-difluoro-5-(trifluoromethyl)bicyclo[3.1.0]hexan-1-yl)methyl)-4-(trifluoromethyl)-1H-pyrazole-5-carboxamido)-2-(S-methylsulfonimidoyl)pyridine 1-oxide;
- 4-(3-cyclopropyl-1-((2,2-difluorobicyclo[2.1.1]hexan-1-yl)methyl)-4-(trifluoromethyl)-1H-pyrazole-5-carboxamido)-2-(S-methylsulfonimidoyl)pyridine 1-oxide;
- 4-(3-cyclopropyl-1-((3,3-difluorobicyclo[2.1.1]hexan-1-yl)methyl)-4-(trifluoromethyl)-1H-pyrazole-5-carboxamido)-2-(S-methylsulfonimidoyl)pyridine 1-oxide;
- 4-(3-cyclopropyl-1-((4-fluorobicyclo[2.1.1]hexan-1-yl)methyl)-4-(trifluoromethyl)-1H-pyrazole-5-carboxamido)-2-(S-methylsulfonimidoyl)pyridine 1-oxide;
- 4-(3-cyclopropyl-1-((4-fluorobicyclo[22.1]heptan-1 yl)methyl)-4-(trifluoromethyl)-1H-pyrazole-5-carboxamido)-2-(S-methylsulfonimidoyl)pyridine 1-oxide;
- 4-(3-cyclopropyl-1-((4,4-difluorobicyclo[4.1.0]heptan-1-yl)methyl)-4-(trifluoromethyl)-1H-pyrazole-5-carboxamido)-2-(S-methylsulfonimidoyl)pyridine 1-oxide;
- 4-(3-cyclopropyl-1-((3,3-difluoro-1-methylcyclobutyl)methyl)-4-(trifluoromethyl)-1H-pyrazole-5-carboxamido)-2-sulfamoylpyridine 1-oxide;
- 4-(1-((3,3-difluoro-1-methylcyclobutyl)methyl)-3-(1,1-difluoroethyl)-4-methyl-1H-pyrazole-5-carboxamido)-2-sulfamoylpyridine 1-oxide;
- 4-(3-(bicyclo [1.1.1]pentan-1-yl)-1-((3,3-difluoro-1-methylcyclobutyl)methyl)-4-(trifluoromethyl)-1H-pyrazole-5-carboxamido)-2-sulfamoylpyridine 1-oxide;
- 2-carbamoyl-4-(3-cyclopropyl-1-((3,3-difluoro-1-methylcyclobutyl)methyl)-4-(trifluoromethyl)-1H-pyrazole-5-carboxamido)pyridine 1-oxide;
- 2-carbamoyl-4-(1-((3,3-difluoro-1-methylcyclobutyl)methyl)-3-(1,1-difluoroethyl)-4-methyl-1H-pyrazole-5-carboxamido)pyridine 1-oxide;
- 4-(3-(bicyclo [1.1.1]pentan-1-yl)-1-((3,3-difluoro-1-methylcyclobutyl)methyl)-4-(trifluoromethyl)-1H-pyrazole-5-carboxamido)-2-carbamoylpyridine 1-oxide;
- 4-(3-cyclopropyl-1-((1-methyl-2-(trifluoromethyl)cyclopropyl)methyl)-4-(trifluoromethyl)-1H-pyrazole-5-carboxamido)-2-sulfamoylpyridine 1-oxide;
- 2-carbamoyl-4-(3-cyclopropyl-1-((1-methyl-2-(trifluoromethyl)cyclopropyl)methyl)-4-(trifluoromethyl)-1H-pyrazole-5-carboxamido)pyridine 1-oxide;
- 4-(3-cyclopropyl-1-((3,3-difluorobicyclo[3.1.0]hexan-1-yl)methyl)-4-(trifluoromethyl)-1H-pyrazole-5-carboxamido)-2-sulfamoylpyridine 1-oxide;
- 2-carbamoyl-4-(3-cyclopropyl-1-((3,3-difluorobicyclo[3.1.0]hexan-1-yl)methyl)-4-(trifluoromethyl)-1H-pyrazole-5-carboxamido)pyridine 1-oxide;
- 4-(3-cyclopropyl-1-((3,3-difluoro-1-methylcyclopentyl)methyl)-4-(trifluoromethyl)-1H-pyrazole-5-carboxamido)-2-sulfamoylpyridine 1-oxide;
- 4-(1-((3,3-difluoro-1-methylcyclopentyl)methyl)-3-(1,1-difluoroethyl)-4-methyl-1H-pyrazole-5-carboxamido)-2-sulfamoylpyridine 1-oxide;
- 2-carbamoyl-4-(3-cyclopropyl-1-((3,3-difluoro-1-methylcyclopentyl)methyl)-4-(trifluoromethyl)-1H-pyrazole-5-carboxamido)pyridine 1-oxide;
- 2-carbamoyl-4-(1-((3,3-difluoro-1-methylcyclopentyl)methyl)-3-(1,1-difluoroethyl)-4-methyl-1H-pyrazole-5-carboxamido)pyridine 1-oxide;
- 3 -cyclopropyl-1-((3,3-difluoro-1-methylcyclobutyl)methyl)-N-(2-(dimethylphosphoryl)pyridin-4-yl)-4-(trifluoromethyl)-1H-pyrazole-5-carboxamide;

- methyl 4-(3-cyclopropyl-1-((tetrahydro-2H-pyran-4-yl)methyl)-4-(trifluoromethyl)-1H-pyrazole-5-carboxamido)picolinate; and
- 4-(3-cyclopropyl-1-((tetrahydro-2H-pyran-4-yl)methyl)-4-(trifluoromethyl)-1H-pyrazole-5-carboxamido)picolinic acid

in the form of the free compound or a physiologically acceptable salt thereof.

**11.** A pharmaceutical dosage form comprising a compound according to any of claims 1 to 10.

**12.** The pharmaceutical dosage form according to claim 11 for use in the treatment of pain.

Europäisches Patentamt
European Patent Office
Office européen des brevets

# EUROPEAN SEARCH REPORT

Application Number
EP 22 21 3353

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2009/049180 A2 (VERTEX PHARMA [US]; JOSHI PRAMOD [US] ET AL.) 16 April 2009 (2009-04-16) * paragraph [0016] * ----- | 1-12 | INV. C07D401/12 C07D405/14 A61P29/00 A61K31/4155 |
| A,D | WO 2020/092187 A1 (MERCK SHARP & DOHME [US]; BRESLIN MICHAEL J [US] ET AL.) 7 May 2020 (2020-05-07) * claims 1, 18 * ----- | 1-12 | |
| A,D | WO 2020/092667 A1 (MERCK SHARP & DOHME [US]; ARASAPPAN ASHOK [US] ET AL.) 7 May 2020 (2020-05-07) * claims 1, 21 * ----- | 1-12 | |
| E | WO 2022/263498 A1 (GRUENENTHAL GMBH [DE]) 22 December 2022 (2022-12-22) * see structures on pages 149, 152, 162 * ----- | 1-12 | |
| | | | TECHNICAL FIELDS SEARCHED (IPC) |
| | | | C07D A61P |

The present search report has been drawn up for all claims

1

| Place of search | Date of completion of the search | Examiner |
|---|---|---|
| Munich | 24 April 2023 | Bakboord, Joan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 21 3353

This annex lists the patent family members relating to the patent documents cited in the above-mentioned European search report. The members are as contained in the European Patent Office EDP file on The European Patent Office is in no way liable for these particulars which are merely given for the purpose of information.

24-04-2023

| Patent document cited in search report | Publication date | Patent family member(s) | Publication date |
|---|---|---|---|
| WO 2009049180 A2 | 16-04-2009 | AU 2008310660 A1 | 16-04-2009 |
| | | CA 2701946 A1 | 16-04-2009 |
| | | CN 101883760 A | 10-11-2010 |
| | | EP 2227453 A2 | 15-09-2010 |
| | | JP 5555169 B2 | 23-07-2014 |
| | | JP 2011500598 A | 06-01-2011 |
| | | NZ 584475 A | 27-07-2012 |
| | | US 2009099233 A1 | 16-04-2009 |
| | | WO 2009049180 A2 | 16-04-2009 |
| WO 2020092187 A1 | 07-05-2020 | EP 3873468 A1 | 08-09-2021 |
| | | US 2022119363 A1 | 21-04-2022 |
| | | WO 2020092187 A1 | 07-05-2020 |
| WO 2020092667 A1 | 07-05-2020 | AR 116939 A1 | 30-06-2021 |
| | | AU 2019372057 A1 | 27-05-2021 |
| | | AU 2022287562 A1 | 02-02-2023 |
| | | BR 112021008524 A2 | 03-08-2021 |
| | | CA 3117927 A1 | 07-05-2020 |
| | | CL 2021001078 A1 | 29-10-2021 |
| | | CN 113272293 A | 17-08-2021 |
| | | CO 2021005553 A2 | 30-07-2021 |
| | | CR 20210209 A | 20-05-2021 |
| | | DO P2021000082 A | 22-07-2021 |
| | | EA 202191177 A1 | 28-07-2021 |
| | | EC SP21030066 A | 31-05-2021 |
| | | EP 3873893 A1 | 08-09-2021 |
| | | IL 282468 A | 31-05-2021 |
| | | JP 2022506146 A | 17-01-2022 |
| | | KR 20210086687 A | 08-07-2021 |
| | | MA 54076 A | 09-02-2022 |
| | | NI 202100029 A | 13-08-2021 |
| | | PE 20211693 A1 | 01-09-2021 |
| | | SG 11202104326T A | 28-05-2021 |
| | | TW 202031643 A | 01-09-2020 |
| | | US 2020140411 A1 | 07-05-2020 |
| | | US 2021387966 A1 | 16-12-2021 |
| | | US 2022289710 A1 | 15-09-2022 |
| | | WO 2020092667 A1 | 07-05-2020 |
| WO 2022263498 A1 | 22-12-2022 | US 2023025025 A1 | 26-01-2023 |
| | | WO 2022263498 A1 | 22-12-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020092187 A
- WO 2020092667 A
- WO 2009049180 A
- WO 2009049183 A
- WO 2009049181 A
- WO 2021113627 A


### Non-patent literature cited in the description


- **LUMPKIN ; CATERINA.** *Nature,* 2007, vol. 445, 858-865
- **CRAWFORD ; CATERINA.** *Toxicologic Pathology,* 2020, vol. 48 (1), 174
- **GOODWIN G ; MCMAHON S.B.** *Nature Reviews Neuroscience,* 2021, vol. 22, 263-274
- **BENNETT D.L. et al.** *Physiol Rev,* 2019, vol. 99, 1079-1151
- **AKOPIAN A.N. et al.** *Nature,* 1996, vol. 379, 257-261
- **SANGAMESWARAN L. et al.** *J. Biol. Chem.,* 1996, vol. 271, 5953-5956
- **PERSSON A.K. et al.** *Mol. Pain.,* 2010, vol. 6, 84
- **RUSH A.M. et al.** *Eur.J.Neurosci,* 2005, vol. 22, 39-49
- **AKOPIAN A.N. et al.** *Nat. Neurosci,* 1999, vol. 2, 541-548
- **NOVAKOVIC S.D. et al.** *J. Neurosci.,* 1998, vol. 18, 2184-2187
- **BLAIR N.T. et al.** *J. Neurosci.,* 2003, vol. 23, 10338-10350
- **RENGANATHAN M et al.** *J. Neurophysiol,* 2001, vol. 86, 629-640
- **CHOI J.S.** *J. Neurophysiol,* 2011, vol. 106, 3173-3184
- **TAN Z.Y. et al.** *J. Neurosci.,* 2014, vol. 34, 7190-7197
- **FABER C.G. et al.** *Ann. Neurol,* 2012, vol. 71, 26-39
- **HAN C et al.** *J. Neurol Neurosurg Psychiatry,* 2014, vol. 85, 499-505
- **KIST A.M. et al.** *PLoS One,* 2016, vol. 11, e0161789
- **EIJKENBOOM I. et al.** *J. Neurol Neurosurg Psychiatry,* 2019, vol. 90 (3), 342-352
- **LAIRD J.M. et al.** *J. Neurosci,* 2002, vol. 22, 8352-8356
- **JARVIS M.F. et al.** *Proc Natl Acad Sci USA,* 2007, vol. 104, 8520-8525
- **JOSHI S.K. et al.** *Pain,* 2006, vol. 123, 75-82
- **ROZA C. et al.** *J Physiol,* 2003, vol. 550, 921-926
- **KORT M.E. et al.** *Bioorg Med Chem Lett,* 2010, vol. 20, 6812-6815
- **SCANIO M.J. et al.** *Bioorg Med Chem,* 2010, vol. 18, 7816-7825
- **PAYNE C.E. et al.** *Br J Pharmacol,* 2015, vol. 172, 2654-2670
- **HILLE, B.** *J. Gen. Physiol.,* 1977, vol. 69, 497-515
- **HILLE. B.** Ion Channels of Excitable Membranes. Sinauer Associates, Inc, 1992, 391-421
- **HONDEGHEM L.M. ; KATZUNG B.G.** *Annu. Rev. Pharmacol. Toxicol.,* 1984, vol. 24, 387-423
- **CATTERALL W.A.** *Trends Pharmacol. Sci.,* 1987, vol. 8, 57-65
- *Chem. Rev.,* 2009, vol. 109 (6), 2551-2651
- **T. W. GREEN ; P. G. M. WUTS.** Protective Groups in Organic Synthesis. Wiley-Interscience, 1999
- *Synthesis,* 2002, 2561-2578
- *Chem. Soc. Rev,* 2021, vol. 50, 8214-8247
- March's Advanced Organic Chemistry. 2007, 1427-1474